# EUROPEAN PATENT APPLICATION

(11) **EP 4 115 885 A1**
(43) Date of publication of application: **11.01.2023**
(21) Application number: 21183823.0
(22) Date of filing: 05.07.2021
(51) Int. Cl.: A61K 31/495, A61P 31/14

(54) **A PHARMACEUTICAL COMPOSITION COMPRISING BAY 86-5277 AND SALTS THEREOF FOR USE IN THE TREATMENT OF VIRAL INFECTIONS AND HYPERINFLAMMATION**

(71) Applicant: Charité - Universitätsmedizin Berlin, 10117 Berlin (DE); Universität Leipzig, 04109 Leipzig (DE)
(72) Inventor: LEHMANN, Irina, 12555 Berlin (DE); LANDMESSER, Ulf, 12205 Berlin (DE); CONRAD, Christian, 14165 Berlin (DE); EILS, Roland, 12555 Berlin (DE); TRUMP, Saskia, 12157 Berlin (DE); CHUA, Robert Lorenz, 10179 Berlin (DE); LUKASSEN, Sören, 10623 Berlin (DE); LIEBIG, Johannes, 13349 Berlin (DE); THÜRMANN, Loreen, 10405 Berlin (DE); HENNIG, Bianca, 113353 Berlin (DE); LAUDI, Sven, 04105 Leipzig (DE)
(74) Representative: Hertin und Partner Rechts- und Patentanwälte PartG mbB

(57) **Abstract**

The invention relates to a pharmaceutical composition comprising BAY 86-5277 or salt thereof for use in the treatment and/or prevention of a medical condition associated with a viral infection and comprising hyperinflammation in a human subject. The invention further relates to a combination of BAY 86-5277 or a salt thereof and at least one other treatment, such as a guideline treatment for severe respiratory disease, for example recommended by the S3 guideline on in-patient treatment of COVID-19, preferably a glucocorticoid, more preferably dexamethasone. The invention further relates to a pharmaceutical composition comprising BAY 86-5277 or salt thereof or said combination with a glucocorticoid, and use of the combination or composition, in the treatment and/or prevention of a viral infection in a subject and/or a medical condition associated with hyperinflammation, preferably hyperinflammation in the respiratory tract, acute respiratory failure, pneumonia, acute respiratory distress syndrome and/or multiorgan failure. In preferred embodiments, the invention relates to the treatment and/or prevention of a medical condition associated with a SARS-CoV infection, preferably SARS-CoV-2 infection.

## Description

The invention relates to the field of pharmaceutical compositions, combinations, and the treatment of medical conditions associated with viral infections.

The invention relates to a pharmaceutical composition comprising BAY 86-5277 or salt thereof for use in the treatment and/or prevention of a medical condition associated with a viral infection and comprising hyperinflammation in a human subject.

In preferred embodiments, the invention relates to a combination of BAY 86-5277 or a salt thereof and at least one other treatment, such as a guideline treatment for severe respiratory disease, for example recommended by the S3 guideline on in-patient treatment of COVID-19, preferably a glucocorticoid, more preferably dexamethasone.

The invention further relates to a pharmaceutical composition comprising BAY 86-5277 or salt thereof or said combination with a glucocorticoid, and use of the combination or composition, in the treatment and/or prevention of a viral infection in a subject and/or a medical condition associated with hyperinflammation, preferably hyperinflammation in the respiratory tract, acute respiratory failure, pneumonia, acute respiratory distress syndrome and/or multiorgan failure. In preferred embodiments, the invention relates to the treatment and/or prevention of a medical condition associated with a SARS-CoV infection, preferably SARS-CoV-2 infection.

### BACKGROUND OF THE INVENTION

Coronaviruses (CoVs) are a large family of viruses that are common in mammals including humans and birds. Whereas alpha- and beta-coronaviruses infect only mammals, infection with gamma- and delta-coronaviruses is mainly found in birds. Coronavirus infections cause respiratory illness in humans and gastroenteritis in animals (Cui, Li, and Shi 2019). Until recently six coronaviruses pathogenic to humans were known. Based on sequence data an animal origin of all human pathogenic coronaviruses can be deduced. There are four endemic human CoVs (HCoV-NL63, HCoV-229E, HCoV-OC43 and HCoV-HKU1) that mainly cause mild respiratory tract infections (Corman, Lienau, and Witzenrath 2019).

The first CoV, highly pathogenic to humans, was SARS-CoV causing an outbreak of severe acute respiratory syndrome (SARS) in 2002 and 2003 in China. Ten years later, Middle East respiratory syndrome coronavirus (MERS-CoV) emerged in Middle Eastern countries.

In December 2019, a novel SARS-coronavirus 2 (SARS-CoV-2) emerged in Wuhan (Hubei province, China). Subsequently, SARS-CoV-2 rapidly spread within China and Asian countries before causing a worldwide pandemic of a new disease called Coronavirus Disease 2019 (COVID-19).

The SARS-CoV-2 virus is a beta-coronavirus, like MERS-CoV and SARS-CoV. Like SARS-CoV and MERS-CoV, the most recently emerged coronavirus SARS-CoV-2 can cause severe respiratory illness, causing challenges to physicians and hospitals worldwide, adding an unprecedented burden to the health care systems of many countries.

Even though a majority of patients with COVID-19 present with mild symptoms, such as cough and fever, or no symptoms at all, hospitalization is required for approximately 5% of infected subjects and patients may develop a critical disease course with acute respiratory failure. Approximately one quarter of subjects hospitalized with COVID-19-associated pneumonia require respiratory support in an intensive care unit (ICU), and the need for invasive mechanical ventilation (IMV) has been associated with high mortality¹⁻³.

It is proposed that the virus directly promotes cell damage, whereby a systemic inflammatory response followed by multiple organ injury is triggered. CoVs also cause a dysfunctional renin-angiotensin system, which increases the pulmonary vascular permeability being a factor for the development of pulmonary edema. Both systemic inflammatory responses and a dysfunctional renin-angiotensin system contribute to a so-called cytokine storm that triggers an acute respiratory distress syndrome (ARDS). Multi-organ failure and/or the onset of ARDS represent a severe state of COVID-19 patients with a high mortality risk within few weeks or days.

Both virus-specific factors and host inflammatory responses determine COVID-19 severity and outcome³⁻⁶. An ineffective early innate antiviral response followed by impaired adaptive immune responses and hyper-inflammation may lead to tissue injury resulting in ARDS, multiorgan failure, and death⁷. Abnormal blood levels of several anti-inflammatory cytokines, chemokines and other mediators have been associated with worse outcomes^{2, 3, 8}. In particular, elevated IL-6 levels were shown to correlate with an increased risk of death^{8, 9}.

Consequently, it has been suggested to use corticoids or more specific immune therapies to control this exacerbated immune response in COVID-19. Among others, dexamethasone has been successfully evaluated in clinical trials for COVID-19 ARDS: in patients hospitalized for COVID-19 dexamethasone treatment lowered 28-day mortality in those patients receiving either supplemental oxygen or mechanical ventilation but not in patients without any respiratory support¹⁰. In patients with moderate to severe ARDS treatment with dexamethasone significantly increased the number of ventilator-free days compared to standard care only¹¹.

In addition to corticosteroids, various immunomodulatory drugs were used in COVID-19 clinical trials, including monoclonal antibodies, some immunosuppressive drugs (such as the IL1-receptor antagonist Anakinra), and interferons. Since elevated IL-6 levels correlated with an increased risk of COVID-19 mortality, many trials focusing on tocilizumab, a monoclonal antibody and IL6 receptor antagonist, were initiated¹². Current results on the use of tocilizumab are conflicting: while the REMAP-CAP trial¹³ reported an improved outcome in critical ill patients and data from RECOVERY¹⁰ described an increased survival of hospitalized COVID-19 patients with respiratory support; other trials showed no effects on mortality and/or clinical improvement, but even a potential risk of adverse events by tocilizumab treatment^{10, 13-19}. Therefore, at the moment, the only recommended therapeutic option in patients hospitalized for COVID-19 needing respiratory support besides supportive care is the administration of dexamethasone (Australian guidelines for the clinical care of people with COVID-19; S3 guideline recommendations for hospital-based therapy of patients with COVID-19.). For patients hospitalized for COVID-19 who require supplemental oxygen, tocilizumab might be considered, but it is recommended only conditionally.

Thus, effective therapies do not currently exist, but are urgently needed. Mortality reduction of available drug therapy in severe COVID-19 disease has been demonstrated so far for dexamethasone and tocilizumab in randomized controlled trials.

Data from single-cell studies provided a mechanistic understanding on how the immune response in the airways is orchestrated in COVID-19 patients. Macrophages that recruit and activate further immune cells such as neutrophils and T cells were identified by us and others as the major players responsible for the exacerbated immune inflammation in the airways of COVID-19 patients²⁰⁻²². We observed a significant induction of *CCL2, CCL3,* and *CCL4* expression in macrophages together with an increased expression of the corresponding chemokine receptor 1 (*CCR1*) in different immune cells in patients with critical COVID-19^{21, 22}. Because binding of the chemokines to their receptor can induce immune cell recruitment into the lung parenchyma with subsequent differentiation into inflammatory phenotypes, CCR1 might represent a promising target in critical COVID-19. IFNa has been shown to induce CCR1. Binding of chemokines to their receptor can induce recruitment of immune cells to the lung parenchyma with subsequent differentiation into inflammatory phenotypes.

The potent and selective CCR1 antagonist BAY 86-5277 and salt thereof (BX471 or ZK 811752 or BAY 3496025 or benzurium sulfate) competitively displaces ligands binding to CCR1 (e.g., CCL3, CCL5) from the human receptor, thereby preventing CCR1-mediated biological responses including immune cell chemotaxis. BAY 86-5277 has demonstrated activity in numerous preclinical *in vitro or in vivo* pharmacology and efficacy models such as an experimental rat model of autoimmune encephalitis (EAE), a rabbit renal graft rejection model, and a mouse renal fibrosis model. Patent applications for BAY 86-5277 and salt thereof (WO1998056771, BX471) and a first medical indication have been published (US31053801, BX471), including VILI, pancreatitis-induced acute lung injury and sepsis models.

Despite the high medical need, no treatment form exists that targets CCR1, by antagonizing its receptor site, for treatment of hyperinflammation, especially in viral infections with SARS-CoV-2. Although BAY 86-5277 is known as a CCR1 antagonist, its administration and therapeutic effect have not been studied either individually or in combination with established drug therapy for severe COVID-19 disease, for example as recommended by the S3 guideline for hospital-based therapy of patients with COVID-19.

### SUMMARY OF THE INVENTION

In light of the prior art, the technical problem underlying the present invention is to provide alternative or improved means for the treatment and/or prevention of a medical disorder associated with a viral infection and comprising hyperinflammation. The technical problem may also be viewed as the provision of means for the treatment and/or reduction of risk of medical conditions associated with a SARS coronavirus, more particular with SARS-CoV-2. The technical problem may also be viewed as the provision of means for the prevention and/or treatment of a SARS coronavirus-associated hyperinflammation in the respiratory tract, acute respiratory failure, pneumonia, acute respiratory distress syndrome and/or multiorgan failure.

This problem is solved by the features of the independent claims. Preferred embodiments of the present invention are provided by the dependent claims.

The invention relates to a pharmaceutical composition comprising BAY 86-5277 or salt thereof for use in the treatment and/or prevention of medical disorder associated with viral infection and comprising hyperinflammation in a human subject.

In some embodiments, the invention relates to the pharmaceutical composition comprising BAY 86-5277 or salt thereof for use in the treatment of a medical disorder associated with a viral infection, such as a coronavirus infection, comprising the treatment and/or prophylaxis of hyperinflammation and/or a SARS coronavirus-associated respiratory disease, and corresponding methods of treatment.

In some embodiments, the subject is or has been treated additionally with a guideline treatment for severe respiratory disease, wherein the guideline treatment is not a CCR1 antagonist.

In some embodiments, the invention relates to the combined administration of a therapeutically effective amount of a CCR1 antagonist, preferably BAY 86-5277 or salt thereof, and a guideline treatment for severe respiratory disease, such as a treatment recommended by the guidelines for hospitalized COVID-19 patients in such treatment.

In some embodiments, the treatment recommended by the guidelines for hospitalized COVID-19 patients is a non-CCR1 antagonist drug, wherein the non-CCR1 antagonist drug is preferably a glucocorticoid, more preferably dexamethasone.

As used herein, the non-CCR1 antagonist drug recommended by the guidelines for hospitalized COVID-19 patients, preferably dexamethasone, may be referred to as a guideline drug.

According to embodiments of the present invention, a composition comprising BAY 86-5277 or salt thereof and/or the drug recommended by the guidelines for hospitalized COVID-19 patients as well as a drug product comprising said composition have a therapeutically relevant effect on inflammation, hyperinflammation, acute respiratory failure, pneumonia, acute respiratory distress syndrome and/or multiorgan failure. In some embodiments, medical conditions are treatable using the present invention, which are associated with SARS-CoV, such as SARS-CoV-2 related diseases involving immune hyperactivation, hyperinflammation and increased levels of CCL2, CCL3, CCL4 or CCR1 in body fluids or cells of a subject. In one embodiment, levels of CCL2, CCL3, CCL4 or CCR1 can be detected in blood, serum, plasma, or cells obtained from a subject, particularly immune cells of the nasopharyngeal area, e.g. nasopharyngeal swabs.

The invention also relates to the combined administration of a therapeutically effective amount of BAY 86-5277 or salt thereof and a therapeutically effective amount of the guideline drug, preferably dexamethasone, in such treatment or prevention. Increased levels of CCL2, CCL3, CCL4 or CCR1 in subjects can lead to a severe course of SARS-CoV viral infection, particularly for COVID-19, including for example immune hyperactivation, hyperinflammation, acute respiratory distress syndrome and increased risk of mortality.

SARS-CoV-infected subjects express CCL2, CCL3, CCL4 or CCR1 to activate immune cells, e.g. macrophages, while SARS-CoV infected subjects who require hospitalization and/or artificial ventilation and/or invasive mechanical ventilation significantly induce CCL2 and CCL3 expression in macrophages together with an increased expression of CCR1 at higher levels compared to controls, as shown in the Examples. As shown below, binding of CCL2, CCL3 or CCL4 to CCR1 can induce recruitment of monocytes to the lung parenchyma with subsequent differentiation into inflammatory macrophages and consecutive recruitment and activation of further immune cells and epithelial damage. The inventors have thus for the first time elucidated a mechanism for severe COVID19 disease course and identified CCR1 as a potential target for therapy. CCR1 represents a promising anti-inflammatory target in the therapy and prevention of hyperinflammation, immune cell hyperactivity, acute respiratory failure, pneumonia, acute respiratory distress syndrome and/or multiorgan failure, especially for COVID-19.

It has proven challenging to elucidate the significance of CCL2, CCL3 or CCR1 in SARS-CoV infected subjects, particularly COVID-19 subjects, and to identify compositions that have a therapeutic effect on CCL2, CCL3, CCL4 or CCR1 expression levels, or for blocking or diminishing binding of CCL2 or CCL3 to CCR1. In the prior art, the development of COVID-19 therapies for immune hyperactivation currently focusses on inhibitors of key immunological players, such as IL-6, TNF and interferon (NCT04359901, NCT04322773and NCT04311697), but such approaches induce severe side effects in treated subjects.

The inventors further found that expression of CCR1, the receptor bound by CCL2 and CCL3, increases in non-resident macrophages and neutrophils concomitantly with the severity of COVID-19. As shown in the Examples, binding of CCL2, CCL3 or CCL4 to CCR1 can induce recruitment of monocytes to the lung parenchyma with subsequent differentiation into inflammatory macrophages and consecutive recruitment and activation of further immune cells and epithelial damage.

Without being bound by theory, the treatment of the present invention seeks to lower binding of CCL2, CCL3 or CCL4 to CCR1 in SARS-CoV infected subjects, thereby delaying or interrupting the recruitment of monocytes to the lung parenchyma with subsequent differentiation into inflammatory macrophages and consecutive recruitment and activation of further immune cells and epithelial damage. In other aspects or embodiments, the invention seeks to aid in the reduction of risk for developing immune hyperactivation after infection with a respiratory viral infection.

The present invention provides solution to these challenges, as described in further detail below. The inhibition or reduction of endogenous binding of CCL2, CCL3 or CCL4 to CCR1 and thus reduced recruitment of monocytes to the lung parenchyma, with subsequent differentiation into inflammatory macrophages in SARS-CoV infected subjects, via BAY 86-5277 or salts thereof and/or the guideline drug, preferably dexamethasone, offers a novel therapeutic strategy to reduce immune hyperactivation, acute respiratory failure, pneumonia, acute respiratory distress syndrome and high mortality risk in SARS-CoV infected subjects. The combination of beneficial therapeutic properties could not have been predicted by a skilled person and therefore relate to non-obvious, special technical features of the invention.

In one embodiment, the subject is hospitalized and receiving treatment comprising respiratory support.

In one embodiment, the virus-infected subject is preferably a hospitalized subject, wherein the hospitalized subject may or may not receive respiratory support.

In one embodiment, the virus-infected subject is hospitalized in an intensive care unit.

In one embodiment, the SARS-CoV infected subject is hospitalized at the intensive care unit.

In one embodiment, the SARS-CoV infected subject is a hospitalized subject, wherein said subject may or may not receive a respiratory support.

In one embodiment, the SARS-CoV-2 infected subject being hospitalized may be at the intensive care unit.

In one embodiment, the SARS-CoV-2 infected subject is a hospitalized subject, wherein said subject may or may not receive a respiratory support.

In hospitalized patients with COVID-19, an increase in oxygen demand prompts the clinician to decide how and when to escalate treatment. An important treatment goal is to avoid the need for invasive mechanical ventilation whenever possible.

In one embodiment, the hospitalized subject receives non-invasive respiratory support, wherein the non-invasive respiratory support preferably comprises continuous positive airway pressure (CPAP) or nasal high-flow oxygen (HFNO).

In one embodiment, the hospitalized subject receives invasive respiratory support, wherein the invasive respiratory support comprises invasive mechanical ventilation, endotracheal tube, tracheostomy tube, and/or extracorporeal membrane oxygenation (ECMO).

Concerns have been raised in the context of COVID-19 that treatment strategies specified above could harm patients through delays in tracheal intubation or exacerbation of lung injury, harm healthcare workers through nosocomial infections, and financially and logistically challenge the healthcare system through the high oxygen demand of the devices.

As described at length herein, the composition comprising BAY 86-5277 or salt thereof and/or the guideline drug, preferably dexamethasone, represents a novel approach towards developing an effective measure to treat and/or prevent a medical disease associated with viral infection, particularly SARS-CoV infection in infected subject, wherein said subject is preferably hospitalized and receiving treatment comprising respiratory support. At present, said patients are not appropriately treated as no effective medication may be available for the patient group described herein. As evident from the experimental support provided (Examples), increased expression of CCR1 significantly correlates with the severity of COVID-19, immune hyperactivation, inflammation and related diseases in the respiratory tract. CCL2-, CCL3-, or CCR1 expression in SARS-CoV-2 infected subjects are unexpectedly associated with increased inflammatory tissue injury, lung injury, and respiratory failure and an increased risk of death.

To systematically compare the differences in cytokine/chemokine expression profiles between individual cases, the inventors compared the expression patterns within non-resident macrophages in longitudinal samples from patients with varying disease severity, as is described in detail in the Examples.

For example, patient BIH-CoV-12 (male, 71 years) is a typical patient with a moderate course of COVID-19. Patient BIH-CoV-07, a middle-aged male with no risk factors, suffered from critical COVID-19 with a transient need for nasal high-flow oxygen ventilation and intensive medical monitoring. He was admitted to the intensive care unit (ICU) with isolated respiratory failure and bipulmonary infiltrates. He was discharged from the ICU after 2 days and left the hospital in good health. Patient BIH-CoV-06 is a 63-year-old man with obesity and a history of smoking who presented with critical COVID-19. He required mechanical ventilation and developed severe acute respiratory distress syndrome (ARDS) and multiorgan failure. After 2 weeks on an ICU, his condition gradually improved, and he could be extubated. Patient BIH-CoV-02 had critical COVID- 19 with severe ARDS and died. The expression patterns of genes encoding cytokines/chemokines were similar in patients BIH-CoV-12 and BIH-CoV-07. Both mechanically ventilated patients with critical COVID-19 (BIH-CoV-02 and BIH-CoV-06) showed increased expression of CCL3 and CCL2 compared with patients BIH-CoV-12 and BIH-CoV-07. In patient BIH-CoV-06, these changes increased over time and converged to a similar pattern as in patient BIH-CoV-02, who died shortly after the observation time point shown. This would be consistent with the clinical course of patient BIH-CoV-06, as he became ill in a critical condition over an extended period of time.

Through employing the treatments of the present invention, the cytokine/chemokine expression profiles are not only used to identify patients who may need this treatment, but the treatment directly addresses the mechanisms underlying disease progression, by the inhibition or reduction of endogenous binding of CCL2, CCL3 or CCL4 to CCR1.

In one embodiment, BAY 86-5277 antagonizes the binding of ligands, i.e. CCL2, CCL3 and CCL4, to its receptor CCR1 in a target cell expressing CCR1, such as macrophages. Through this antagonistic effect, BAY 86-5277 or salt thereof delays or interrupts the recruitment of monocytes to the lung parenchyma with subsequent differentiation into inflammatory macrophages and consecutive recruitment and activation of further immune cells and epithelial damage by each dose administered.

In one embodiment, dexamethasone binds in the ligand binding domain of glucocorticoid receptor (GR) and competes for binding with endogenous ligands like e.g. cortisol, in a GR expressing target cell. Dexamethasone may reduce number, activity, and movement of inflammatory cells into the bronchial submucosa, resulting in decreased airway responsiveness by each dose administered. In one embodiment, reduction of inflammation, immune hyperactivation, acute respiratory failure, pneumonia, acute respiratory distress syndrome and/or multiorgan failure is amplified when BAY 86-5277 or salt thereof and/or dexamethasone are administered in the inventive combination.

In one embodiment, the physiological response of antagonizing binding to CCR1 can be determined by quantifying CCL2, CCL3, CCL4 and/or CCR1 levels in cells, blood, plasma, serum, from a virus-infected patient, preferably cells from nasopharyngeal area from COVID-19 subject, treated with BAY 86-5277 or salt thereof and/or dexamethasone. In one embodiment, the physiological response to antagonizing binding to CCR1 can be determined by evaluating clinical parameters of a virus-infected subject treated with BAY 86-5277 or salt thereof and/or dexamethasone. In some embodiments, the evaluation of clinical parameters includes monitoring the progression of medical disease associated with the viral infection, such as COVID-19, and the clinical status of the virus-infected subject, e.g., infected with SARS-CoV, treated with BAY 86-5277 or salt thereof and/or dexamethasone.

In some embodiments, the clinical parameters for monitoring physiological response comprise those of the WHO clinical progression scale, such as a need for oxygen/invasive ventilation, ventilation rate, inspiratory oxygen fraction (FiO₂), oxygen partial pressure (paO₂), carbon dioxide partial pressure (paCO₂), pH, oxygen application (e.g. nasal, high-flow, non-invasive ventilation, invasive ventilation), ventilation mode on invasive ventilation, ventilation settings on invasive ventilation, electrocardiography, acute physiology and chronic health evaluation (APACHE), simplified acute physiology score (SAPS), sequential organ failure assessment (SOFA), glasgow coma scale (GCS), screening for delirium, blood pressure, need for vasopressor (amount), lactate, heart rhythm, heart rate, inotrope therapy (other than cardiac glycosides), urine volume per 24h, urea, creatinine, or other laboratory parameters (e.g. liver: AST (ASAT), ALP (AP), PPT (Quick)/INR, bilirubin, pancreas: lipase; others: CK, leukocytes, PLT, Hb, CRP, PCT, potassium, BNP, troponin).

In one embodiment, the effectiveness and physiological response of the treatment, as described herein, is determined by a decreased WHO clinical progression scale value compared to said value determined before treatment initiation.

In one embodiment, the effectiveness and physiological response of the treatment, as described herein, is determined by a decreased CCL2, CCL3, CCL4 and/or CCR1 levels compared to said levels determined before treatment initiation.

In one embodiment, CCL2, CCL3, CCL4 and/or CCR1 levels are determined as described in the Examples.

In one embodiment, the effectiveness and physiological response of the treatment, as described herein, is determined by increased respiratory support-free days.

In one embodiment, the effectiveness and physiological response of the treatment, as described herein, is determined by improvements in simplified acute physiology score (SAPS), and/or sequential organ failure assessment (SOFA) and/or glasgow coma scale (GCS) compared to said scores determined before treatment initiation .

In one embodiment, the effectiveness and physiological response of the treatment, as described herein, is determined by increased number of days after extubation.

In one embodiment, the effectiveness and physiological response of the treatment, as described herein, is determined by reduced severity of gas exchange impairment (paO₂/FiO₂).

In one embodiment, the effectiveness and physiological response of the treatment, as described herein, is determined by increased ICU free days and reduced duration of hospitalization.

In one embodiment, prior to initiating treatment or prevention of virus infected subjects, e.g. SARS-CoV-2 infected and hospitalized subjects, a sample, e.g. is collected prior to treatment. Said sample is preferably used for determining CCL2, CCL3, CCL4 or CCR1 levels and/or laboratory parameters described herein in cells, blood, plasma or serum according to the standard analytics of the prior art and by means of methods described in the Examples. More details for sample handling and measurement are described in the Examples.

BAY 86-5277 has been tested in clinical studies in humans in Phase I studies in healthy volunteers. Both intra gastric and oral administration of a liquid formulation, immediate-release tablets and extended-release tablets were found to be safe in terms of vital signs, electrocardiogram, and safety laboratory parameters at all doses tested. Administration of a single dose of the formulation was escalated up to 1200 mg without reaching a maximum tolerated dose. BAY 86-5277 has been further investigated in at least three clinical phase IIa studies, including in multiple sclerosis, psoriasis, and endometriosis. In all phase II studies, BAY 86-5277 was found generally well tolerated and with a safety profile acceptable to patients.

The present invention therefore represents a tailored intervention for reducing or blocking binding of CCL2, CCL3 or CCL4 to CCR1 by the CCR1 antagonist BAY 86-5277 in combination with dexamethasone, leading to reduction of immune hyperactivation, acute respiratory failure, pneumonia, acute respiratory distress syndrome, multiorgan failure, and/or mortality risk in virus infected subjects, such as SARS-CoV or SARS-CoV-2, particularly in hospitalized COVID19 subject, or only one of each therapeutic.

In one embodiment, the subject receives non-invasive respiratory support (including supplementary oxygen) and/or invasive mechanical ventilation.

In one embodiment, the subject receives non-invasive respiratory support comprising a face mask and oxygen supply to the patient's upper airway.

In one embodiment, the subject receives invasive respiratory support comprising endotracheal tube, tracheostomy tube and/or extracorporeal membrane oxygenation (ECMO).

In some embodiments, the medical disorder comprises hyperinflammation in the respiratory tract, acute respiratory failure, pneumonia, acute respiratory distress syndrome and/or multiorgan failure.

In some embodiments, the patient suffers from an infection, preferably with a SARS coronavirus (SARS-CoV). As used herein, SARS coronavirus refers to a coronavirus that leads to severe acute respiratory syndrome (SARS). Clinically, patients show respiratory symptoms such as dry cough and shortness of breath, fever or diarrhea. But symptoms associated with acute liver injury, heart injury or kidney injury can also occur. Older patients (above 60 years), patients with chronic diseases (e.g. cardiovascular diseases, diabetes, cancer, COPD) or immune compromised patients are considered to be at a higher risk to face a severe development of SARS.

In some embodiments, the medical disorder is associated with a respiratory viral infection.

In some embodiments, the respiratory viral infection is also referred to as a lung and airway infection. Medical disorders associated with a respiratory viral infection comprise bronchiolitis, interstitial pneumonia, or parenchymal infection, severe acute respiratory syndrome, acute respiratory distress syndrome, and respiratory tract hyperinflammation.

In some embodiments, the pharmaceutical composition, or the two drugs of the combination, is intended for use in the treatment and/or prevention of medical disorder is associated with a respiratory viral infection, wherein the respiratory viral infection includes the common cold, and infection due to respiratory syncytial virus, influenza, coronavirus (including SARS) and adenovirus.

In some embodiments, a target cell for the treatment is a cell infected with a virus, such as endothelial cells, in particular cells in the vascular endothelial, nasal mucosa, bronchus and/or lung cells.

In some embodiments, the medical disorder is associated with a SARS coronavirus infection.

In some embodiments the medical condition associated with a SARS coronavirus is preferably COVID-19 or a SARS coronavirus-associated respiratory disease. As used herein, SARS Coronavirus refers to a Coronavirus that leads to severe acute respiratory syndrome (SARS).

SARS is typically induced via droplet transmission and replication of the virus could be shown in the upper and lower respiratory tract or gastrointestinal mucosa. In parallel the virus is also capable of directly invading cells of different organs such as liver, kidney, heart and brain. Another distinct mechanism appears to be the direct invasion of the virus in T-cells. A significant number of SARS patients, who suffer COVID-19, have a clinically low concentration of lymphocytes in the blood, also known as lymphocytopenia. Clinically, patients show respiratory symptoms such as dry cough and shortness of breath, fever or diarrhea. But symptoms associated with acute liver injury, heart injury or kidney injury can also occur. In a less severe state of SARS, patients can show mild or even no symptoms.

In some embodiments, the medical disorder is associated with a SARS-CoV-2 infection.

The invention therefore relates to corresponding methods of treatment, comprising the administration of the pharmaceutical composition, or the two drugs of the combination, as described herein to a subject in need thereof in order to treat and/or prevent a medical condition associated with a SARS coronavirus.

In a preferred embodiment, the pharmaceutical composition, or the two drugs of the combination, for use as a medicament as described herein, is characterized in that the treatment of viral infections comprises the treatment of a subject with detectable viral genome in target cells compared to a control, such as healthy controls.

Additionally, the pharmaceutical combination of the present invention is used for pre- and/or post-exposure prophylaxis of the disease. The pharmaceutical composition, or the two drugs of the combination, described herein can be used as a prophylactic in groups of patients at risk of developing a severe disease course of COVID-19 infection, including the need for hospitalization, respiratory support, acute respiratory failure, pneumonia, acute respiratory distress syndrome, immune hyperactivation, multi-organ failure, and death.

In a further aspect, CCL2, CCL3, CCL4 and CCR1 expression and drug-induced therapy efficacy can also be detected and quantified by an in vitro method for determining the presence or absence of CCL2, CCL3, and CCR1 expression in a sample, wherein drug-induced therapy efficacy can be further determined by evaluating clinical parameter as described herein.

In a further aspect, viral infections and drug-induced therapy efficacy can also be detected and quantified by an in vitro method for determining the presence or absence of SARS-CoV-2 viral genomic material in a sample. Detection of viral genomes or parts thereof can be carried out by amplifying the genetic viral material by means of a polymerase chain reaction (PCR), as shown in the Example. By way of example, a method may be employed selected from the group consisting of nucleic acid amplification methods, such as PCR, qPCR, RT-PCR, qRT-PCR.

The invention therefore relates to methods of treatment and/or prevention of medical disorders associated with viral infections and patient groups described herein. The invention also relates to combined methods of diagnostics and treatment based on CCL2, CCL3, CCL4 and CCR1 expression, or other clinical parameters, such as a WHO clinical progression scale, the need for respiratory support, viral genomes detected and subsequent viral clearance, respectively.

The features of the invention regarding methods of treatment and diagnosis, and descriptions of the pharmaceutical composition for use in the treatment of various medical conditions, apply to the pharmaceutical combination itself, and vice versa.

In some embodiments, the subject has an elevated level of one or more cytokines and/or chemokines selected from CCL2, CCL3, CCL4 and/or chemokine receptor CCR1, wherein said level is elevated in comparison to a level in one or more healthy control subjects and is determined in a sample comprising a bodily fluid or cells from the subject.

In one embodiment, blood samples for differential blood work, CRP, PCT, and additional cytokine measurements including CCL2, CCL3, CCL4, CCR1 are obtained on day 1, 4, 7, 10, 14, 21, and 28 from the subject. In one embodiment, nasopharyngeal swabs are collected on day 1, 4, 7, 10, 14, and 21 for single-cell transcriptome analyses and viral load determination. Single-cell transcriptome analyses and viral load determination are performed as described in the Examples.

As is shown below, by conducting a thorough study, the inventors surprisingly demonstrated an association between CCL2-, CCL3-, or CCR1 expression in virus-infected subjects and immune hyperactivation, inflammatory tissue injury, lung injury, respiratory failure, and an increased risk of death, and in relation thereto, found a beneficial medical use of the pharmaceutical composition comprising BAY 86-5277 and/or a guideline drug, preferably dexamethasone. The inventors surprisingly find in a study that higher CCL2-, CCL3-, CCL4, or CCR1 expression in immune cells in SARS-CoV-2 infected subjects can be associated with severe and critical course of COVID19, preferably acute respiratory failure, pneumonia, acute respiratory distress syndrome and/or multiorgan failure, and death.

The present invention is related to treating a risk of developing immune hyperactivation, inflammation, respiratory failure, and diseases in the respiratory tract (e.g. acute respiratory distress syndrome) in a virus-infected subject, e.g. a SARS-CoV-2 infected and hospitalized subject, with therapeutically relevant CCL2-, CCL3-, CCL4, or CCR1 expression in immune cells. The composition provided by the present disclosure may be capable of preventing, delaying or stopping the development of severe and critical course of medical disorder associated with viral infection, particularly SARS-CoV-2. The use of samples received from COVID-19 patients and controls in two cohorts is a particular advantage of the present disclosure and a critical strength of the clinical study. The present disclosure is the first to show an association with CCL2-, CCL3-, CCL4, or CCR1 expression and severe and critical courses of COVID19 disease in hospitalized patients and providing means to prevent or to treat said disease.

In some embodiments, the subject is or has been treated with a guideline treatment for severe respiratory disease, such as with a non-CCR1 antagonist guideline drug, wherein the non-CCR1 antagonist guideline drug can be a glucocorticoid.

Guidelines recommend therapeutic options in patients hospitalized for COVID-19 needing respiratory support. An S1 guideline on intensive care therapy for patients with COVID-19 was published first in March 2020. The current S3 guideline (May 2021) covers the entire inpatient care setting comprising current recommendations on drug therapy (tocilizumab, tofacitinib, monoclonal antibodies) and palliative treatment. Immunosuppressive therapies that constrain the immune system, such as those now based on dexamethasone, are intended to prevent the immune system from overreacting, and showed beneficial effects in more severe courses of COVID-19.

In one embodiment, the non-CCR1 antagonist guideline drug is solely administered to the subject described herein.

In one embodiment, the non-CCR1 antagonist guideline drug is administered in combination with a CCR1 antagonist, preferably BAY 86-5277 or salt thereof, to the subject described herein

In one embodiment, the non-CCR1 antagonist guideline drug is a glucocorticoid.

In one embodiment, the non-CCR1 antagonist guideline drug is tocilizumab.

In one embodiment, the non-CCR1 antagonist guideline drug is tofacitinib.

In one embodiment, the non-CCR1 antagonist guideline drug is a monoclonal antibody.

In one embodiment, the glucocorticoid is solely administered to the subject described herein.

In one embodiment, the glucocorticoid is administered in combination with a CCR1 antagonist, preferably BAY 86-5277 or salt thereof, to the subject described herein

In one embodiment, tocilizumab is solely administered to the subject described herein.

In one embodiment, tofacitinib is solely administered to the subject described herein.

In one embodiment, tocilizumab is administered in combination with a CCR1 antagonist, preferably BAY 86-5277 or salt thereof, to the subject described herein

In one embodiment, tofacitinib is administered in combination with a CCR1 antagonist, preferably BAY 86-5277 or salt thereof, to the subject described herein.

In one embodiment, a monoclonal antibody is solely administered to the subject described herein.

In one embodiment, a monoclonal antibody is administered in combination with a CCR1 antagonist, preferably BAY 86-5277 or salt thereof, to the subject described herein In one embodiment, the glucocorticoid is dexamethasone or salt thereof.

In one embodiment, a therapeutic drug recommended by the guideline for hospitalized COVID19 patients used herein may include a glucocorticoid. Corticosteroids (also called glucocorticosteroids or glucocorticoids) are potent anti-inflammatory agents. Glucocorticoids are therefore used in medicine to treat diseases associated with a hyperactive immune system. Diseases associated with a hyperactive immune system comprise infections, particularly viral infections, allergies, asthma, autoimmune diseases, and sepsis. Beside natural glucocorticoids, various synthetic glucocorticoids are available.

In one embodiment, dexamethasone is solely administered to the subject described herein.

In one embodiment, dexamethasone is administered in combination with a CCR1 antagonist, preferably BAY 86-5277 or salt thereof, to the subject described herein.

In some embodiments, BAY 86-5277 or salt thereof and dexamethasone or salt thereof, act synergistically to reduce inflammation, inhibit infection of a target cell and/or inhibit replication of a SARS coronavirus, preferably SARS-CoV-2. In some embodiments, the active agents are administered at a dosage and frequency configured to act synergistically to achieve these effects.

In embodiments of the invention, the pharmaceutical combination may be administered to various patient groups, for example therapeutically (e.g. to patients with SARS-related illnesses, including critically ill patients) or prophylactically (e.g. to patients who are at risk of developing a severe course of the disease, or to patients who are infected with a SARS Coronavirus but have not yet developed serious health issues and are not at risk developing a severe course of the disease).

A skilled person is capable, based on the experimental and written support provided herein, to carry out the invention, in combination with common knowledge in the art. By employing the quantitative *in vitro* assessment described in the examples, or by using alternative quantitative and qualitative means for determining severity of gas exchange, WHO clinical progression scale, level of respiratory support, ICU requirement, reduced expression of CCL2, CCL3, CCL4, and/or CCR1, reduction in immune hyperactivation, reduction in viral infection, viral load, the combination of pharmaceutical agents and their necessary doses can be determined and adjusted in order to obtain synergistic effects due to the inventive combination. A skilled person can determine such an effect without undue effort.

In some embodiments, the treatment is administered at a dosage and frequency configured to prevent and/or to reduce a level of hyperinflammation and/or to reduce a level of one or more anti-inflammatory cytokines and/or chemokines and/or functional chemokine receptor to a level of at least 20% below a level at treatment initiation.

In one embodiment, said treatment is administered at a dosage and frequency configured to prevent and/or to reduce a level of chemokines to a level of at least 5%, 10%, 20%, 30%, 40% or at least 50% below a level at treatment initiation.

In one embodiment, said treatment is administered at a dosage and frequency configured to prevent and/or to reduce a level of chemokines to a level of at least 20% below a level at treatment initiation, wherein said chemokines include preferably CCL2, CCL3, and CCL4.

In one embodiment, said treatment is administered at a dosage and frequency configured to prevent and/or to reduce a level of anti-inflammatory cytokines to a level of at least 20% below a level at treatment initiation, wherein said anti-inflammatory cytokines include preferably IL-6 and IL8.

In one embodiment, said treatment is administered at a dosage and frequency configured to prevent and/or to reduce a level of functional chemokine receptor to a level of at least 20% below a level at treatment initiation, wherein said the functional chemokine receptor include CCR1.

A skilled person is capable, based on the experimental and written support provided herein, to carry out the invention, in combination with common knowledge in the art. By employing the quantitative measures described in the examples, or by using alternative quantitative means for determining reductions in levels of one or more anti-inflammatory cytokines and/or chemokines and/or functional chemokine receptor or hyperinflammation, the combination of pharmaceutical compounds and their necessary doses can be determined and adjusted in order to obtain beneficial effects due to the inventive application of the composition.

In one embodiment, the preferred administered dosage and frequency of BAY 86-5277 (e.g. 600 mg per dose and 2400 mg per day) or salt thereof and/or dexamethasone (6 mg per day) has an antagonistic effect on endogenous binding of CCL2 or CCL3 or CCL4 to CCR1 and reducing activation and migration of immune cells,
- wherein said dosage reduces level of hyperinflammation lower as before treatment initiation, preferably a level of hyperinflammation at least 20% lower as before treatment initiation, and/or
- wherein said dosage reduces level of antinflammatory cytokines lower as before treatment initiation, preferably a level of antinflammatory cytokines at least 20% lower as before treatment initiation, and/or
- wherein said dosage reduces level of chemokines lower as before treatment initiation, preferably a level of chemokines at least 20% lower as before treatment initiation, and/or
- wherein said dosage reduces level of chemokine receptor lower as before treatment initiation, preferably a level of chemokine receptor at least 20% lower as before treatment initiation.

In some embodiments, said treatment comprises administering BAY 86-5277 or salt thereof at 500-5000 mg/day to a human subject, preferably at 1000-3000 mg/day.

In one embodiment, BAY 86-5277 or salt thereof is administered to a human subject in an amount of 100 mg to 5000 mg, preferably 200 mg to 4000 mg, more preferably 500 mg to 3000 mg, or 1000 mg to 2500 mg, or 1500 mg to 2500 mg, preferably about 1000 mg, 1500 mg, 2000 mg or 2400 mg per day. In preferred embodiments, the dose of BAY 86-5277 or salt thereof is an oral, daily dose of between 1000-3000 mg, preferably between 1500 mg-2500 mg, more preferably 2400 mg.

In some embodiments, the treatment comprises administration of BAY 86-5277 or a salt thereof 2 to 4 times daily for 5 to 15 days, preferably 4 times daily for 10 days, wherein the route of administration is preferably via a drinking solution of 1 to 100 ml, preferably 5 to 15 ml per dose, via oral delivery or via a gastric tube.

In some embodiments, the BAY 86-5277 liquid formulation contains 60.0 mg active pharmaceutical ingredient per ml solution, and has been developed e.g. for clinical trials. BAY 86-5277 liquid formulation needs to be reconstituted before administration by adding the diluent to the bottle with the active pharmaceutical ingredient. In humans, plasma concentrations above 100 ng /mL BAY 86-5277 can be reached within approx. 15min, 30min, 1h, 2h, 3h, 4h, 5h, 6h, 7h, 8h, 9h, 10h, 11h or 12h after the first dose and can be maintained throughout steady state following three times oral administration of daily 600 mg BAY 86-5277 (extended released tablet formulation). The dosing rationale was supported by pharmacodynamic data obtained in a clinical phase I study as described below.

In preferred embodiments, the compounds are administered in concentrations or amounts, or according to dosage regimes, already established in the art, such as those for which regulatory approval has been issued (e.g. by the FDA or EMA), or in doses currently being assessed during phase 2 clinical trial, and/or according to the maximum allowed dose according to a phase I trial.

The above embodiments, regarding particular relative amounts, are based on clinically allowed or currently trialed doses of the various compounds described herein, being combined into a pharmaceutical composition as described herein.

In some embodiments, said treatment comprises administering a glucocorticoid, preferably dexamethasone or salt thereof at 1 to 20 mg/day, preferably 3 to 10 mg/day or it's corresponding glucocorticoid equivalent dose, respectively.

For example, a (hyper)inflammation and/or immune response modulation may be effectively treated by the administration of from about 0.5 mg to about 1 g, preferably 1 mg to about 500 mg, more preferably 3 mg to about 100 mg, even more preferably 6 mg with dexamethasone per patient per day.

In one embodiment, the pharmaceutical combination comprising dexamethasone described herein is administered to the subject for 10 days with 6 mg/day, preferably administered either as drinking solution per oral solution or via a gastric tube or intravenous.

Another example, (hyper)inflammation and/or an immune response modulation may be effectively treated by the administration of between 1000-3000 mg, preferably between 1500 mg-2500 mg, more preferably 2400 mg oral, daily dose BAY 86-5277 per patient per day, combined with a non CCR1 antagonist drug recommended by the guidelines for hospital-based COVID-19 patients, preferably glucocorticoid, more preferably dexamethasone as described above.

In one embodiment, (a.) the BAY 86-5277 or salt thereof is in a pharmaceutical composition in admixture with a pharmaceutically acceptable carrier, and (b.) glucocorticoid, preferably dexamethasone or salt thereof is in a separate pharmaceutical composition in admixture with a pharmaceutically acceptable carrier.

In one embodiment, (a.) BAY 86-5277 or salt thereof and (b.) a glucocorticoid, preferably dexamethasone or salt thereof are present in a kit, in spatial proximity but in separate containers and/or compositions.

In one embodiment, (a.) BAY 86-5277 or salt thereof and (b.) a glucocorticoid, preferably dexamethasone or salt thereof are combined in a single pharmaceutical composition in admixture with a pharmaceutically acceptable carrier.

In one embodiment, (a.) BAY 86-5277 or salt thereof and (b.) dexamethasone or salt thereof have relative amounts of 10000:1 to 1:1000 by weight, preferably 1000:1 to 1:1 by weight, more preferably about 300:1 by weight.

In one embodiment, the pharmaceutical combination as described herein is characterized in that the BAY 86-5277 and salt thereof is in a pharmaceutical composition in admixture with a pharmaceutically acceptable carrier, and dexamethasone and its salt thereof is in a separate pharmaceutical composition in admixture with a pharmaceutically acceptable carrier. The pharmaceutical combination of the present invention can therefore in some embodiments relate to the presence of two separate compositions or dosage forms in proximity to each other. The compounds in combination are not required to be present in a single composition.

Single preparations, combinational preparations or compositions are known to the skilled person who is able to evaluate compatible carrier materials and formulation forms suitable for both active compounds in the combination. In some embodiments, the quantity of active ingredient that can be combined with the carrier materials to produce a single dosage varies depending on the patient being treated and the particular route of administration.

Further examples of pharmaceutical compositions, pharmaceutical combinations, treatments, subjects, target genes, and targets are described in detail below.

The features of the invention regarding methods of treatment and diagnosis, and descriptions of the composition comprising BAY 86-5277 and/or dexamethasone for use in the treatment of various medical conditions, apply to the composition itself, and vice versa.

### DETAILED DESCRIPTION OF THE INVENTION

### Immune cell and Inflammation

An "immune cell" includes any cell of the vertebrate immune system, including lymphocytes such as B-cells, killer T-cells (i.e., CD8+ T-cells), helper T-cells (i.e., CD4+ T-cells, including Th1 and Th2 cells), natural killer cells, and yδ T-cells, monocytes, macrophages, neutrophils, dendritic cells, mast cells, eosinophils, and basophils.

The term "Inflammation" also refers to the biological response of a tissue to noxious stimuli such as pathogens, damaged cells (e.g., wounds), and/or irritants. The term "inflammatory response" refers to a specific mechanism that causes and regulates inflammation and, in addition to other mechanisms known in the art, activation migration of immune cells, or cytokine production, vasodilatation. Ideally, inflammation is a protective function of the intended body that both eliminates harmful stimuli and initiates the process of healing one or more affected tissues. However, hyperinflammation or chronic inflammation, described herein, is associated with various diseases such as viral infections, e.g. SARS-CoV-2 infection, hay fever, atherosclerosis, rheumatoid arthritis and other diseases known in the medical community. A pulmonary hyperinflammation has several foundations, of which pro-inflammatory chemokines, mainly the imbalance between interleukins and anti-inflammatory cytokines, play a major role.

In some embodiments, the CCR1 antagonist, preferably BAY 86-5277 or salt thereof, modulate inflammatory response of immune cells, or modulate the levels or activities of various inflammatory molecules, among others. In some embodiments, the CCR1 antagonist, preferably BAY 86-5277 or salt thereof, combined with a drug recommend by the guideline for hospital-based patient modulate inflammatory response of immune cells, or modulate the levels or activities of various inflammatory molecules, among others.

In one embodiment, the invention may include, but is not limited to, the modulation of inflammatory responses, including acute and chronic inflammatory responses, systemic inflammatory responses, local inflammatory responses, and inflammatory responses at the cellular level, whether in vivo, ex vivo, or in vitro. Examples of inflammatory response-modulating activities include, without limitation, modulating the growth, activity, or trafficking of various immune cells, and modulating the production or secretion of various cytokines. Hence, embodiments of the present invention include CCR1 antagonists, and derivatives thereof, which modulate inflammation, such as by increasing or decreasing an inflammatory response, and thereby possess therapeutically beneficial activity in the treatment and prophylaxis of diseases or conditions associated with inflammation. In some embodiments, the subjects exhibit, or are at risk for exhibiting, an increased or pathological inflammatory response, or an insufficient inflammatory response.

As used herein, a "therapeutically effective amount" of BAY 86-5277 or salt thereof refers to an amount that is capable of reducing the need for oxygen supplementation, reducing the need for non-invasive ventilation, reducing the need for invasive ventilation, increasing survival, modulating or regulating an inflammatory response or inflammation in a subject in any desired way, as compared to a non-treated subject or to a subject only being treated with a non-CCR1 antagonist drug treatment recommended by the guidelines for hospital-based COVID-19 patients, preferably glucocorticoid, more preferably dexamethasone.

A "therapeutically effective amount" of the a non-CCR1 antagonist drug recommended by the guidelines for hospital-based COVID19 patients, preferably a glucocorticoid, more preferably dexamethasone, refers to an amount that is capable of reducing the need for oxygen supplementation, reducing the need for non-invasive ventilation, reducing the need for invasive ventilation, increasing survival, modulating or regulating an inflammatory response or inflammation in a subject in any desired way, as compared to a non-treated subject. Examples of an inflammatory response-modulating activity include modulating activation of immune cells (e.g. macrophages), reducing migration of immune cells such as granulocytes (e.g., neutrophils, eosinophils) or lymphocytes to selected tissues, preferably the lung. In one embodiment, an inflammatory response-modulating activity includes the modulation of cytokine production. Other examples will be apparent from the description provided herein and the understanding in the art.

The term "modulating" includes "increasing" or "stimulating," as well as "decreasing" or "reducing," typically in a statistically significant or a physiologically significant amount.

The terms "enhance" or "enhancing," or "increase" or "increasing," or "stimulate" or "stimulating," refer generally to the ability of one or agents or compositions to produce or cause a greater physiological response (i.e., downstream effects) in a cell, as compared to the response caused by either no treatment or a non-CCR1 antagonist drug treatment recommended by the guidelines for hospital-based COVID19 patients, preferably glucocorticoid, more preferably dexamethasone. A measurable physiological response may include reduced (hyper)inflammation, reduced CCR1 expression levels, reduced CCL3 expression levels and/or reduced CCL4 expression levels among others apparent from the understanding in the art and the description herein. Among other methods known in the art, single cell analyses represent one way to measure cellular responses to agents provided herein. A measurable physiological response may also include a clinical response, such as the increased number of ventilator-free days, increased number of days without supplemental oxygen, increased number of days of mechanical ventilation, increased number of ICU-free days, increased viral clearance, increased survival, enhanced gas exchange or clinical marker of inflammation.

In some embodiments, clinical parameters for monitoring physiological response comprise WHO clinical progression scale, need for oxygen/invasive ventilation, ventilation rate, inspiratory oxygen fraction (FiO₂), oxygen partial pressure (paO₂), carbon dioxide partial pressure (paCO₂), pH, oxygen application (e.g. nasal, high-flow, non-invasive ventilation, invasive ventilation), ventilation mode on invasive ventilation, ventilation settings on invasive ventilation, Electrocardiography, acute physiology and chronic health evaluation (APACHE), simplified acute physiology score (SAPS), sequential organ failure assessment (SOFA), glasgow coma scale (GCS), screening for delirium, blood pressure, need for vasopressor (amount), lactate, heart rhythm, heart rate, inotrope therapy (other than cardiac glycosides), urine volume per 24h, urea, creatinine, laboratory parameters (e.g. liver: AST (ASAT), ALP (AP), PPT (Quick)/INR, bilirubin, pancreas: lipase; others: CK, leukocytes, PLT, Hb, CRP, PCT, potassium, BNP, troponin)

An "increased" or "enhanced" number is typically a "statistically significant" number, and may include an increase that is 1.1, 1.2, 2, 3, 4, 5, 6, 7, 8, 9, 10, 15, 20, 30 or more times (e.g., 500, 1000 times) (including all integers and decimal points in between and above 1), e.g., 1.5, 1.6, 1.7. 1.8, etc.).

The term "reduce" may relate generally to the ability of CCR1 antagonist solely, preferably BAY 86-5277 or salt thereof, or in combination with a non-CCR1 antagonist drug treatment recommended by the guidelines for hospital-based COVID19 patients, preferably glucocorticoid, more preferably dexamethasone, to "decrease" a relevant physiological or cellular response, such as a symptom of a disease or condition described herein, as measured according to routine techniques in the diagnostic art. Examples include decreased activation of immune cells such as macrophages in the lung, and decreased inflammation of the lung. A measurable physiological response may include the decreased number of ventilator-dependent days, reduced need for oxygen supplementation, reduced number of mechanical ventilation dependent days, decreased number of days at ICU, reduced duration of hospitalization, reduced viral load, decreased mortality, reduced gas exchange impairment, decreased inflammation, as measured, for example, by body temperature, redness, swelling, or clinical marker of inflammation. Relevant physiological or cellular responses (in vivo or in vitro) will be apparent to persons skilled in the art. A "decrease" in a response may be statistically significant as compared to the response produced by no treatment or a non CCR1 antagonist drug treatment recommended by the guidelines for hospital-based COVID19 patients, preferably glucocorticoid, more preferably dexamethasone, and may include, for example, a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

In one embodiment, immune monitoring for evaluating systemic effects of CCR1 inhibition comprise multiplexed assay based on flow cytometry (FACS) to measure CCL2, CCL3, CCL4, IL-6, IL-8 and IL1β simultaneously, transcriptome analysis on single-cell level, transcriptome analysis on multiple-cell level, ELISA and qPCR. In one embodiment, samples for immune monitoring comprise blood, serum, plasma, or cells of the nasopharyngeal area, e.g. nasopharyngeal swabs. The chemokines and cytokines were selected based on observations by the inventors and other studies suggesting these proteins as potential markers for COVID-19 severity and include the known ligands of CCR1.

As used herein, the term "target gene" refers to a nucleic acid sequence coding for a protein involved in immune response and/or inflammation, preferably CCL2, CCL3, CCL4, IL-6, IL-8, IL1ß, and neutrophil elastase.

The expression of the target gene in the sample is compared to the expression of the target gene in a control sample (e.g., a sample of target cells in culture that express the target gene) that is not exposed to a CCR1 antagonist, e.g. BAY 86-5277. A value of 100% may be assigned to the expression of the target gene in a control sample. In particular embodiments, inhibition or reduction of expression of a target gene is achieved when the level of target gene expression in the test sample or test mammal relative to the level of target gene expression in the control sample is about 95%, 90%, 85%, 80%, 75%, 70%, 65%, 60%, 55%, 50%, 45%, 40%, 35%, 30%, 25%, 20%, 15%, 10%, 5%, or 0%. In other words, the CCR1 antagonist (i.e. BAY 86-5277) reduces or inhibits expression of a target gene by at least about 5%, 10%, 15%, 20%, 25%, 30%, 35%, 40%, 45%, 50%, 55%, 60%, 65%, 70%, 75%, 80%, 85%, 90%, 95%, or 100% in a test compared to the level of target gene expression in a control sample or control mammal that has not been exposed to a CCR1 antagonist, e.g. BAY 86-5277.

In addition, as a measure for neutrophil activation, neutrophil elastase concentrations are also determined in serum samples (100 µl per patient and time-point) by ELISA. In one embodiment, nasopharyngeal swabs are collected from subjected and subjected to single-cell RNA sequencing. In addition to routine diagnostics as part of the standard patient care, blood samples for differential blood work, CRP, PCT, and additional cytokine measurements are obtained on Day 1, 4, 7, 10, 14, 21, and 28. Nasopharyngeal swabs (NS) are collected on Day 1, 4, 7, 10, 14, and 21 for single-cell transcriptome analyses and viral load determination.

In one embodiment, samples for immune monitoring for evaluating systemic effects of CCR1 inhibition are also collected every day, every second day, every 3^{rd} day, every 4^{th} day, every 5^{th} day, every 6^{th} day, every week, every second week, every 3^{rd} week, every month.

### Medical Indications

As used herein, the "patient" or "subject" may be a vertebrate. In the context of the present invention, the term "subject" includes both humans and animals, particularly mammals, and other organisms.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In particular, the treatment described herein relates to either reducing or preventing a severe course of a Coronavirus infection or symptoms thereof via the activation of the chemokines CCL3 and CCL4 and their chemokine receptor 1 (CCR1) in immune cells within the respiratory tract. The prophylactic therapy as described herein is intended to encompass prevention or reduction of risk of an acute respiratory distress syndrome in Coronavirus infection, preferably due to a reduced likelihood of hyperinflammation via activation of the chemokines CCL3 and CCL4 and their chemokine receptor 1 (CCR1) in immune cells within the respiratory tract thereof described herein. The interventional therapy as described herein is intended to encompass prevention or reduction of risk of an acute respiratory distress syndrome and death in Coronavirus infection, preferably due to a reduced likelihood of hyperinflammation via activation of the chemokines CCL3 and CCL4 and their chemokine receptor 1 (CCR1) in immune cells within the respiratory tract thereof described herein.

As used herein, a "patient with symptoms of an infectious disease" is a subject who presents with one or more of, without limitation, fever, diarrhea, fatigue, muscle aches, coughing, if have been bitten by an animal, having trouble breathing, severe headache with fever, rash or swelling, unexplained or prolonged fever or vision problems. Other symptoms may be fever and chills, very low body temperature, decreased output of urine (oliguria), rapid pulse, rapid breathing, nausea and vomiting. In preferred embodiments, the symptoms of an infectious disease are fever, diarrhea, fatigue, muscle aches, rapid pulse, rapid breathing, nausea and vomiting and/or coughing. As used herein "infectious disease" comprises all diseases or disorders that are associated with bacterial and/or viral and/or fungal infections.

As used herein, a "patient with symptoms of hyperinflammation", in particular in the respiratory tract, is a subject who presents with one or more of, without limitation, a hyperinflammatory phenotype characterized by elevated pro-inflammatory cytokines compared to a healthy subject, for example that shows an increased incidence of shock, high levels of C-reactive protein (≥75 mg/L or a doubling in 24 h from >50 mg/L), high levels of ferritin (>1500 pg/L), and/or higher risk of mortality.

As used herein, a "patient with symptoms of an acute respiratory distress syndrome" (ARDS) is a subject who presents with one or more of, without limitation, severe shortness of breath, labored rapid breathing, low blood pressure, tachycardia, confusion, extreme tiredness, and/or cyanosis. According to the Berlin definition[1], ARDS is present when the following criteria apply: (i) Respiratory symptoms begin acutely within one week of an event or worse; (ii) Diagnostic imaging (x-ray or CT scan) reveals bilateral diffuse infiltrates that cannot be explained by pleural effusion, atelectasis, or space-occupying lesion alone; (iii) Interstitial edema, resulting in a respiratory failure that cannot be explained by cardiac causes (e.g., acute heart failure) or volume overload; (iv) Horovitz quotient (PaO2/FiO2) ≤ 300 mmHg. (Acute Respiratory Distress Syndrome - The Berlin Definition, JAMA 2012 Jun 20;307(23):2526-33)

An acute respiratory distress syndrome (ARDS) occurs when fluid builds up in the tiny, elastic air sacs (alveoli) in the lungs, comprising a diffuse alveolar damage and acute respiratory failure. Acute lung failure is often associated with multiorgan failure and has a very high mortality.

As used herein, a patient with "symptoms of a viral infection of the respiratory tract" is a subject who presents with one or more of, without limitation, cold-like symptoms or flu-like illnesses, such as fever, cough, runny nose, sneezing, sore throat, having trouble breathing, headache, muscle aches, fatigue, rapid pulse, rapid breathing, nausea and vomiting, loss of taste and/or smell and/or malaise (feeling unwell).

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection.

In embodiments, the patient at risk of developing SARS is an asymptomatic patient that shows no specific symptoms of SARS (yet). An asymptomatic patient may be at risk of developing SARS because the patient has been in contact with a person infected with a SARS Coronavirus. For example, the asymptomatic patient may have been identified as being at risk of developing SARS by a software application (app) that is installed on his smart phone or corresponding (portable) device and that indicates physical proximity or short physical distance to an infected patient that uses a corresponding app on its respective mobile device/smart phone. Other methods of determining contact/physical proximity to an infected person are known to the skilled person and equally apply to the method of the invention.

In some embodiments, the patient that has or is at risk of developing a severe acute respiratory syndrome (SARS) has a coronavirus infection.

Coronaviruses are a group of related viruses that cause diseases in mammals and birds. The scientific name for coronavirus is Orthocoronavirinae or Coronavirinae. Coronavirus belongs to the family of Coronaviridae. The family is divided into Coronavirinae and Torovirinae sub-families, which are further divided into six genera: Alphacoronavirus, Betacoronavirus, Gammacoronavirus, Deltacoronavirus, Torovirus, and Bafinivirus. While viruses in the genera Alphacoronaviruses and Betacoronaviruses infect mostly mammals, the Gammacoronavirus infect avian species and members of the Deltacoronavirus genus have been found in both mammalian and avian hosts.

In humans, coronaviruses cause respiratory tract infections that can be mild, such as some cases of the common cold, and others that can be lethal, such as SARS, MERS, and COVID-19. Coronaviruses are enveloped viruses with a positive-sense single-stranded RNA genome and a nucleocapsid of helical symmetry. The genome size of coronaviruses ranges from approximately 27 to 34 kilobases, the largest among known RNA viruses.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2).

Coronaviruses vary significantly in risk factor. Some can kill more than 30% of those infected (such as MERS-CoV), and some are relatively harmless, such as the common cold. Coronaviruses cause colds with major symptoms, such as fever, and a sore throat, e.g. from swollen adenoids, occurring primarily in the winter and early spring seasons. Coronaviruses can cause pneumonia (either direct viral pneumonia or secondary bacterial pneumonia) and bronchitis (either direct viral bronchitis or secondary bacterial bronchitis). Coronaviruses can also cause SARS.

Advances in nucleic acid sequencing technology (commonly termed Next-Generation Sequencing, NGS) are providing large sets of sequence data obtained from a variety of biological samples and allowing the characterization of both known and novel virus strains. Established methods are therefore available for determining a Coronavirus infection.

Viruses encode a collection of proteins required to ensure self-replication and persistence of the encoding virus. Enzymes for genome mRNA production and genome replication, proteases for protein maturation, proteins for genome encapsidation, and proteins for undermining the host anti-viral responses can be identified conserved protein motifs or domains. Likely because of selective pressures, viral genomes are streamlined and the functional protein content encoded by viruses is much higher than for a cellular organisms. Thus, describing a viral genome by the collection of encoded protein domains is a potentially useful classification method. Viral evolution can therefore be followed and novel strains of coronavirus can be determined based on sequence comparison to known coronavirus strains.

SARS is induced via droplet transmission and replication of the virus could be shown in the upper and lower respiratory tract or gastrointestinal mucosa. In parallel the virus is also capable of directly invading cells of different organs such as liver, kidney, heart and brain. Another distinct mechanism appears to be the direct invasion of the virus in T-cells. A significant number of SARS patients, who suffer COVID-19, have a clinically low concentration of lymphocytes in the blood, also known as Lymphocytopenia. Clinically, patients show respiratory symptoms such as dry cough and shortness of breath, fever or diarrhea. But symptoms associated with acute liver injury, heart injury or kidney injury can also occur. In a less severe state of SARS, patients can show mild or even no symptoms.

Clinical and scientific investigations show that SARS-CoVs bind to the epithelial cells via the Angiotensin converting enzyme 2 receptor (ACE2). ACE2 is a cell membrane linked carboxypeptidase which is expressed in vascular endothelia, kidney, bladder, heart, nasal mucosa, bronchus and lung. As one consequence binding of the virus leads to an epithelial and endothelial cell damage along with vascular leakage, which triggers a secretion of pro-inflammatory cytokines and chemokines. The virus also mediates ACE2 downregulation and shedding which further promotes a dysfunctional renin-angiotensin system (RAS). Once the RAS is disturbed it can lead an inflammatory response and to vascular permeability. Focusing on the respiratory system, ACE2 shedding can lead to pulmonary vascular permeability and subsequently to pulmonary edema.

Older patients (above 60 years), patients with chronic diseases (e.g. cardiovascular diseases, diabetes, cancer, COPD) or immune compromised patients are considered to be at a higher risk to face a severe development of SARS. Smoking and obesity are also considered as risk factors.

Embodiments of a SARS Coronavirus include, without limitation, any coronavirus that induces a SARS or SARS-similar pathology. Particular embodiments include, without limitation, the SARS Coronavirus (SARS-CoV-1) first discovered in 2003 (as described above), the Middle East respiratory syndrome (MERS-CoV) first discovered in 2012, and the SARS-CoV-2, which causes COVID-19, a disease which brought about the 2019-2020 coronavirus pandemic.

The strain SARS-CoV-2 causes COVID-19, a disease which brought about the ongoing 2019-2020 coronavirus pandemic. The disease was first identified in December 2019 in Wuhan, the capital of China's Hubei province, and spread globally. Common symptoms include fever, cough, and shortness of breath. Other symptoms may include muscle pain, diarrhea, sore throat, loss of taste and/or smell, and abdominal pain. While the majority of cases result in mild symptoms, some progress to viral pneumonia and multi-organ failure.

Coronaviruses can be determined by molecular techniques, for example sequence-based analysis, for example by PCR-based amplification of viral genetic material. Genome-wide phylogenetic analysis indicates that SARS-CoV-2 shares 79.5% and 50% sequence identity to SARS-CoV and MERS-CoV, respectively. However, there is 94.6% sequence identity between the seven conserved replicase domains in ORF1ab of SARS-CoV-2 and SARS-CoV, and less than 90% sequence identity between those of SARS-CoV-2 and other-CoVs, implying that SARS-CoV-2 belongs to the lineage of Beta-CoVs.

Similar to other CoVs, the SARS-CoV-2 virion with a genome size of 29.9 kb possesses a nucleocapsid composed of genomic RNA and phosphorylated nucleocapsid (N) protein. The nucleocapsid is buried inside phospholipid bilayers and covered by two di erent types of spike proteins: the spike glycoprotein trimmer (S) that exists in all CoVs, and the hemagglutinin-esterase (HE) only shared among some CoVs. The membrane (M) protein and the envelope (E) protein are located among the S proteins in the viral envelope. The SARS-CoV-2 genome has 5' and 3' terminal sequences (265 nt at the 5' terminal and 229 nt at the 3' terminal region), which is typical of -CoVs, with a gene order 5'-replicase open reading frame (ORF) 1ab-S-envelope(E)-membrane(M)-N-30. The predicted S, ORF3a, E, M, and N genes of SARS-CoV-2 are 3822, 828, 228, 669, and 1260 nt in length, respectively. Similar to SARS-CoV, SARS-CoV-2 carries a predicted ORF8 gene (366 nt in length) located between the M and N ORF genes.

According to Jin et al (Jin et al. 2020), in the initial 41 patients, fever (98%), cough (76%), and myalgia or fatigue (44%) were the most common symptoms. Less common symptoms were sputum production (28%), headache (8%), hemoptysis (5%), and diarrhea (3%). More than half of patients developed dyspnea. The average incubation period and basic reproduction number (R0) were estimated to be 5.2 d (95% CI: 4.1-7.0) and 2.2 (95% CI, 1.4-3.9), respectively.

The pharmaceutical composition of the present invention may be used to treat diseases of the respiratory tract with and without known viral infection involvement, such as acute viral infection including the common cold, and infection due to respiratory syncytial virus, influenza, coronavirus (including SARS) and adenovirus; obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis and chronic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay l0 fever); and nasal polyposis.

### Respiratory support

"Non-invasive respiratory support" and "non-invasive ventilation" refers to the administration of ventilatory support via a face mask or nasal interface to the patient's upper airway. Noninvasive ventilation is used to avoid invasive ventilation. Its use even in acute respiratory failure is possible in some settings.

"Invasive respiratory support" and "invasive ventilation" refers to the administration of ventilatory support via endotracheal tube ot tracheostomy tube. With extracorporeal membrane oxygenation the blood is oxygenated and decarboxylated directly without using the patient's lung function. ECMO can be used in addition or as substitute for invasive ventilation.

### Viral infection and viral load:

As used herein, the term "viral infection" describes a disease state in which a virus invades healthy cells. Viruses hijack use the cell's reproductive machinery to multiply or replicate and finally dissolves the cell, leading to cell death, the release of viral particles and infection of other cells by the newly produced progeny viruses. The term "viral load" refers to a quantitative measure of viral genomes per invaded cell. The determination of viral genomic material may be employed in such a method. A latent infection by certain viruses is also a possible consequence of a viral infection. The term "viral growth" relates to the infection and replication of a virus and the production of viral particles during and after the infection of a host cell.

### Glucocorticoid

In one embodiment, a therapeutic drug recommend by the guideline for hospitalized COVID19 patients used herein may include a glucocorticoid. Corticosteroids (also called glucocorticosteroids or glucocorticoids) are potent anti-inflammatory agents and belong to the corticosteroids, a class of steroid hormones. Glucocorticoids bind to the glucocorticoid receptor, which is present in almost all vertebrate cells. The corticosteroid treatment produce a reduction in the number, activity, and movement of inflammatory cells into the bronchial submucosa, resulting in decreased airway responsiveness. Glucocorticoids are therefore used in medicine to treat diseases associated with a hyperactive immune system. Diseases associated with a hyperactive immune system comprise infections, particularly viral infections, allergies, asthma, autoimmune diseases, and sepsis. Beside natural glucocorticoids, various synthetic glucocorticoids are available.

In one embodiment, the corticosteroids dexamethasone is used. Dexamethasone. Dexamethasone is known to bind in the ligand binding domain of glucocorticoid receptor and compete for binding with endogenous ligands like e.g. Cortisol (Necela, 2003).

In some embodiments, the corticosteroid to be used in the combinations of the invention can be selected from a group of dexamethasone, prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

In one embodiment, corticosteroids can be selected from a group of budesonide, beclomethasone, triamcinolone, dexamethasone, mometasone, ciclesonide, fluticasone, flunisolide, dexamethasone sodium phosphate and esters thereof as well as 6α,9α-difluoro-17α-[(2-furanylcarbonyl)oxy]-11β-hydroxy-16α-methyl-3-oxoandrosta-1,4-diene-17β-carbothioic acid (S)-fluoromethyl ester.

In one embodiment, corticosteroids include budesonide, beclomethasone dipropionate, mometasone furoate, ciclesonide, triamcinolone, triamcinolone acetonide, triamcinolone hexaacetonide and fluticasone propionate optionally in the form of their racemates, their enantiomers, their diastereomers and mixtures thereof, and optionally their pharmacologically-compatible acid addition salts. In one embodiment, the corticosteroids budesonide, beclomethasone dipropionate, mometasone furoate, ciclesonide or fluticasone propionate may also be used.

Any reference to corticosteroids of the present invention includes a reference to salts or derivatives thereof which may be formed from the corticosteroids. Examples of possible salts or derivatives include: sodium salts, sulphobenzoates, phosphates, isonicotinates, acetates, propionates, dihydrogen phosphates, palmitates, pivaiates, farnesylates, aceponates, suleptanates, prednicarbates, furoates or acetonides. In some cases the corticosteroids may also occur in the form of their hydrates.

### Antagonist

A receptor "antagonist", as used herein, is described as a type of receptor ligand or drug that binds to and blocks a receptor, thereby blocking or attenuating a biological response. Antagonists are therefore also called blockers; e.g., alpha blockers, beta blockers.

Antagonists have affinity but no efficacy for their cognate receptors. Binding disrupts the interaction and inhibits the function of an agonist or inverse agonist at the receptors. Binding sites of antagonists comprise the active site or allosteric site on a receptor or a unique binding site that is not normally involved in the biological regulation of the receptor's activity. Binding of an antagonist to a receptor may be reversible or irreversible. Most drug antagonists achieve potency by competing with endogenous ligands or substrates for structurally defined receptor binding sites.

### Chemokine

Chemokines belong to the family of selective chemotatic cytokines and bind to their receptors, such as CCR1, triggering a signaling cascade to migrate or activate immune cells toward the chemokine source. Chemokines therefore play a critical role in the maintenance of host defense as well as in the development of the immune response.

Chemically, the family of chemokine molecules are divided into two groups: the "C-X-C" subfamily and the "C-C" subfamily. The characteristic feature of these two subfamilies is the presence of four cysteine residues at highly conserved positions in the molecules. In the "C-C" chemokine subfamily, the first two residues are adjacent to each other, whereas in the "C-X-C" subfamily, a single amino acid residue separates the cysteine residues. A recent description of a "-C-" chemokine appears to represent a new family of chemokines, as the "-C" chemokine lacks two of the four cysteine residues present in the "C-C" subfamily or the "C-X-C" subfamily.

Functionally, chemokines are classified into homeostatic and inflammatory chemokine groups. Homeostatic chemokines are constitutively produced in certain tissues and are responsible for basal leukocyte migration. Homeostatic chemokines usually include CCL14, CCL19, CCL20, CCL21, CCL25, CCL27, CXCL12, and CXCL13. Inflammatory chemokines are produced under pathological conditions in response to pro-inflammatory stimuli, such as IL-1, TNF-alpha, LPS, or viruses, and actively participate in the inflammatory response by attracting immune cells to the site of inflammation. Examples include: CXCL-8, CCL2, CCL3, CCL4, CCL5, CCL11, CXCL10.

### CCR1, CCR1 antagonist - BAY 86-5277 or salt thereof

The CCR1 chemokine receptor CCR1 (chemokine receptor 1) is highly expressed in tissues affected in different autoimmune, inflammatory, proliferative, hyperproliferative and immunologically mediated diseases e.g. asthma and chronic obstructive pulmonary disease. Moreover, inflammatory cells (e.g. neutrophils and monocytes/macrophages) contribute to the pathogenesis of respiratory diseases such as COPD by secretion of proteolytic enzymes, oxidants and pharmacologic mediators. These cells are dependent on the function of CCR1 for recruitment and activation in lung tissues.

A desirable property for a drug acting at the CCR1 receptor is that it has high, potency e.g. as determined by its ability to inhibit the activity of the CCR1 receptor.

WO01/098273 describes compounds having activity as pharmaceuticals, in particular as modulators of chemokine receptor (especially CCR1 chemokine receptor), salts thereof and pharmaceutical formulations, and their potential use in treating various diseases.

Modulation of chemokine receptor activity is intended to comprise antagonism and/or partial antagonism of activity associated with a particular chemokine receptor, preferably the CCR1 receptor. The term composition, as used herein, comprise a product containing the indicated components in the indicated amounts, as well as any product resulting directly or indirectly from the combination of the indicated components in the indicated amounts.

The term "CCR1 receptor antagonist", as used herein, also refers to a non-peptide CCR1 receptor antagonists.

The non-peptide CCR1 receptor antagonists preferred in the context of the invention are described as piperazine derivatives in U.S. Patent 6,207,665. The non-peptidic CCR1 receptor antagonists may have asymmetric carbon atoms in their structure. The non-peptide CCR1 receptor antagonists may therefore exist as single stereoisomers, racemates, and mixtures of enantiomers and diastereomers. All such single stereoisomers, racemates and mixtures thereof are within the scope of the present invention. The absolute configuration of certain carbon atoms within the non-peptidic CCR1 receptor antagonists, if known, is indicated by the corresponding absolute descriptor R or S. The descriptor "trans" is used to indicate that the R1a substituents are on opposite sides of the piperazine plane. The descriptor "cis" is used to indicate that the R1a substituents are on the same side of the piperazine plane.

In some embodiments, the CCR1 antagonist BAY 86-5277 or salt thereof is used.

A "therapeutically effective amount" refers to that amount of a CCR1 receptor antagonist, preferably BAY 86-5277 or salt thereof, that when administered to a patient, preferably a SARS-CoV-2 infected patient in need thereof, is sufficient to effectuate, in particular, the treatment, as defined below, of hyperinflammation. The amount of non-peptidic CCR1 receptor antagonist that constitutes a "therapeutically effective amount" varies depending on the non-peptidic CCR1 receptor antagonist used, the severity of a subject's COVID19 disease, and/or the age of the treated subject, but may be routinely determined by one skilled in the art in light of his own knowledge and this disclosure.

The compound BAY 86-5277 and compositions thereof are particularly useful in modulating chemokine receptor activity, preferably CCR1. Accordingly, the compound BAY 86-5277, preferably used herein, are particularly inhibits at least one function or property of a mammalian CCR1 protein, e.g., a human CCR1 protein. The ability of a compound to inhibit such a function may be demonstrated in a binding assay (e.g., ligand binding or promoter binding), a signaling assay (e.g., activation of a mammalian G protein, induction of a rapid and transient increase in the concentration of free cytosolic calcium), and/or a cellular response function (e.g., stimulation of chemotaxis, exocytosis, or release of inflammatory mediators by leukocytes).

A CCR1 antagonist is, for example, BAY 86-5277 or salt thereof ((2R)- 1-[[2-[(aminocarbonyl)amino]-4-chlorophenoxy]acetyl]-4-[(4-fluorophenyl)methyl]-2-methylpiperazine monohydrochloride) or a compound disclosed in WO2001/062728 or WO2001/098273, such as N-(2{(2S)-3[{(3R)-l-[(4-chlorophenyl)methyl]-3-pyrrolidiny 1}amino]-2-hy droxypropoxy }-4-fluoropheny l)acetamide, N-(2 {(2S)-3 [{(3S)- 1-[(4-chlorophenyl)methyl]-3-pyrrolidinyl}amino]-2-hydroxypropoxy}-4-fluoropheny l)acetamide, N-(2- {(2S)-3-[1-{(4-chlorobenzoy l)-4-piperidinyl }amino]-2-hydroxypropoxy }-4-hydroxyphenyl)acetamide, (2- {[(2S)-3- {[(2R,5S)- 1-(4-chlorobenzyl)-2,5-dimethylpiperidin-4-yl]amino}-2-hydroxy-2-methylpropyl]oxy}-4-fluorophenyl)acetic, acid, (2-{[(2S)-3-{[(3S,4R)-l-(4-chlorobenzyl)-3-methylpiperidin-4-yl]amino}-2-hydroxy- 2-methylpropy1] oxy}-4-fluoropheny l)acetic acid, (2- {[(2S)-3- {[(3R,4R)- 1-(4-chlorobenzyl)-3-methylpiperidin-4-yl]amino}-2-hydroxy-2-methylpropyl]oxy}-4- fluorophenyl)acetic acid, (2-{[(2S)-3-{[(2R,4S,5S)-l-(4-chlorobenzyl)-2,5-dimethylpiperidin-4-yl]amino}-2-hydroxy-2-methylpropyl]oxy}-4-fluorophenyl)acetic acid, (2-{[(2S)-3-{[(2R,4R,5S)-l-(4-chlorobenzyl)-2,5-dimethylpiperidin-4-yl]amino}-2- hydroxy-2-methylpropyl]oxy}-4-fluorophenyl)acetic acid, (2-{[(2S)-3-{[(2S,4R,5R)-l-(4- chlorobenzyl)-2,5-dimethylpiperidin-4-yl]amino}-2-hydroxy-2-methylpropyl]oxy}-4- fluorophenyl)acetic acid, (2-{[(2S)-3-{[(2S,4S,5R)-l-(4-chlorobenzyl)-2,5-dimethylpiperidin-4-y1] amino}-2-hydroxy-2-methylpropyl]oxy}-4-fluorophenyl)acetic acid, Methyl (2-{[(2S)-3-{[l-(4-chlorobenzyl)piperidin-4-yl]amino}-2- hydroxypropyl]oxy}-4-fluorophenyl)propanoate, N-[2-({2S}-3-[(l- [4-chlorobenzy1] -4- piperidinyl)amino]-2-hydroxypropoxy)-4-chlorophenyl acetamide, N-[2-({2S}-3-[(l-[4- chlorobenzyl]-4-piperidinyl)amino]-2-hydroxy-2-methylpropoxy)-4-hydroxyphenyl] acetamide, N-[2-({2S}-3-[(l- [4-chlorobenzy1] -4-piperidinyl)amino]-2-hydroxy-2- methylpropoxy)-4-fluorophenyl] acetamide, N-[5-chloro-[2-({2S}-3-[(l-[4-chlorobenzyl]-4-piperidinyl)amino]-2-hydroxy-2-methylpropoxy)-4-hydroxyphenyl] acetamide, N-[5- chloro-[2-({2S}-3-[( 1-[4-chlorobenzyl]-4-piperidinyl)amino]-2-hydroxy-2- methylpropoxy)-4-hydroxyphenyl] propaneamide, (2- {[(25)-3- {[1-(4-chlorobenzyl)piperidin-4-yl]amino}-2-hydroxy-2-methylpropyl]oxy}-4-fluorophenyl)methanesulfonic acid, N-5-chloro-(2-{(2S)-3-[l-{(4-chlorobenzyl)-4- piperidinyl }amino]-2-hydroxypropoxy}-4-hydroxypheny l)-N' -cyclopropyl-urea, N-(2- {(2S)-3- [1-{(4-chlorobenzyl)-4-piperidiny1}amino]-2-hydroxypropoxy }-phenyl)-N' -ethylurea, (2S)-l-(2-ethylphenoxy)-3[(l-[4-chlorobenzyl]4-piperidinyl)amino]propan-2-ol, (2S)- 1-[2-(-hydroxyethyl)phenoxy]-2-methyl-3 [( 1-[4-chlorobenzyl]-4- piperidinyl)amino]propan-2-ol, 2-({2S}-3-[(l-[4-chlorobenzyl]-4-piperidinyl)amino]-2- hydroxy-2-methylpropoxy)benzaldehyde, 2-({2S}-3-[(l-[4-chlorobenzyl]-4- piperidinyl)amino]-2-hydroxypropoxy)-N-cyclopropylbenzamide, Methyl 2-( {2S }-3-[( 1- [4-chlorobenzyl]-4-piperidinyl)amino]-2-hydroxypropoxy)-4-fluorobenzoate, N-(2-{[(2S)- 3-(5-chloro-l H,3H-spiro[l-benzofuran-2,4'-piperidin]-r-yl)-2-hydroxypropyl]oxy}-4-hydroxyphenyl)acetamide, N-(2-{[(2S)-3-(5-chloro-l'H-spiro[l,3-benzodioxole-2,4'- piperidin]- 1'-yl)-2-hydroxypropyl]oxy }-4-hydroxyphenyl)acetamide, 2-{[(2S)-3-(5-chlorol'H, 3H-spiro[l-benzofuran-2,4'-piperidin]-r-yl)-2-hydroxypropyl]oxy}-4-hydroxy-N- methylbenzamide, 2-{[(2S)-3-(5-chloro-1H,3H-spiro[ 1-benzofuran-2,4'-piperidin]- 1'-yl)- 2-hydroxypropy l]oxy }-4-hydroxybenzoic acid, N-(2- {[(2 S)-3 -(5-chloro- 1H ,3H-spiro [2- benzofuran- 1,4'-piperidin] -1'-yl)-2-hydroxypropy l]oxy }-4-hydroxypheny l)acetamide; 2-{ [(2S)-3-(5-chloro-l H,3H-spiro[2-benzofuran- 1,4'-piperidin]- 1'-yl)-2- hydroxypropyl]oxy}-4-hydroxy-N-methylbenzamide, N-(2-{[(2S)-3-(5-fluoro-l'H,3Hspiro[ l-benzofuran-2,4'-piperidin]-r-yl)-2-hydroxypropyl]oxy}-4- hydroxyphenyl)acetamide, 2-{[(2S)-3-(5-fluoro-l'H,3H-spiro[l-benzofuran-2,4'- piperidin]-r-yl)-2-hydroxypropyl]oxy}-4-hydroxy-N-methylbenzamide, N-[2-({(2S)-3- [(2R)-5-chloro- 1H,3H-spiro[l -benzofuran-2,3'-pyrrolidin]- l'-yl]-2-hydroxypropyl}oxy)-4- hydroxyphenyljacetamide, N-(2-{[(2S)-3-(5-chloro-l'H,3H-spiro[l-benzofuran-2,4'- piperidin]-l'-yl)-2-hydroxypropyl]oxyl-4-hydroxyphenyl)urea, 4-fluoro-2-{[(2S)-3-(5- fluoro- 1H ,3H-spiro[ 1-benzofuran-2,4'-piperidin]- 1'-yl)-2-hydroxypropyl]oxy }benzoic acid, N-(2- {[(2S)-3-(5-chloro- 1H,3H-spiro[ 1-benzofuran-2,4'-piperidin]- 1'-yl)-2- hydroxypropyl]oxy}-4-fluorophenyl)urea, N-(2-{[(2S)-2-amino-3-(5-fluoro-l H ,3Hspiro[ l-benzofuran-2,4'-piperidin]-r-yl)propyl]oxy}-4-hydroxyphenyl)acetamide, 2-[(2S)- 3-(5-chlorospiro[benzofuran-2(3H),4'-piperidin]-r-yl)-2-hydroxypropoxy]-benzaldehyde, (aS)-5-chloro- a-[[2-(2-hydroxyethyl)phenoxy]methyl]-Spiro[benzofuran-2(3H),4'- piperidine]- 1'-ethanol, (aS)-5-chloro-a-[[2-(hydroxymethyl)phenoxy]methyl]- Spiro[benzofuran-2(3H),4'-piperidine]-l'-ethanol, N-(2-{[(2S)-3-(5-chloro-l H ,3H-spiro[lbenzofuran- 2,4'-piperidin]-r-yl)-2-hydroxypropyl]oxy}-5-chloro-4-hydroxyphenyl)acetamide, 2-Chloro-5- {[(2S)-3 -(5-chloro- 1'H,3H-spiro[l -benzofuran-2,4'-piperidin] -1'-yl)-2-hydroxypropy Ijoxy }-(4- {acetylamino }phenoxy)acetic acid, 5- {[(25)-3- (5-Chloro-r//,3 H-spiro[l-benzofuran-2,4'-piperidin]-r-yl)-2-hydroxypropyl]oxy}-(4- {acetylamino }phenoxy)acetic acid, {2-Chloro-5-{[(25)-3-(5-chloro-r H,3H-spiro[lbenzofuran- 2,4'-piperidin]- 1'-yl)-2-hydroxypropyl]oxy }-4- [(methylamino)carbonyl]phenoxy }acetic acid, 2- {2-Chloro-5-{ [(25)-3 -(5-chloro- 1'H,3Hspiro[ l-benzofuran-2,4'-piperidin]-r-yl)-2-hydroxypropyl]oxy}-4-[(methylamino)carbonyl]phenoxy }-2-methylpropanoic acid, (2-Chloro-5- {[(25)-3-(5- chloro- 1'H,3 H- spiro[ 1-benzofuran-2,4'-piperidin]- 1'-yl)-2-hydroxypropyl]oxy }-4- {[(3.S)-3-hydroxypyrrolidin-l-yl]carbonyl}phenoxy)acetic acid, 5-Chloro-2-{[(25)-3-(5-chlororH,3H-spiro[l-benzofuran-2,4'-piperidin]-r-yl)-2-hydroxypropyl]oxy}-4-(cyanomethoxy)benzoic acid, 2-{[(2S)-3-(5-chloro-l H,3H-spiro[l-benzofuran-2,4'- piperidin]-r-yl)-2-hydroxypropyl]oxy}-5-chloro-4-(2,2-difluoroethoxy)benzoic, 5-Chloro-2-{[(25)-3-(5-chloro- 1'H,3H-spiro[ 1-benzofuran-2,4'-piperidin]-1'-yl)-2-hydroxypropyl]oxy}-4-(3 ,3,3-trifluoropropoxy)benzoic acid, N-(2- {3-[5 -chloro-1H,3H-spiro[l -benzofuran-2,4'-piperidin]- 1'-yl]propoxy}phenyl)acetamide, Methyl 3-(2- {[(2S)-3-(5-chloro- 1'H3H-spiro[ 1-benzofuran-2,4'-piperidin]- 1'-yl)-2-hydroxypropyl]oxy}-4-fluorophenyl)propanoic acid, N-(2- {[(2S)-3-({spiro [indole-2-4'-piperidin]-3 (1H)-one}-1'-yl)-2-hydroxypropyl]oxy}-4-hydroxyphenyl)acetamide, or (2-{[(2S)-3-(5-Chloro-l' H,3Hspiro[l-benzofuran-2,4'-piperidin]-l'-yl)-2-hydroxypropyl]oxyl-4-fluorophenyl)methanesulfonic acid, or a pharmaceutically acceptable salt thereof (such as a hydrochloride, trifluoroacetate, sulphate, (hemi)fumarate, benzoate, furoate or succinate salt)); or CCX634.

### BAY 86-5277 and salts thereof

BAY 86-5277 is the active compound of the potent and selective CCR1 antagonist BX471 (also referred to: ZK 811752 or BAY 3496025 or benzursulfate). This active compound can occur in different salt forms, e.g. as sulfate or hydrochloride. BAY 86-5277 was developed by Bayer AG (formerly by Schering AG, Berlin / Berlex Inc, Montville) and produced as oral drug formulations including immediate release (IR) and extended release (ER) tablet formulations in accordance with Good Manufacturing Practice (GMP). Between 2000 and 2007, the drug was investigated in eight clinical phase I and four clinical phase lla studies in 4 different indications.

Preclinical pharmacology: The compound competitively displaces ligands for CCR1 (e.g., CCL3, CCL5) from the human receptor, preventing CCR1-mediated biological response including chemotaxis of immune cells. BAY 86-5277 has demonstrated activity in numerous preclinical in vitro or in vivo pharmacology and efficacy models such as a rat experimental model of autoimmune encephalitis (EAE), a rabbit renal transplant rejection model, a mouse renal fibrosis model.

Preclinical safety: The compound underwent a comprehensive toxicological program which supported the conduct of the clinical phase I and II program. The toxicological studies include investigations of acute toxicity as well as repeated dose toxicity with intragastric administration for up to 26 weeks in rats and up to 52 weeks in dogs. The compound showed neither mutagenic activity nor reproductive toxicity. The compound was extensively tested in safety pharmacology studies investigating its potential effects on vital organ systems including pulmonary function. The overall results did not reveal effects that are expected to have an impact to the health of humans.

Competition binding studies in human embryo kidney (HEK) cells transfected with either the human or animal CCR1 demonstrated that Bayer's CCR1 antagonist BAY 86-5277 was able to effectively compete with both 125l-labelled MIP-1α and RANTES. The compound bound with high affinity and met the preset criteria for potency. Based on these studies, the compound had a >10,000-fold selectivity for CCR1 compared to all of the GPCR's examined. Furthermore, three in vitro assays demonstrated that BAY 86-5277 is an efficient and effective antagonist of the human CCR1 receptor, e.g. by inhibiting leukocyte migration specifically related to the CCR1 receptor since BAY 86-5277 did not affect the directed migration of cells in response to the ligands of CCR5, CCR2, CXCR1, CXCR2, or CXCR4.

Neutrophils are an important component of the inflammatory response that characterizes acute lung injury (ALI) and their activation plays a central role in the development of most cases of this disease. Blockade of CCR1 by BAY 86-5277 has proven to be beneficial in two different murine models of lung injury and in a model of sepsis (PMID: 26472813).

Pharmacokinetics: The drug underwent a series of preclinical and clinical investigations in vitro and in vivo to characterize pharmacokinetics of BAY 86-5277 and its metabolites in humans as well as in different animal species.
Clinical Phase I. The safety, tolerability, pharmacodynamics and pharmacokinetics of BAY 86-5277 in humans have been investigated in I studies in healthy volunteers. Single dose i.v. as well oral administrations of liquid formulation, IR and ER tablets were found safe with respect to vital signs, ECG and safety laboratory parameters at all doses tested. Single dose administration of the ER formulation was escalated up to 1200 mg without reaching a maximum tolerated dose. When ER formulation was given repeatedly three times per day over 7 days at doses of each 600 mg up to 1200 mg, gastrointestinal adverse events including nausea, diarrhea and vomiting were observed as most common adverse events (table 1). *⁵⁸* 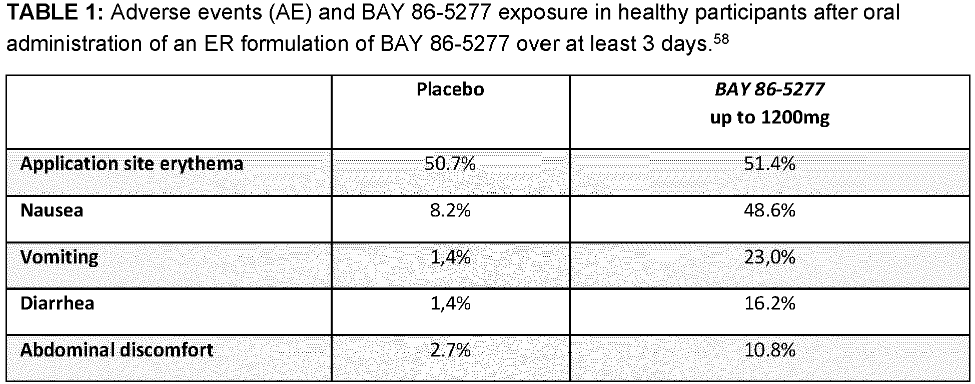
*Clinical Phase II.* Three clinical phase IIa studies in multiple sclerosis, psoriasis, and endometriosis were performed with more than 100 patients receiving the active substance. All studies were conducted with dosing regimens of up to 600 mg BAY 86-5277 orally three times per day over 6 to 12 weeks in in psoriasis or endometriosis, and 16 weeks in multiple sclerosis.

Despite evidence for target engagement in clinical phase I (by inhibition of *ex vivo* MIP1-α-induced CD11b expression in peripheral blood monocytes), the phase II studies could not demonstrate proof-of-concept or evidence for clinical efficacy of BAY 86-5277 in any of these indications. As the consequence, the development of BAY 86-5277 was not continued by Bayer AG beyond 2007.

In clinical phase II studies, BAY 86-5277 was found generally well tolerated and with a safety profile acceptable to patients. Most common AEs were gastrointestinal disorders such as nausea, vomiting and diarrhoea.

Liquid formulation: The BAY 86-5277 liquid formulation contains 60.0 mg active pharmaceutical ingredient (API) per ml solution and has been developed for clinical trials. The IMP is stored in a two-bottle system: Diluent and API (powder) are provided in separate amber glass bottles. The IMP liquid formulation needs to be reconstituted before administration by adding the diluent to the bottle with the API.

Dosing rationale: In humans, plasma concentrations above 100 ng /mL BAY 86-5277 could be reached and could be maintained throughout steady state following three times oral administration of daily 600 mg BAY 86-5277 (ER tablet formulation). With such concentrations, a mean trough CCR1 blockade of at least 90% can be achieved, based on the drug's known receptor-binding and plasma protein-binding properties. The dosing rationale was supported by pharmacodynamic data obtained in a clinical phase I .⁵⁹

Data on (pre)clinical efficacy and safety in other indications. The safety, tolerability, pharmacodynamics and pharmacokinetics of BAY 86-5277 in human have been investigated in phase I studies in healthy volunteers. Single dose i.v. as well oral administrations of drinking solution, IR and ER tablets were found safe in clinical and preclinical studies with respect to vital signs, ECG and safety laboratory parameters at all doses tested. Single dose administration of the ER formulation was escalated up to 1200 mg without reaching a maximum tolerated dose. When ER tablet formulation was given repeatedly over days or weeks, gastrointestinal adverse events including nausea, diarrhea and vomiting were observed as most common adverse events. The dosing rationale was supported by pharmacodynamic data obtained in a clinical phase I study in healthy subjects who were treated with single doses of BAY 86-5277 or salt thereof.^{58,59} In peripheral blood monocytes obtained from blood samples of these subjects, a dose dependent blockade of ex vivo MIP1-α-inducedCD11b expression was found, which returned to baseline after discontinuation of the drug- Overall data on safety and tolerability supported the start of clinical phase II. Clinical Phase II. Clinical phase la studies in multiple sclerosis, psoriasis, and endometriosis were performed in more than 100 randomized patients. All studies were conducted with dosing regimens of 600 mg BAY 86-5277 three times a day per os in patients receiving the active substance. Despite evidence for target engagement in clinical phase I (by inhibition of ex vivo MIP1-α-inducedCD11b expression in PBMCs), the phase II studies could not demonstrate proof of concept or evidence for clinical efficacy of BAY 86-5277 in any of these indications. As the consequence, the development of BAY 86-5277 was not continued by Bayer AG beyond 2007. In all phase II studies, BAY 86-5277 was found generally well tolerated and with a safety profile acceptable to patients. Most common adverse events were gastrointestinal disorders (in 5 to 20 % of patients) such as nausea, vomiting and diarrhoea occurring. Taken data from preclinical, human phase I and phase II studies together, the safety margin of BAY 86-5277 is considered to be sufficiently large to allow an oral administration of the planned 600 mg BAY 86-5277 three times daily (ER formulation). Following three times oral administration of daily 600 to 1200 mg BAY 86-5277, plasma concentrations above 100 ng/mL can be reached within 15min, 30min, 1h, 2h, 3h, 4h, 5h, 6h, 7h, 8h, 9h, 10h, 11h, or 12h after the first dose and can be maintained throughout the entire treatment period. CMC: An oral formulation containing the active substance and pharmaceutical excipients typically used for oral dosage forms is intended to be used for clinical studies. The GMP status of drug substance as well as of drug product manufacturing is at stage of early clinical development (phase I/lla). A CMO is not assigned to manufacture GMP material (drug substance) but might be assigned to support drug product manufacturing or testing.

A salt of the CCR1 antagonist BAY 86-5277 can be BX471 (or BAY 3496025 or ZK811752) and can have the following general molecular formula C₂₁ H₂₄CIFN₄O₃ × H₂SO4 as sulfate with a relative molecular mass of 532.98 g/mol or as hydrochloride the general formula C₂₁H₂₄CIFN₄O₃ × HCl with a relative molecular mass of. 471.3 g/mol. The active compound BAY 86-5277 has the following general molecular formula C₂₁H₂₄ClFN₄O₃ with a relative mass of 434.89 g/mol.

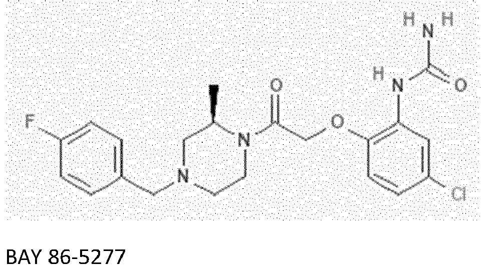

"Pharmaceutically acceptable salt" of the compound of the invention includes both acid and base addition salts. "Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid and the like, and organic acids such as acetic acid, propionic acid, pyruvic acid, maleic acid, malonic acid, succinic acid, fumaric acid, tartaric acid, citric acid, benzoic acid, mandelic acid, methanesulfonic acid, ethanesulfonic acid, p-toluenesulfonic acid, salicylic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, zinc, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic bases are isopropylamine, diethylamine, ethanolamine, trimethylamine, dicyclohexylamine, choline and caffeine.

In one embodiment, BAY 86-5277 is used as N-[5-chloro-2-[2-[(2R)-4-[(4-fluorophenyl)methyl]-2-methyl-1-piperazinyl]-2-oxoethoxy]phenyl]-urea or R-N-[5-chloro-2-[2-[4-[(4-fluorophenyl)meth-yl]-2-methyl-1-piperazinyl]-2-oxoethoxy]phenyl]ureahydrochloric acid salt.

### Guideline - Recommendations for hospital-based therapy of patients with COVID-19

The term "guideline treatment for severe respiratory disease" encompasses any treatment, either via administration of a substance or other treatment, included in a standardized protocol for treatment of severe respiratory disease. For example, guideline treatment for severe respiratory disease encompasses those treatments provided in the "Clinical management of severe acute respiratory infection (SARI) when COVID-19 disease is suspected" as provided by the WHO, dated 13.03.2020. Further examples of "guideline treatments for severe respiratory disease" are those described below, in the context of terms "guideline drug", "drug recommended by the guidelines", and "non-CCR1 antagonist guideline drug".

The terms "guideline drug", "drug recommended by the guidelines", and "non-CCR1 antagonist guideline drug", as used herein, refers to any drug therapy recommended in S1, S2k or S3 guideline.

A guideline is known to be constantly modified and adapted to the current state of medical and scientific knowledge. The term "guideline drug" also comprise any prospective recommendations, provided they do not include a CCR1 antagonist. Preferably, the guideline is used for the treatment of hospitalized COVID19 patients. However, other viral diseases may also have guidelines for the treatment of hospitalized patients suffering from such a disease and their drug therapy recommendations may be included herein.

Guidelines for the treatment of hospitalized COVID19 patients, particularly those with severe COVID19, also comprise guidelines for the treatment of COVID19 patients that is not a guideline as defined in Guideline - Recommendations for Inpatient Treatment of Patients with COVID-19 (AWMF Registry No. 113/001), such as WHO COVID-19 Clinical management: living guidance and Coronavirus Disease 2019 (COVID-19) Treatment Guidelines of the NIH, as amended.

The current S3 guideline (May 2021) covers the entire inpatient care setting comprising current recommendations on drug therapy (tocilizumab, tofacitinib, monoclonal antibodies) and palliative treatment.

Immunosuppressive therapies that constrain the immune system, such as those now based on dexamethasone, to prevent the immune system from overreacting showed beneficial effects in more severe courses of COVID-19. More targeted immunomodulatory approaches, e.g. with antibodies against the IL-6 receptor (tocilizumab, tofacitinib, sarilumab), showed promising results in the REMAP-CAP trial (Angus DC et al. Effect of Hydrocortisone on Mortality and Organ Support in Patients With Severe COVID-19: The REMAP-CAP COVID-19 Corticosteroid Domain Randomized Clinical Trial. Jama. 2020;324(13):1317-29. doi:10.1001/jama.2020.17022), but no effects [e.g. TOCIBRAS (Veiga, V.C. et al. Effect of tocilizumab on clinical outcomes at 15 days in patients with severe or critical coronavirus disease 2019: randomised controlled trial. BMJ 372, n84 (2021).) or even the possibility of an increased risk of adverse effects (e.g. BACC Bay (Stone J.H. et al. Efficacy of Tocilizumab in Patients Hospitalized with Covid-19. N Engl J Med 383, 2333-2344 (2020)))] in other trials.

An S1 guideline on the intensive care treatment of patients with COVID-19 was first published in March 2020 (Kluge S, et al. [Recommendations for critically ill patients with COVID-19]. Medizinische Klinik, Intensivmedizin und Notfallmedizin. 2020;115(3):175-7. doi:10.1007/s00063-020-00674-3). The current S3 guideline was subsequently updated several times for intensive care therapy and expanded in November 2020 as an S2k guideline to include the overall inpatient setting (Kluge S, et al. Recommendations on Inpatient Treatment of Patients with COVID-19. Deutsches Arzteblatt international. 2021;118(Forthcoming). doi:10.3238/arztebl.m2021.0110). The most recent update was as an S3 guideline. For this version of the guideline (May 2021), systematic searches were conducted on the topics of drug therapy (tocilizumab, tofacitinib, monoclonal antibodies) and palliative treatment, and recommendations were agreed upon in the guideline group. Existing recommendations were partially updated and confirmed. Accordingly, this guideline refers to the entire inpatient care sector. For the outpatient setting, we refer to the recommendations of the German Society for General and Family Medicine (Deutsche Gesellschaft für Allgemeinmedizin und Familienmedizin e.V. Neues Coronavirus (SARS-CoV-2) - Informationen für die hausärztliche Praxis. DEGAM S1-Handlungsempfehlung. https://www.awmf.org/leitlinien/detail/ll/053-054.html. IetzterZugriff23.02.2021). The guideline includes recommendations for diagnosis, hygiene measures, measures in acute hypoxemic respiratory insufficiency (oxygen administration, high-flow oxygen therapy, noninvasive ventilation, intubation, extubation, invasive ventilation and adjuvant measures, tracheostomy) as well as measures to be taken in case of circulatory arrest and cardiopulmonary resuscitation and for thromboembolism prophylaxis.

Airway procedures include intubation, mask ventilation, bronchoscopy, open suctioning, videolaryngoscopy, laryngeal mask, coniotomy, manual ventilation, and tracheostomy.

Measures for acute hypoxemic respiratory failure include oxygen administration, high-flow oxygen therapy, or noninvasive ventilation. Therapeutic priorities in the presence of hypoxemia or respiratory insufficiency initially include administration of oxygen via nasal tube, Venturi mask, and high-flow oxygen therapy (HFNC). High-flow oxygen therapy is commonly used for acute hypoxemic respiratory failure and can reduce the need for intubation without significantly affecting mortality compared with conventional oxygen therapy. In cases of progressive deterioration of gas exchange and increased oxygen demand, the indication for CPAP therapy or noninvasive ventilation (NIV) or invasive ventilation should be reviewed. Because rapid deterioration may occur in these patients, continuous monitoring under constant intubation must be ensured. HFNC and NIV should be performed in the ICU in acute hypoxemic insufficiency. In general, a restrictive fluid regimen should be used in patients with acute respiratory failure. The goal in acute hypoxemic respiratory failure in COVID-19 is to ensure adequate oxygenation. An SpO₂ ≥ 90% (> 88% in COPD patients) or a PaO₂ > 55 mmHg should be achieved. The guideline recommends, in patients with COVID-19 and hypoxemic respiratory insufficiency (PaO₂/FiO₂ = 100-300 mmHg) to attempt therapy with high-flow oxygen therapy (HFNC) or CPAP/non-invasive ventilation under continuous monitoring and constant intubation, and to consider intubation and invasive ventilation in patients with COVID-19 and more severe hypoxemia (PaO₂/FiO₂ < 150 mmHg) and respiratory rates > 30/min. When PaO₂/FiO₂ is < 100 mmHg, intubation and invasive ventilation should usually be performed.

After extubation, oxygenation via oxygen face mask is ideally performed.

Invasive ventilation and adjuvant measures: In ventilated patients with COVID-19 and ARDS, tidal volume should be ≤ 6 ml/kg standard body weight, and end-expiratory airway pressure should be ≤ 30 cm H2O. Regarding the setting of positive end-expiratory pressure (PEEP), for patients in the early phase (without classic consolidations, high compliance, expected low recruitable), PEEP setting according to the values of the LOW-FiO2/PEEP table seems reasonable. In patients with severe ARDS and refractory hypoxemia (PaO₂/FiO₂ quotient < 80 or 60 mmHg), the use of veno-venous ECMO is a therapeutic option to stabilize gas exchange.

In the setting of invasive ventilation, tracheostomy can accelerate weaning from the respirator, creating intensive care capacity.

Circulatory arrest may include the use of defibrillation, airway management via endotracheal intubation, cardiopulmonary resuscitation, mechanical chest compression device, and a thrombolytic agent (thromboembolism prophylaxis/anticoagulation).

Rehabilitation: after severe and critical courses of COVID-19, initiation of rehabilitation is an essential component of care. Depending on the need for rehabilitation, for example, indication-specific pneumological, cardiological or neurological rehabilitation, inpatient or outpatient rehabilitation, or psychotherapeutic / psychosomatic treatment may be considered.

A separate chapter is devoted to drug therapy. Anti-infective drug therapy should be used in cases of suspected bacterial community-acquired pneumonia, nosocomial pneumonia or pneumonia, or bacterial co-infection with an extrapulmonary focus, more specifically antibiotic therapy should be initiated. Antiviral and immunomodulatory approaches are pursued as further specific drug therapy, which recommends the corticosteroid dexamethasone according to the current status. As another, tocilizumab can be administered to COVID-19 treatment in COVID-19 patients with progressively severe disease. However, tocilizumab is not suitable in disease with no or low oxygen demand and with existing invasive ventilation. The SARS-CoV-2 specific monoclonal antibody. Bamlanivimab should not be used in adult patients with PCR-proven moderate to severe SARS-CoV-2 infection (WHO scale 4-6) in the inpatient setting. Clinical benefit of bamlanivimab in patients hospitalized for COVID-19 with moderate to severe SARS-CoV-2 infection (WHO scale 4-6) was not demonstrated in the trial (the ACTIV3/TICO trial). In early SARS-CoV-2-infected hospitalized patients without respiratory COVID19 symptoms (< 72 h after first positive PCR and/or < 7 days since symptom onset), with at least one risk factor for a severe course, therapy with SARS-CoV-2 specific monoclonal antibodies can be given. Convalescent plasma should not be used in hospitalized patients with COVID-19. No recommendation can be derived on specific subgroups based on current evidence. A potential for a beneficial effect of lopinavir/ritonavir is not postulated.

### Therapeutically effective

As used herein to refer to the compounds, compositions, combinations and / or dosage forms suitable for use in contact with a subject that produces a result that in and of itself helps to treat and/or cure a medical condition, or for example show other beneficial effect against a medical condition, prevention of onset of a disease, condition or symptom in a patient, inhibition of a symptom of a condition, prevention of progression of the symptom, amelioration of a symptom of a condition, or induction of regression of the disease and/or symptom. The phrase "therapeutically effective" is intended to include, in some embodiments and within the scope of sound medical judgment, some toxicity, irritation, allergic reactions, and/or other problems or complications, but commensurate with a reasonable benefit/risk ratio.

### Treatment

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a particular disease or symptom, or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In particular, the treatment described herein relates to modulating immune response, inflammation, reducing the risk of developing an acute respiratory distress syndrome, viral clearance either by autophagy or by blockade of viral cell entry. Therefore, the physiological response, clinical parameter described herein, viral load may be assessed on a daily basis, every two days, every three days, every four days, every five days, every six days or every week. The prophylactic therapy as described herein is intended to encompass prevention or reduction of risk of SARS-CoV associated acute respiratory distress syndrome, (hyper)inflammation, increase in viral load, e.g. with SARS-CoV-2, due to a blockade of viral cell entry and/or viral replication after treatment with the compounds described herein.

In some embodiments, a time interval between treatment or prevention comprises a period of several hours (2, 3, 4, 5, 6, 7, 8, 9, 10, 11 or 12) several days (2, 3, 4, 5, 6, or 7), several weeks (1, 2, 3, 4, 5, 6, 7, or 8), or months (2, 3, 4, 5, or 6), preferably 4 weeks to 3 months. In some embodiments, the time interval from one treatment or prevention to the next subsequent treatment can be the same, nearly the same or can change.

In some embodiments, the treatment period in which the CCR1 antagonist, preferably BAY 86-5277 or salt thereof, and/or the drug recommended by the guideline are administered to the subject can be >1 day, >2 days, >3 days, >4 days, >5 days, >6 days, >7 days, >8 days, >9 days, >10 days, >11 days, >12 days, >13 days, >two weeks, >three weeks, or >1 month, preferably 10 days.

### Subject

As used herein, the term "subject" refers to a mammal, such as humans, but can also be another animal, such as a domestic animal (e.g. a dog, cat or the like), a farm animal (e.g. a cow, sheep, pig, horse or the like) or a laboratory animal (e.g. a monkey, rat, mouse, rabbit, guinea pig or the like). The term "patient" refers to a "subject" suffering from or suspected of suffering from a viral infection.

In one embodiment, treatment criteria comprise a positive laboratory-confirmed SARS-CoV-2 PCR test, a WHO clinical progression score above 0, and an onset of COVID-19 symptoms < 10 days before treatment. In one embodiment, the WHO clinical progression score may be 0, 1, 2, 3, 4, 5, 6, 7, 8, or 9, preferably 5 or 6. In one embodiment, the onset of COVID-19 symptoms may be <1, <2, <3, <4, <5, <6, <7, <8, <9, <10, <11, <12, <13, <14, <15 days or above, preferably <10 days.

### Host cell and target cell

The term "host cell" and "target cell", as used herein, refers to a single cell or cell culture that may be or has been a recipient of at least one of the agents described herein, individually or in combination. Host cells include progeny of a single host cell, which progeny may not necessarily be completely identical (in morphology or overall DNA complement) to the original parent cell due to natural, random or intentional mutations and/or changes. In one embodiment, the host cell also refers to a cell into which an infectious agent (e.g. a virus) has invaded or is capable of invading.

### Combined administration:

According to the present invention, the term "combined administration", otherwise known as coadministration or joint treatment, encompasses in some embodiments the administration of separate formulations of the compounds described herein, whereby treatment may occur together, within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g. orally by ingesting separate tablets simultaneously. A combination medicament, such as a single formulation comprising multiple agents disclosed herein, and optionally additional anti-viral and/or anti-inflammatory medicaments, may also be used in order to co-administer the various components in a single administration or dosage.

A combined therapy or combined administration of one agent may occur together or precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-24 h of each other and, more preferably, within about 3-12 h of each other, with a delay time of only about 6 h being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

In the meaning of the invention, any form of administration of the multiple agents described herein is encompassed by combined administration, such that a beneficial additional therapeutic effect, preferably a synergistic effect, is achieved through the combined administration of the two agents.

### Pharmaceutical Combination

According to the present invention, a "pharmaceutical combination" preferably relates to the combined presence of a CCR1 antagonist, preferably BAY 86-5277 or salt thereof, with a drug recommended by the guideline for hospital-based therapy of patients with COVID-19, preferably a glucocorticoid, more preferably dexamethasone, i.e. in proximity to one another, or the use of the two components in combined administration. In one embodiment, the combination is suitable for combined administration.

In one embodiment, the pharmaceutical combination as described herein is characterized in that the CCR1 antagonist, preferably BAY 86-5277 or salt thereof, is in a pharmaceutical composition in admixture with a pharmaceutically acceptable carrier, and the drug recommended by the guideline for hospital-based therapy of patients with COVID-19, preferably a glucocorticoid, more preferably dexamethasone, is in a separate pharmaceutical composition in admixture with a pharmaceutically acceptable carrier. The pharmaceutical combination of the present invention can therefore in some embodiments relate to the presence of two separate compositions or dosage forms in proximity to each other. The agents in combination are not required to be present in a single composition.

In one embodiment, the pharmaceutical combination as described herein is characterized in that the CCR1 antagonist, preferably BAY 86-5277 or salt thereof, and the drug recommended by the guideline for hospital-based therapy of patients with COVID-19, preferably a glucocorticoid, more preferably dexamethasone, according to any one of the preceding claims are present in a kit, in spatial proximity but in separate containers and/or compositions. The production of a kit lies within the abilities of a skilled person. In one embodiment, separate compositions comprising two separate agents may be packaged and marketed together as a combination. In other embodiments, the offering of the two agents in combination, such as in a single catalogue, but in separate packaging is understood as a combination.

In one embodiment, the pharmaceutical combination as described herein is characterized in that the CCR1 antagonist, preferably BAY 86-5277 or salt thereof, and the drug recommended by the guideline for hospital-based therapy of patients with COVID-19, preferably a glucocorticoid, more preferably dexamethasone, are combined in a single pharmaceutical composition in admixture with a pharmaceutically acceptable carrier. Combination preparations or compositions are known to a skilled person, who is capable of assessing compatible carrier materials and formulation forms suitable for both agents in the combination.

### Pharmaceutical Compositions and Methods of administration

The present invention also relates to a pharmaceutical composition comprising the compounds described herein. The invention also relates to pharmaceutically acceptable salts of the compounds described herein, in addition to enantiomers and/or tautomers of the compounds described.

The term "pharmaceutical composition" refers to a combination of the agent as described herein with a pharmaceutically acceptable carrier. The phrase "pharmaceutically-acceptable" refers to molecular entities and compositions that do not produce a severe allergic or similar untoward reaction when administered to a human. As used herein, "carrier" or "carrier substance" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions.

The pharmaceutical composition containing the active ingredient may be in a form suitable for oral use, for example, as tablets, chewing tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions. Tablets contain the active ingredient in admixture with non-toxic pharmaceutically acceptable excipients which are suitable for the manufacture of tablets. The tablets may be uncoated or they may be coated by known techniques to delay disintegration and absorption in the gastrointestinal tract and thereby provide a sustained action over a longer period.

In one embodiment, the pharmaceutical composition comprising the CCR1 antagonist, preferably BAY 86-5277 or salt thereof, described herein is supplied to the subject four times daily as liquid formulation, preferably 600 mg BAY 86-5277 per single dose, preferably administered either as drinking solution per oral solution or via a gastric tube.

Administration of the compounds of the invention, or their pharmaceutically acceptable salts, in pure form or in an appropriate pharmaceutical composition, can be carried out via any of the accepted modes of administration or agents for serving similar utilities. Thus, administration can be, for example, orally, nasally, parenterally, topically, transdermally, or rectally, sublingually, intramuscular, subcutaneously, or intravenously in the form of solid, semi-solid, lyophilized powder, or liquid dosage forms, such as for example, tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and a compound of the invention as the/an active agent, and, in addition, may include other medicinal agents, pharmaceutical agents, carriers, adjuvants, etc.

Dosage levels of the order of from about 0.01 mg to about 500 mg per kilogram of body weight per day are useful in the treatment of the indicated conditions. For example, a (hyper)inflammation and/or immune response modulation may be effectively treated by the administration of from about 0.5 mg to about 5 g, preferably 500 mg to about 4 g, more preferably 1 g to about 3 g, even more preferably 2.4 g, of the CCR1 antagonist, preferably BAY 86-5277, per patient per day.

Dosage levels of the order of from about 0.01 mg to about 500 mg per kilogram of body weight per day are useful in the treatment of the indicated conditions with a non CCR1 antagonist drug recommended by the guidelines for hospital-based COVID19 patients, preferably glucocorticoid, more preferably dexamethasone. For example, a (hyper)inflammation and/or immune response modulation may be effectively treated by the administration of from about 0.5 mg to about 1 g, preferably 1 mg to about 500 mg, more preferably 3 mg to about 100 mg, even more preferably 6mg with a non CCR1 antagonist drug recommended by the guidelines for hospital-based COVID19 patients, preferably glucocorticoid, more preferably dexamethasone, per patient per day.

In one embodiment, the pharmaceutical composition comprising dexamethasone described herein is administered to the subject for 10 days with 6 mg/day, preferably administered either as drinking solution per oral solution or via a gastric tube or intravenous.

Another example, (hyper)inflammation and/or an immune response modulation may be effectively treated by the administration of from about 0.5 mg to about 5 g, preferably 0.5 mg to about 4 g, more preferably 1 g to about 3 g, even more preferably 2.4 g, of the CCR1 antagonist, preferably BAY 86-5277, per patient per day, combined with a non CCR1 antagonist drug recommended by the guidelines for hospital-based COVID19 patients, preferably glucocorticoid, more preferably dexamethasone.

The amount of active ingredient that may be combined with the carrier materials to produce a single dosage form will vary depending upon the host treated and the particular mode of administration. For example, a formulation intended for the oral administration of humans may vary from about 5 to about 95% of the total composition. Dosage unit forms will generally contain between from about 1 mg to about 5000 mg of active ingredient. It will be understood, however, that the specific dose level for any particular patient will depend upon a variety of factors including the activity of the specific compound employed, the age, body weight, general health, sex, diet time of administration, route of administration, rate of excretion, drug combination and the severity of the particular disease undergoing therapy. The dosage effective amount of compounds according to the invention will vary depending upon factors including the particular compound, toxicity, and inhibitory activity, the condition treated, and whether the compound is administered alone or with other therapies.

The invention relates also to a process or a method for the treatment of the mentioned pathological conditions. The compounds of the present invention can be administered prophylactically or therapeutically, preferably in an amount that is effective against the mentioned disorders, to a warm-blooded animal, for example a human, requiring such treatment, the compounds preferably being used in the form of pharmaceutical compositions.

In some embodiments, a patient may receive therapy for the treatment and/or management of the viral infection before, during or after the administration of the therapeutically effective regimen of the compound of the invention, or a pharmaceutically acceptable salt thereof. Non-limiting examples of such a therapy include pain management, anti-inflammatory drugs, oxygen supply, antibody therapy, immunotherapy, targeted therapy (i.e. therapy directed toward a specific target or pathway, e.g. immune virus replication, etc.), and any combination thereof. In some embodiments, the patient has not previously received a therapy for the treatment and/or management of virus infection.

### Synergy

To determine or quantify the degree of synergy or antagonism obtained by any given combination, a number of models may be employed. Typically, synergy is considered an effect of a magnitude beyond the sum of two known effects. In some embodiments, the combination response is compared against the expected combination response, under the assumption of non-interaction calculated using a reference model (refer Tang J. et al. (Tang, Wennerberg, and Aittokallio 2015))

Commonly utilized reference models include the Highest single agent (HSA) model (Berenbaum 1989), the Loewe additivity model (LOEWE 1953), the Bliss independence model (BLISS 1939), and more recently, the Zero interaction potency (ZIP) model (Yadav et al. 2015). The assumptions being made in these reference models are different from each other, which may produce somewhat inconsistent conclusions about the degree of synergy. Nevertheless, according to the present invention, when any one of these models indicates synergy between the agents in the combination as described herein, it may be assumed synergy has been achieved. Preferably, 2, 3 or all 4 of these models will reveal synergy between any two agents of the combination described herein.

Without limitation, four reference models are preferred, which can produce reliable results: (i) HSA model, where the synergy score quantifies the excess over the highest single drug response; (ii) Loewe model, where the synergy score quantifies the excess over the expected response if the two drugs are the same compound; (iii) Bliss model, where the expected response is a multiplicative effect as if the two drugs act independently; and (iv) ZIP model, where the expected response corresponds to an additive effect as if the two drugs do not affect the potency of each other.

The most widely used combination reference, and preferred model for determining synergy, is "Loewe additivity", or the "Loewe model" (Loewe 1928; LOEWE 1953; Loewe and Muischnek 1926), or "dose additivity" which describes the trade-off in potency between two agents when both sides of a dose matrix contain the same compound. For example, if 50% inhibition is achieved separately by 1 uM of drug A or 1 uM of drug B, a combination of 0.5 uM of A and 0.5 uM of B should also inhibit by 50%. Synergy over this level is especially important when justifying the clinical use of proposed combination therapies, as it defines the point at which the combination can provide additional benefit over simply increasing the dose of either agent.

As a further example of determining Loewe Additivity (or dose additivity), let *d₁* and *d₂* be doses of compounds 1 and 2 producing in combination an effect e. We denote by *Dₑ₁* and *Dₑ₂* the doses of compounds 1 and 2 required to produce effect e alone (assuming these conditions uniquely define them, i.e. that the individual dose-response functions are bijective). *dₑ₁*/*Dₑ₂* quantifies the potency of compound 1 relatively to that of compound 2. *d₂Dₑ₁*/*Dₑ₂* can be interpreted as the dose of compound 2 converted into the corresponding dose of compound 1 after accounting for difference in potency. Loewe additivity is defined as the situation where *d₁* + *d₂Dₑ₁*/*Dₑ₂* = *Dₑ₁* or *d₁*/*Dₑ₁* + *d₂*/*Dₑ₂* = 1. Geometrically, Loewe additivity is the situation where isoboles are segments joining the points (*Dₑ₁*, *0*) and (*0*, *Dₑ₂*) in the domain *(d₁, d₂).* If we denote by *f₁(d₁), f₂(d₂)* and the dose-response functions of compound 1, compound 2 and of the mixture respectively, then dose additivity holds when *d₁*/*f₁⁻¹ (f₁₂ (d₁, d₂))* + *d₂*/*f₂⁻¹ (f₁₂ (d₁, d₂)) =* 1.

### FIGURES

The invention is further described by the figures. These are not intended to limit the scope of the invention.

### Short description of the figures:

**Figure 1****: Study design of the randomized, double-blind, and placebo-controlled Clinical Phase II Trial.**
**Figure 2****: Illustration of the experimental setup and the patient cohort used in this study.**
**Figure 3****: Identification, distribution and classification of cells from the upper respiratory tract.**
**Figure 4** **Immune-epithelial cell interaction in COVID-19.**
**Figure 5****: Differences between moderate and critical COVID-19 severity are attributed to non-resident macrophage over-expression of cytokines/chemokines and extravasation.**
**Figure 6****: Interaction of chemokines with their** respective **receptors comparing controls, to moderate and critical COVID-19 cases.**
**Figure 7****: Expression levels of CCL2, CLL3 and their receptors CCR1, CCR2, and CCR5 in immune cells of the lower respiratory tract.**
**Figure 8****: Perturbed expression in the CCL3/CCL4-CCR1 axis correlates with disease severity.**

### Detailed description of the figures:

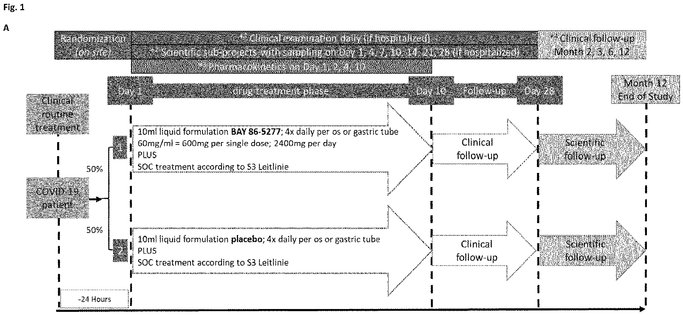

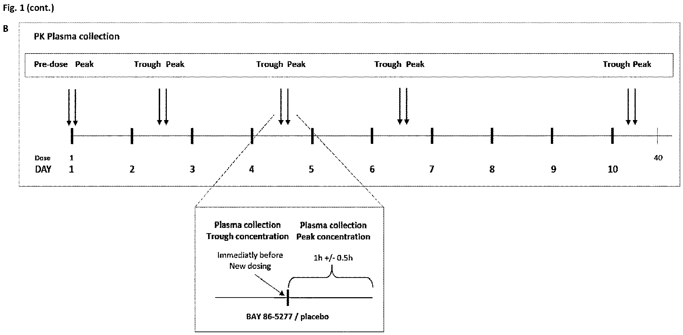

### Figure 1: Study design of the randomized, double-blind, and placebo-controlled Clinical Phase II Trial.

**(A)** In this study, safety and efficacy of the CCR1 antagonist BAY 86-5277 is evaluated in hospitalized COVID-19 patients. Schematic representation of the study design. Hospitalized COVID-19 patients receive adequate treatment directly after admission to the hospital (grey, prior clinical study start) and get informed about the study. Those patients who agree to participate in the clinical trial and sign the ICF are randomized to the two treatment arms, BAY 86-5277 or placebo. The treatment duration is 10 days and combines current standard of care (SOC) with treatment with the investigational drug or placebo and subsequent follow-up until Day 28, after which scientific follow-up continues until Month 12. At study start, 6 mg of dexamethasone for 10 days is the SOC treatment for COVID-19 patients recruited into the study. **(B)** Schematic representation of the sample collection to determine pharmacokinetics of BAY 86-5277. Samples are taken at a maximum of 4 time points (Day 1, 2, 4 and 10). *1 Daily hospital visits are conducted to monitor vital signs (e.g, blood pressure, heart rate, respiratory rate, blood oxygen percentage) until discharge. *2 Blood draws are performed on Day 1, 4, 7, 10, 14, 21, and 28 to determine whether the treatment reduces inflammatory blood markers. Nasopharyngeal swabs are taken on Day 1, 4, 10, and 21 for the evaluation of the viral load over time and scRNA-Seq analysis. *3 Pharmacokinetics is performed using EDTA plasma from a sub-cohort during the initial phase of the study. *4 A clinical follow-up study of two questionnaires is conducted. No additional site visits are required. [os = oral solution]

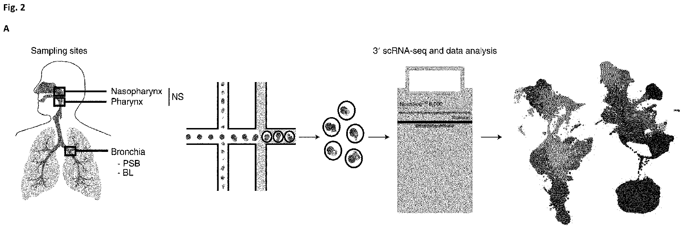

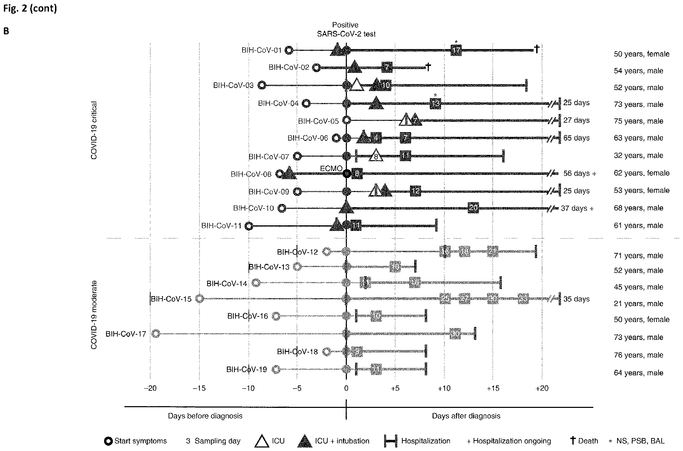

### Figure 2: Illustration of the experimental setup and the patient cohort used in this study.

**(A)** Schematic representation of the experimental workflow. Depicted are the sampling sites (left) and the 3' scRNA-seq library preparation using 10X Genomics (middle) followed by data analysis revealing cell type identity (right). **(B)** Overview of the patient cohort. Given are age, sex and COVID-19 severity as well as onset of symptoms, hospitalization duration and sampling time points in days after onset of symptoms, with all patients being temporally aligned to the day of positive SARS-CoV-2 test. Admission to the ICU is also depicted if applicable. One patient required ECMO. We obtained NSs from all patients and, in addition, PSBs and BLs from patients BIH-CoV-01 and BIH-CoV-04 (marked with *). The sampling day relative to the onset of symptoms is given as a number in a square or triangle. PSB., protected specimen brush, NS. Nasopharynx/Pharynx.

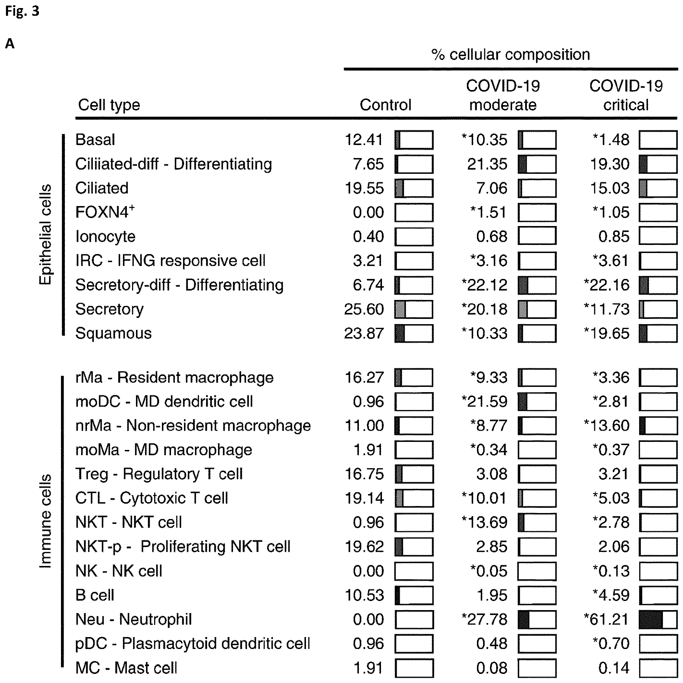

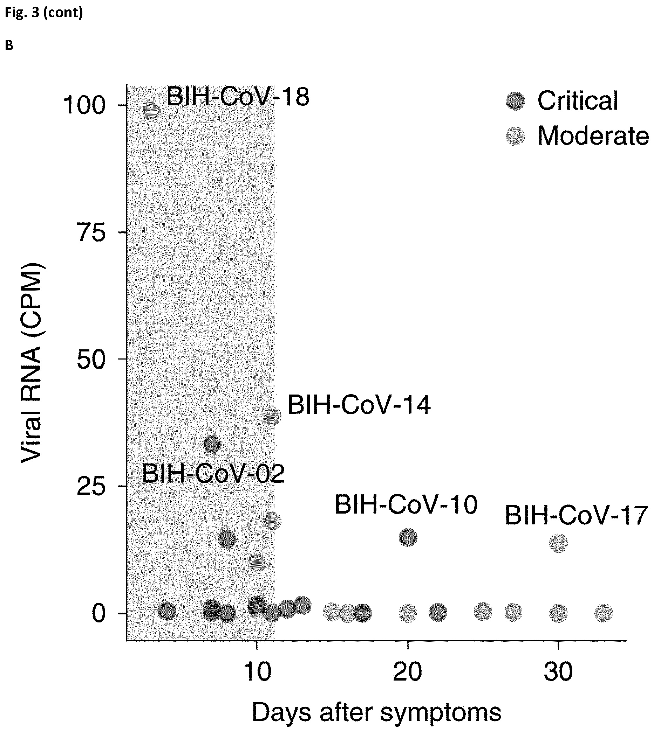

### Figure 3: Identification, distribution and classification of cells from the upper respiratory tract.

**(A)** Table displaying the total contribution of each cell type aggregated for the controls. COVID-19 moderate and critical cases in percentage of total epithelial and immune cell types, respectively. Significant differences compared to control samples as calculated by logistic regression followed by Tukey's post hoc test and Benjamini-Hochberg correction (P < 0.05) are indicated by *. (**B**) Viral RNA reads within aggregated pseudo-bulks for each patient sample in CPM against days after symptoms. Typically, only very low numbers of viral RNA reads were detected 11 d after first symptoms and later. NS samples were used in this analysis: n = 5 controls, n = 8 patients with moderate COVID-19 and n = 11 patients with critical COVID-19. PSB., protected specimen brush, NS. Nasopharynx/Pharynx.

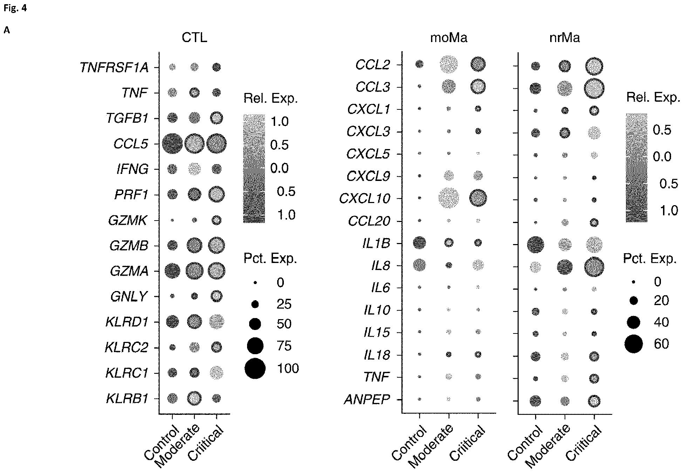

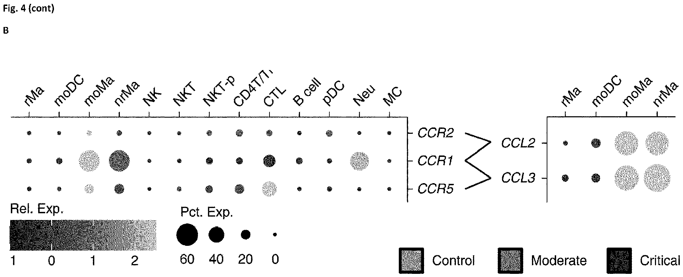

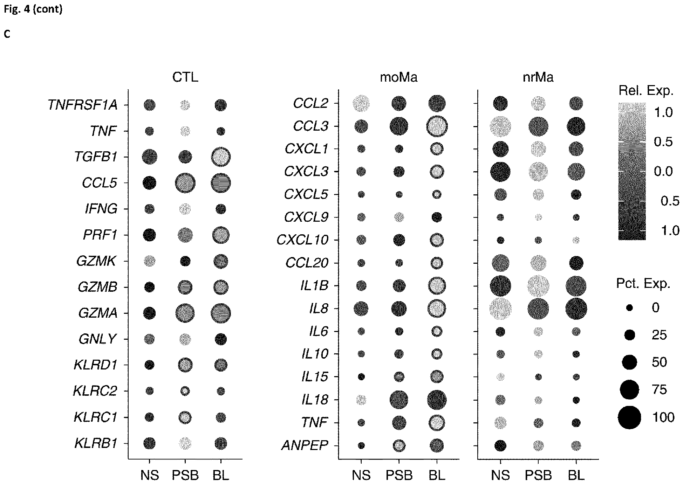

### Figure 4: Immune-epithelial cell interaction in COVID-19.

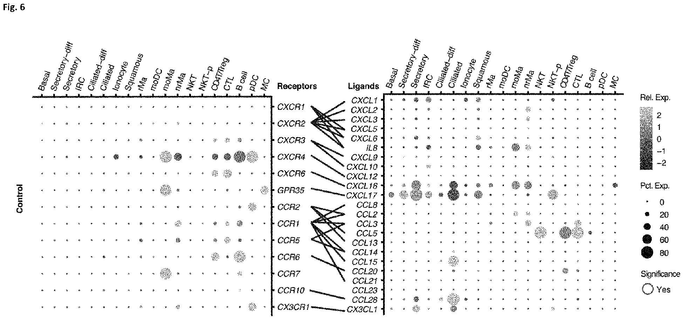

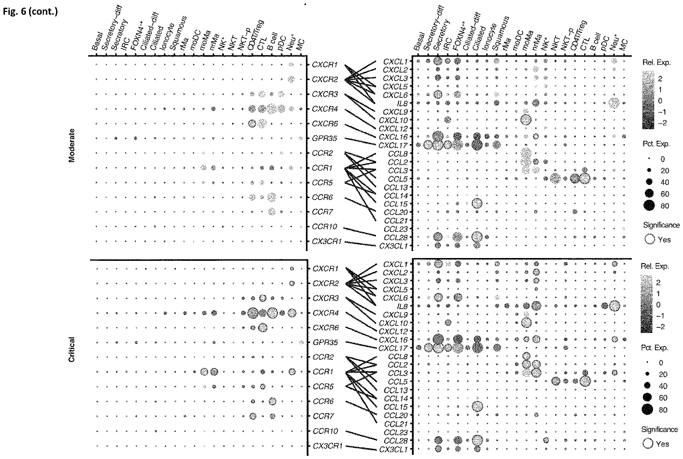

(**A**) Dot plots of pro-inflammatory and cytotoxic mediators in CTL, moMa and nrMa. Significantly altered expression (Benjamini-Hochberg adjusted two-tailed, negative-binomial P < 0.05) in critical versus moderate (circles around critical) and moderate versus controls (circles around moderate) is marked by a black circle. (**B**), Dot plots of chemokine/receptor expression in the different immune cells of patients with critical COVID-19 (n = 11). Scaling according to Fig. 6. (C). Dot plots of inflammatory and cytotoxic mediators from NS, PSB and BL collected from patients with critical COVID-19 (BIH-CoV-01 and BIH-CoV-04). Visualization as in c. g, CASP3 expression in immune and epithelial cell subtypes of these patients. Comparing PSB versus NS (top) and BL versus NS (bottom). Significant decreases (nasal versus other sampling site) are indicated. Rel. Exp., relative gene expression; Pct. Exp., percentage of cells expressing the gene, PSB., protected specimen brush, NS. Nasopharynx/Pharynx.

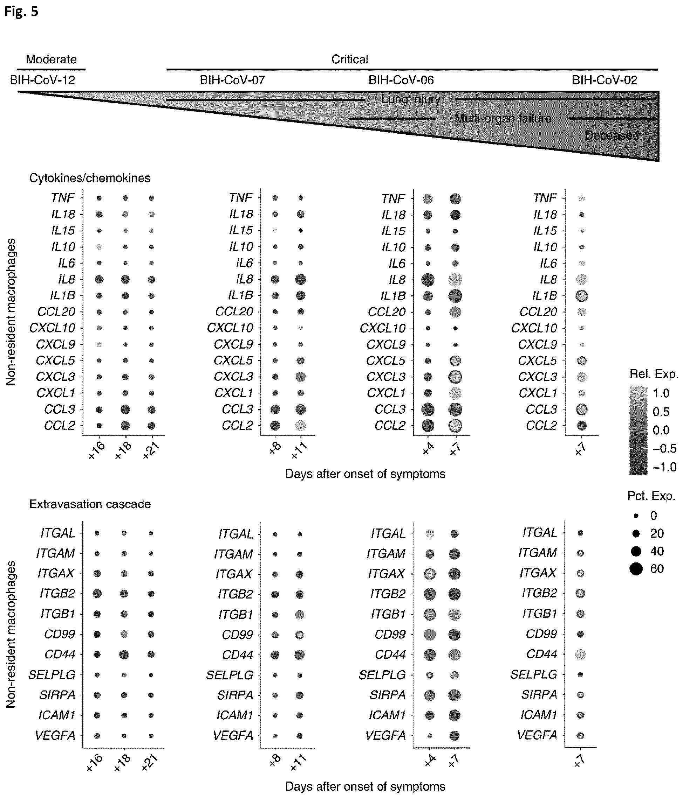

### Figure 5: Differences between moderate and critical COVID-19 severity are attributed to non-resident macrophage over-expression of cytokines/chemokines and extravasation.

Dot plots depicting the average expression of specific cytokines/chemokines and extravasation cascade genes of patients with moderate (BIH-CoV-12) and critical (BIH-CoV-02, BIH-CoV-06 and BIH-CoV-07) COVID-19, ordered by severity as indicated by different clinical features. The sampling days per patient are reflected by 'days after onset of symptoms'. Expression levels are grayscale coded; the percentage of cells expressing the respective gene is size coded. Significant changes for each time point in the patients with critical COVID-19 versus an average of the three time points of the patient with moderate COVID-19 (BIH-CoV-12) (left panel) are marked by a black circle (Benjamini-Hochberg adjusted two-tailed, negative-binomial P < 0.05). Rel. Exp., relative gene expression; Pct. Exp., percentage of cells expressing the gene.

### Figure 6: Interaction of chemokines with their respective receptors comparing controls, to moderate and critical COVID-19 cases.

Expression of chemokines and their respective receptors in the different cellular subpopulations is depicted by dot plots. Connecting lines represent receptor/ligand pairs as obtained from the molecular cell atlas of the human lung 65 complemented by literature research. Expression levels are grayscale coded while the percentage of cells expressing the gene are depicted by the different dot sizes. Black circles show significance (Benjamini-Hochberg adjusted two-tailed negative-binomial p-value < 0.05) of expression when comparing critical and moderate patients (critical panels) or moderate patients and controls (moderate panels) as calculated by a two-sided significance test. (n = 5 moderate and 6 critical COVID-19 patients, 10 samples each). Nasopharyngeal swab samples were used in this analysis: n = 5 control samples, n = 14 moderate COVID-19 samples, and n = 13 critical COVID-19 samples. Rel. Exp. = relative gene expression, Pct. Exp. = percentage of cells expressing the gene.

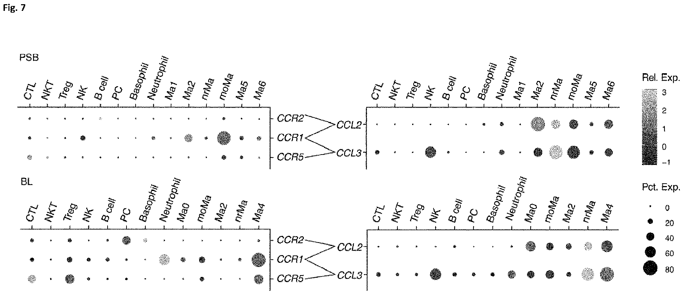

### Figure 7: Expression levels of CCL2, CLL3 and their receptors CCR1, CCR2, and CCR5 in immune cells of the lower respiratory tract.

Dot plot depicting the average expression of CCL3/CCR1, CCR5 and CCL2/CCR2, CCR1 in different immune cells in PSB and BL samples for patients BIH-CoV-01 and BIH-CoV-04. Connecting lines represent receptor/ligand pairs. Expression levels are grayscale coded while the percentage of cells expressing the gene are depicted by different dot sizes. PSB - protected specimen brush, BL - bronchial lavage. Rel. Exp. = relative gene expression, Pct. Exp. = percentage of cells expressing the gene. PSB, protected specimen brush, NS, Nasopharynx/Pharynx.

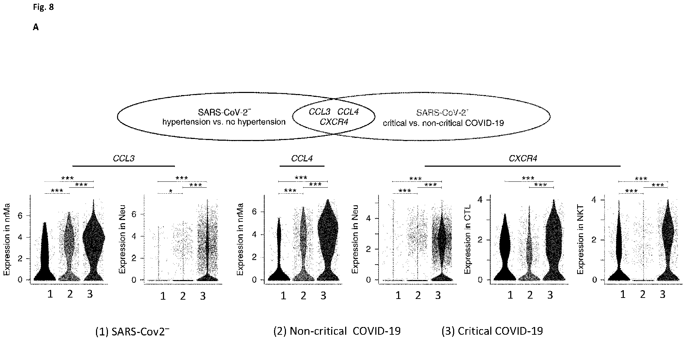

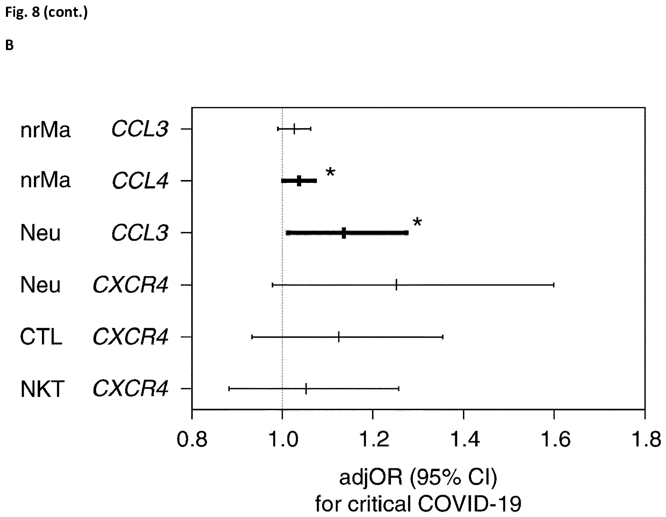

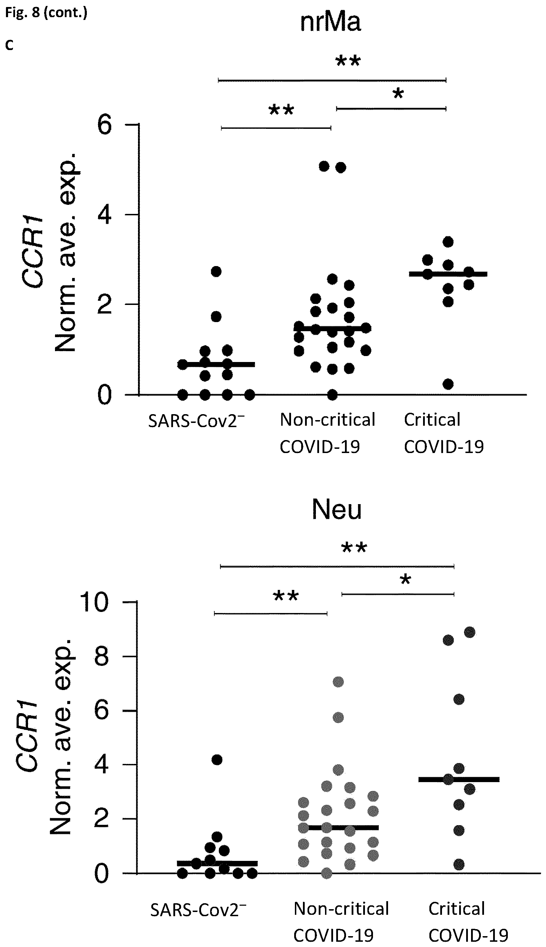

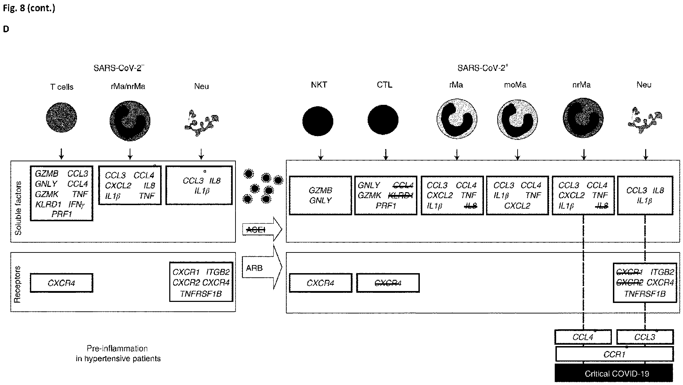

### Figure 8: Perturbed expression in the CCL3/CCL4-CCR1 axis correlates with disease severity.

(**A**) Schematic layout of the overlay between differentially expressed genes (DEGs) in hypertensive/non-hypertensive patients and DEGs between critical/non-critical patients in all immune cell subtypes. For the genes found in the overlap, violin plots depict expression in patients negative for SARS-CoV-2 and non-critical/critical patients positive for SARS-CoV-2. Significant differences are based on Benjamini-Hochberg adjusted P values calculated using MAST. (**B**) Adjusted logistic regression analyses of all genes and cell populations found in this overlay in association to critical COVID-19 (confounder: age, gender, days after onset of symptoms and study center). The forest plot shows adjusted odds ratios with whiskers representing the 95% CI. Significant relationships are depicted in bold. (**C**) Not only CCL3 and CCL4 were significantly associated with severity but also their receptor CCR1. Shown are mean expression levels per patient in nrMA (non-resident macrophages) and Neu (neutrophils), respectively. Significances were derived from a two-sided Mann-Whitney U test. (**D**) The left panel summarizes all genes related to immune mediators and receptors that we found to be elevated in HT+/CVD+/- patients negative for SARS-CoV-2 compared to HT-/CVD- patients independent of ACEI/ARB treatment. The right panel shows those genes significantly upregulated in patients positive for SARS-CoV-2 when comparing ACEI+ (n = 10 underlined) to ARB+ (n = 15,non-marked). Genes associated with critical COVID-19 are marked with °. *P < 0.05, **P < 0.005, ***P < 0.0005.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

Described in more detail below is:
- Clinical trial protocol
- Methods, study design and clinical characteristics of patient cohorts analyzed
- Significant association between interaction between immune cells and epithelial cells that is likely to contribute to the clinical observations of heightened inflammatory tissue damage, lung injury and respiratory failure
- significant correlation of the clinical course of COVID19 disease with changes in the cellular and transcriptional landscape
- a significant induction of CCL2 and CCL3 expression in macrophages together with an increased expression of CCR1, the receptor for both chemokines, in patients with critical COVID-19.
- providing a mechanistic schematic diagram demonstrating CCR1 as promising anti-inflammatory targets in COVID-19.

### Example 1: Clinical trial protocol for a CCR1 antagonist treatment in patients hospitalized with COVID-19 - A multi-centric, randomized, double-blind, and placebo-controlled clinical phase II trial.

The clinical manifestation of coronavirus disease 2019 (COVID-19) ranges from asymptomatic or mild symptoms to critical illness with acute respiratory distress syndrome (ARDS). Approximately one-quarter of patients hospitalized with COVID-19-associated pneumonia require respiratory support in an intensive care unit (ITS). Severe manifestations of COVD-19, particularly with the need for invasive mechanical ventilation (IMV), are associated with high mortality. This severe expression of COVID-19 is associated with an ineffective early antiviral response as well as an ineffective adaptive immune response, which can result in hyperinflammation. This hyperinflammation can cause tissue damage, leading to the development of ARDS and/or multiorgan failure, ultimately associated with an increased risk of mortality. The only current recommended therapeutic option in patients with severe COVID-19 progression is the administration of dexamethasone and standard supportive ARDS therapy with the use of prone positioning.

This study includes hospitalized COVID-19 patients on supplemental oxygen, but not yet IMV, who are classified as stage 5-6 according to the COVID-19 WHO clinical progression scale.

### Objectives and endpoints

Administration of BAY 86-5277 reduces the immune response to infection, thereby reducing the risk of associated secondary complications, such as ARDS and multiple organ failure. The primary objective is to reduce the risk of critical illness progression in hospitalized, oxygen-dependent, or noninvasively ventilated-COVID-19 patients.

Primary endpoints include ventilator-free days during the clinical trial. Mechanical ventilation is one of the key clinical features of a severe or critical COVID-19 course, as well as a surrogate parameter for the utilization of intensive care resources. Thus, the number of ventilator-free days in COVID-19 patients in this phase II clinical trial provides a good measure for assessing disease progression, such as improved prognosis, if the cohort of patients receiving BAY 86-5277 has more ventilator-free days than the placebo group. Mortality enters the assessment as "zero ventilator-free days."

### Other clinical objectives (secondary objectives)

In addition to the primary objective and endpoint, other clinical parameters are collected and documented for a more detailed assessment of the clinical course under BAY 86-5277 or placebo treatment.

### Additional clinical endpoints (secondary endpoints) comprise:

1. WHO clinical progression scale at day 28;
2. Severity of gas exchange disturbance (ratio paO₂/FiO₂);
3. Duration of ventilation;
4. Duration of mechanical ventilation (if applicable);
5. ITS-free days on day 28;
6. Duration of hospitalization
7. Sepsis-related Organ Failure Assessment day 1, 10, 28;
8. Mortality (date and cause).

Besides the careful and intensive clinical monitoring, additional scientific analyses are performed to define the pharmacokinetics of the BAY 86-5277 as liquid solution in COVID-19 patients and to better understand the immunomodulatory effect of CCR1 blockage by BAY 86-5277.
(1) Documentation of clinical endpoints (primary and secondary) at Day 60 as recommended by the FDA for pivotal studies,
(2) Scientific analyses at Days 1, 4, 7, 10, 14, 21, and 28, and at (2), 3, 6, and 12 months comprises analysis of cellular response to CCR1 antagonist BAY 86-5277.
   (i) Immune monitoring comprising measurements of chemokines and cytokines (FACS/ELISA) for evaluating the systemic effects of CCR1 inhibition: Immune parameters are assessed for all samples with methods using less than 200 µl of serum per patient. A multiplexed assay based on flow cytometry (FACS) is used to measure CCL2, CCL3, CCL4, IL-6, IL-8 and IL1β simultaneously with a necessary serum volume of 50 µl per patient and time-point. In addition, as a measure for neutrophil activation, neutrophil elastase concentrations are determine in serum samples (100 µl per patient and time-point) by ELISA.
   (ii) Longitudinal single cell transcriptome analyses of upper respiratory tract cells (nasopharyngeal swabs): To determine the effect of CCR1 inhibition on the primary site of SARS-CoV-2 infection - the upper airways - with minimal burden for the patient, cells of the nasopharyngeal area are collected by nasopharyngeal swabs and their transcriptional response is evaluated. As such, effects of CCR1 inhibition in this area sensitive to SARS-CoV-2 entry is evaluated and potential differences in the longitudinal cellular response of the different subpopulations are characterized. Nasopharyngeal swabs are collected from all patients. However, only for a subset of patients, nasopharyngeal samples are subjected to single-cell RNA sequencing (scRNA-Seq). To ensure for equal numbers of patients form the placebo- and BAY 86-5277-treated group in this sub-cohort, the analysis is performed in an unblinded manner.
   (iii) Pharmacokinetic analyses are performed and side effects [=(serious) adverse events] are monitored to assess the tolerability of BAY 86-5277 . In the initial phase of the study, EDTA plasma samples are collected from a subgroup of patients to determine pharmacokinetics (PK) of BAY 86-5277 as liquid solution. Samples are analyzed for total drug concentration and fraction unbound by a central laboratory.

### Study design

The clinical phase II trial is conducted in compliance with this protocol, Good Clinical Practice (GCP) guidelines, and all applicable regulatory requirements. The trial starts with approval by the relevant Institutional Review Board (IRB; ethics vote) and competent authority (CA). Blinding is accomplished by the coding of the investigational drug product BAY 86-5277 and placebo prior to shipment. The study teams at the four study sites does not have knowledge of whether the vial contains BAY 86-5277 or the placebo.

Figure 1a shows the overall workflow of the clinical phase II trial. In the clinical phase II trial, only patients with a confirmed SARS-CoV-2 infection and a 5-6 score on the COVID-19 WHO clinical progression scale are enrolled. We enroll the following patient cohorts:
- directly after admission to hospital: COVID-19 patient with symptoms according to WHO clinical progression scale 5-6
- hospitalized COVID-19 patients with initially moderate COVID-19-related symptoms (below WHO clinical progression scale 5) who reach WHO clinical progression scale 5-6 overtime and during hospitalization.
- Patients hospitalized for any other reason than COVID-19-related symptoms who are either infected with SARS-CoV-2 during hospitalization or develop first COVID-19 related symptoms during hospitalization are excluded.

All Subjects qualifying for the clinical phase II trial are randomized at the clinical study site to participate either in the placebo arm or the BAY 86-5277 arm of the study. The double-blind character of the study diminishes knowledge about the assignment to one of the treatment arms for the study team and the subjects themselves. All Subjects of the clinical trial also receive the recommended Standard of Care (SOC). Currently, the SOC is dexamethasone.

SOC and either the IMP or the placebo are administered for 10 days. SOC is administered as recommended (currently 1x daily 6 mg per os or i.v.), while the IMP or placebo is administered 4x daily per os. or via a gastric tube. [os = oral solution; i.v. = intra venous]

Subjects can receive dexamethasone as SOC up to three days prior to inclusion and initiation of the IMP/placebo treatment phase of CATCOVID. To ensure the simultaneous administration of SOC and IMP/placebo over the entire treatment phase for all patients in this study, dexamethasone administration is elongated in these patients as follows:
- Day 11 and Day 12: 4 mg daily per os or i.v.
- Day 13 and Day 14: 2 mg daily per os or i.v.
- no dexamethasone administration from Day 15 onwards

The patients are monitored in a clinical follow-up phase until Day 28. During that period, subjects are assessed daily while hospitalized. Patients are expected to be hospitalized for at least 10 days and most of the COVID-19 patients for the entire clinical trial phase until Day 28. Discharged patients are asked to attend study visits at Day 28 as the last day of the clinical trial.

The primary objective of the study is the reduction of a critical disease progression in hospitalized COVID-19 patients with the need for oxygen supplementation. Mechanical ventilation is one of the key clinical features of severe COVID-19. The survey of ventilator-free days, i.e. the reduced need for mechanical ventilation, allows an assessment of whether BAY 86-5277 reduces progression to more severe courses of COVID-19. The primary endpoint is the number of ventilator-free days during the study period of 28 days. Death is counted as "zero ventilator-free days".

Pharmacokinetics: In the initial phase of the study, EDTA plasma samples are collected of a subgroup of subjects to determine pharmacokinetics (PK) of BAY 86-5277. Blinding is maintained. The subgroup consists of 24 patients, each 12 patients from the IMP and placebo arm. The subgroup is defined by the CTO Charité who has access to the patient data in an unblinded fashion. The EDTA plasma samples of all patients are collected for PK analysis of the IMP-treated samples. The first PK sample is obtained on Day 1 before administration of the initial dose (pre-dose). To determine peak concentrations plasma samples is collected 1h +/- 0.5h after IMP administration. Trough concentrations are determined from plasma samples taken right before an IMP dose is given. Plasma samples are obtained on Day 1, 2, 6 and 10 (see Figure 1b for details). PK peak and trough samples can be taken at any time. The exact date and time of blood draw is recorded in the electronic case report form.

Samples are analyzed for total drug concentration and fraction unbound by a central laboratory. Processing, storage and shipment of the samples are described in detail in a standard operating procedure (SOP). If sample collection and processing during off-hours is not practically feasible, it is not considered a protocol deviation if not all samples can be taken.

Chemokine/Cytokine measurements and single cell transcriptomics: In addition to routine diagnostics as part of the standard patient care, blood samples for differential blood work, CRP, PCT, and additional cytokine measurements are obtained on Day 1, 4, 7, 10, 14, 21, and 28. Nasopharyngeal swabs are collected on Day 1, 4, 7, 10, 14, and 21 for single-cell transcriptome analyses and viral load determination.

Long-term clinical follow-up studies: Individual Subjects are monitored until Month 12 in follow-up studies using the SF36 questionnaire and a questionnaire evaluating Long-COVID. Subjects are interviewed via phone, no site visits are required. Questionnaires are surveyed for day 60 (as recommended by the FDA), month 3, 6, and 12.

### Clinical assessments

Administration schema: The treatment period is 10 days in which either the investigational drug product or the placebo is administered. Each treatment arm receives the respective medication (BAY 85-5277 or placebo) 4x daily as 10 ml liquid formulation (BAY 86-5277: 60 mg/ml = 600 mg per single dose), administered either as drinking solution per os or via a gastric tube.

At the same time, all subjects (BAY 85-5277 - and placebo-arm of the study) receives the SOC treatment, currently dexamethasone, according to the S3 Guideline: Dexamethasone is administered for 10 days with 6 mg/day per os or intravenous.

Clinical parameters: (1) Daily monitoring of COVID-19 progression comprise (i) WHO clinical progression scale, (ii) need for oxygen/invasive ventilation, ventilation rate, FiO₂, paO₂, paCO₂, pH, (iii) oxygen application: nasal, high-flow, non-invasive ventilation, invasive ventilation, (iv) ventilation mode and settings (if on invasive ventilation), (v) Sepsis-related Organ Failure Assessment, Simplified Acute Physiology Score
(2) Daily monitoring of clinical status/organ failure comprise (i) brain: GCS, screening for delirium; (ii) circulation: blood pressure, need for vasopressor (amount), lactate; (iii) heart: rhythm, heart rate, inotrope therapy (other than digitoxine); (iv) kidney: urine volume /24h (if available), urea, creatinine; (v) liver: AST (ASAT), ALP (AP), PPT (Quick)/INR, bilirubin; (vi) pancreas: lipase, (vii) other: CK, Leukocytes/PLT/Hb/CRP/PCT, K+; (viii) standard set of routine laboratory parameters If clinically indicated, a 12-lead ECG is performed and BNP and troponin are determined.

### Safety assessment

An adverse event is any untoward medical occurrence in a subject to whom a medicinal product is administered and which does not necessarily have a causal relationship with this treatment. An adverse event could be abnormal laboratory parameters, diseases, signs or symptoms, which occur or worsen after enrolment of the patient in the clinical trial. Adverse reactions are all untoward and unintended responses to an investigational medicinal product related to any dose administered.

A serious adverse event or serious adverse reaction is any untoward medical occurrence or effect that at any dose:
- results in death,
- is life-threatening,
- requires hospitalization or prolongation of existing hospitalization,
- results in persistent or significant disability or incapacity,
- results in a congenital anomaly or birth defect,
- or is another medically important event.

Adverse events and severe adverse events which are characteristic for severe and critical disease progression of COVID-19 patients are excluded from the notification requirement. All events have to be documented.

Adverse event are evaluated to determine:
- the severity grade (assessment of intensity),
- whether it constitutes a SAE (assessment of seriousness),
- its relationship to the investigational drug product/study procedure (assessment of causality),
- its expectedness (assessment of expectedness),
- its duration (start and end dates or if continuing at final exam),
- action taken (e.g., investigational drug product dosage adjusted/ temporarily interrupted/ permanently discontinued, hospitalization, treatment),
- outcome.

Methods and Timing: The safety surveillance system is based on documentation and reporting of SAEs to the Sponsor. A continuous medical assessment is performed by the Sponsor to identify any risk for patient's given by study conduct or study procedures. All reported SAEs are subject to a second assessment in a timely manner. If a SUSAR occurs, it is reported to the relevant IRB and competent authority in accordance with regulatory requirements. Investigators are trained (web-based online course) how to document, assess and report (S)AEs.

In addition, a Data Safety Monitoring Board (DSMB) is established, a group of independent and external experts, monitoring patient safety and treatment efficacy throughout the study and advise the Sponsor/LKP. The main responsibility of the DSMB is the protection of the patient's safety throughout the clinical trial duration. Members of the DSMB are experts of the disease, understand the drug, and familiar with the patient population and study protocol.

### Methods for Examples 2-5

**Patient recruitment and ethics approval.** All patients were enrolled either in the prospective observational cohort study (Pa-COVID-19)²³ at Charité - Universitätsmedizin Berlin or the prospective SC2-Study at University Hospital Leipzig, which both included all patients with COVID-19 who were hospitalized between March 11 and May 7, 2020, at either hospital. Both studies were approved by the respective institutional ethics committee of either Charité - Universitätsmedizin Berlin (EA2/066/20) or the Medical Faculty of the University of Leipzig (123/20-ek) and were conducted in accordance with the Declaration of Helsinki. We obtained signed informed consent from all patients before inclusion in our study.

**Patient cohort.** The classification of COVID-19 severity was based on WHO guidelines10. All patients with COVID-19 (median age, 61 years; range, 21-76 years; 15 males/4 females) described common cold symptoms such as dry cough and fever, followed by malaise, cephalalgia and myalgia at the onset of the disease. In addition, dyspnea was a common initial symptom of the patients with critical COVID-19. The median duration from first symptoms until hospital admission was 9 d for patients with moderate COVID-19 (range, 2-19 d) and 6 d for patients with critical COVID-19 (range, 1-9 d). Patients with moderate COVID-19 were hospitalized for a median duration of 8 d, whereas patients with critical COVID- 19 were hospitalized for a median duration of 25 d, with some patients being hospitalized for more than 9 weeks. Most of the patients with critical COVID-19 required mechanical ventilation within the first 9 d after the onset of symptoms, with a median duration of ICU length of stay of 25 d. The only exception was patient BIH-CoV-07, who was admitted to the ICU for 2 d requiring nasal high-flow oxygen therapy. Patient BIH-CoV-08 was the only patient in this study cohort who required extracorporeal membrane oxygenation (ECMO) support. The sequential organ failure assessment (SOFA) scores as well as the respiratory system sub-score of SOFA of the patients in the ICU were used as an indicator for multi-organ dysfunction or lung injury, respectively (Table 2). Sex, age, information on the onset of COVID-19 symptoms, diagnosis and hospitalization for each individual are provided in Fig. 2b and Table 2. Two patients with critical COVID-19 (BIH-CoV-01 and BIH-CoV-02) died later in the disease course. We included samples of five control patients. These patients were hospitalized for an elective surgery (orthopedic/aesthetic), tested negative for SARS-CoV-2 and had no cold- or flu-like symptoms (BIH-Con-01 to BIH-Con-05; two males and three females; median age, 34 years; range, 24-41 years). Notably, our cohort does not reflect the general patient distribution admitted to Charité or University Hospital Leipzig with regard to sex, age or COVID- 19 severity, as the patients were randomly chosen based on their presence in the hospital and willingness to participate in this study. As a result, the critical and moderate patient groups are not matched with respect to days after onset of symptoms. Hence, in each statistical comparison, we considered 'days after symptoms' as a confounding factor in the statistical model (for details, see below) to correct for this imbalance. We received NSs from all controls and patients with COVID-19 (for sampling days relative to the onset of symptoms, see Fig. 2b and Table 2). In addition, we investigated samples obtained from PSBs and BLs from two patients: BIH-CoV-01 and BIH-CoV-04.

**Isolation and preparation of single cells from human airway specimens.** We used human airway specimens freshly procured using NSs, bronchial brushes or BLs for scRNA-seq. The NSs and bronchial brushes taken from donors were directly transferred into 500 µl of cold DMEM/F12 medium (Gibco, 11039). Processing of all samples started within 1 h after collection and under biosafety S3 conditions (non-infectious donor nasopharyngeal samples were processed under biosafety S1 and S2 conditions). Nasopharyngeal and bronchial samples were pre-treated with 500 µl of 13 mM DTT (AppliChem, A2948), followed by dislodging the cells from the swabs/ brushes by carefully pipetting the solution onto the swab/brush. The swab/brush was dipped 20 times in the solution to ensure the release of all cells and then discarded. BL samples were immediately treated with an equal volume of 13 mM DTT. Afterwards, all following steps were identical for NS, PSB and BL samples. All samples were incubated on a thermomixer at 37 °C at 500 r.p.m. for 10 min and then spun down at 350g at 4 °C for 5 min (but see next paragraph). The supernatant was carefully removed, followed by inspection of the cell pellet for any trace of blood. If so, cells were resuspended in 500 µl of 1 × phosphate-buffered saline (PBS) (Sigma-Aldrich, D8537) and 1 ml of RBC Lysis Buffer (Roche, 11814389001), incubated at 25 °C for 10 min and subsequently spun down at 350g at 4 °C for 5 min. The cell pellet was resuspended in 500 µl of Accutase (Thermo Fisher, 00-4555-56) and incubated at room temperature for 10 min to achieve a single-cell suspension. Afterwards, 500 µl of DMEM/F12 supplemented with 10% fetal bovine serum (FBS) was mixed into the cell suspension, followed by centrifugation at 350g at 4 °C for 5 min. After removal of the supernatant, the cell pellet was resuspended in 100 µl of 1 × PBS, and the suspension was passed through a 35-µm cell strainer (Falcon, 352235) to remove cellular aggregates, followed by cell counting using a disposable Neubauer chamber (NanoEnTek, DHC-N01). Cell and gel bead emulsions were generated by loading the cell suspension into the 10X Chromium Controller using the 10X Genomics Single Cell 3' Library Kit v3.1 (10X Genomics: PN 1000223, PN 1000157, PN 1000213 and PN 1000122). The subsequent steps of reverse transcription, cDNA amplification and library preparation were performed according to the manufacturer's protocol. Note that the incubation at 85 °C during reverse transcription was extended to 10 min to ensure virus inactivation. The final libraries were pooled (S2 flowcell: up to eight samples; S4 flowcell: up to 20 samples) and sequenced with the NovaSeq 6000 Sequencing System with either S2 or S4 flow cells (Illumina; paired end, single indexing). Samples that were not immediately prepared for cell encapsulation were resuspended in cryopreservation medium (20% FBS (Gibco, 10500), 10% DMSO (Sigma-Aldrich, D8418) and 70% DMEM/F12) and stored at -80 °C. Frozen cells were thawed quickly at 37 °C, spun down at 350g at 4 °C for 5 min and proceeded with normal processing. In cases where satisfactory cell suspensions were achieved without protease treatment, the samples were filtered through a 20-µm cell strainer (pluriSelect, 43-50020-03) and directly loaded for encapsulation.

**qRT-PCR.** After DTT treatment of the patient samples, 140 µl of supernatant was used for the extraction of viral RNA with the QIAmp Viral RNA Mini Kit (Qiagen, 52904). Quantitative RT-PCR for the E gene of SARS-CoV-2²⁴ of the extracted RNA in technical triplicates along with three tenfold dilutions of standardized SARS-CoV-2 genome equivalents was performed using the SuperScript III One-Step RT-PCR Kit (Thermo Fisher, 12574026) and the Roche LightCycler 480.

**Pre-processing and data analysis.** The raw 3' scRNA-seq data were processed using CellRanger version 3.1.0 (10X Genomics). The transcripts were aligned to a customized reference genome in which the SARS-CoV-2 genome (Refseq-ID: NC_045512) was added as an additional chromosome to the human reference genome hg19 (10X Genomics, version 3.0.0). An entry summarizing the entire SARS-CoV-2 genome as one 'gene' was appended to the hg19 annotation gtf file, and the genome was indexed using 'cellranger_mkref'. After pre-processing, contaminating ambient RNA reads were filtered by using SoupX²⁵ version 1.2.2 (https://github.com/constantAmateur/SoupX) and MUC1, MUC5AC and MUC5B as marker genes. The filtered expression matrices were loaded into R version 3.6.1 with Seurat version 3.1.4.9012 (https://github.com/ satijalab/seurat), where further filtering was done to remove cells with fewer than 200 genes expressed or more than 15% mitochondrial transcripts. The quality of the scRNA-seq data set was assessed by plotting the number of unique molecular identifiers (UMIs) and genes per cell for each sample. The quality was consistent across samples, and differences in RNA and gene content could be ascribed to cell-type-specific effects. An upper cutoff for the number of UMIs was manually determined for each sample based on a plot of gene count versus UMI count, with values ranging between 50,000 and 200,000 UMIs. After quality control filtering, the samples were normalized to 10,000 reads, scaled and centered. For integration, 3,000 shared highly variable genes were identified using Seurat's 'SelectlntegrationFeatures()' function. Integration anchors were identified based on these genes using canonical correlation analysis²⁶ with 90 dimensions as implemented in the 'FindTransferAnchors()' function. The data were then integrated using 'lntegrateData()' and scaled again using 'ScaleData()'. Principal component analysis (PCA) and uniform manifold approximation and projection (UMAP) dimension reduction with 90 principal components were performed. A nearest-neighbor graph using the 90 dimensions of the PCA reduction was calculated using 'FindNeighbors()', followed by clustering using 'FindClusters()' with a resolution of 0.6. Viral load was calculated on the raw data matrices output by CellRanger. All reads aligning to the SARS-CoV-2 genome per sample were aggregated and divided by the total number of reads in that sample. Multiplication by 106 then yielded the CPM viral load values. Cell-cell interactions based on the expression of known ligand-receptor pairs in different cell types were calculated using CellPhoneDB⁴⁹ version 2.1.2 (https:// github.com/Teichlab/cellphonedb) on data sub-sampled according to the following strategy: to decrease the effect of samples with high cell numbers, all samples were randomly down-sampled to the size of the smallest sample. A maximum of 2,000 cells per cell type were kept to reduce calculation times. Transcription factor importance was scored using arboreto²⁷ 0.1.5 and pyscenic²⁸ 0.10.0. To infer the cluster and lineage relationships between the different epithelial cell types identified, Monocle3 was used^{22,29} (https://github.com/cole-trapnell-lab/ monocle3). A subset of the epithelial cells was used for trajectory inference: basal, secretory-diff, secretory, IFNG-responsive, FOXN4+, ciliated-diff and ciliated cells. After scaling and dimensional reduction by PCA, the UMAP was calculated for this subset. UMAP embeddings and cell clusters generated from Seurat were used as input, and trajectory graph learning and pseudo-time measurement through reversed graph embedding were performed with Monocle3. To identify genes that are significantly regulated as the cells differentiate along the cell-to-cell distance trajectory, we used the differentialGeneTest() function implemented in Monocle2²². This function fits a vector generalized additive model (VGAM) from the VGAM 1.1-2 package for each gene in our data set where the gene expression levels are modeled as a smooth nonlinear function of the pseudo-time value of each cell. Thereafter, genes were ranked by their order of importance based on q values (adjusted P values after Benjamini-Hochberg correction), where q values less than 0.05 were considered as pseudo-time-dependent genes. The expression change of genes, for which q < 1 × 10-250 and which are biologically informative for the corresponding differentiation pathways, was plotted along the cell-to-cell distance (pseudo-time). For the basal-diff trajectory, we performed a VGAM fit on basal, basal-diff, ciliated-diff and ciliated clusters. For the IFNG-responsive cell pathway, the basal, secretory-diff, IFNG responsive, ciliated-diff and ciliated cell types were extracted. Basal, secretory-diff, secretory, FOXN4+, ciliated-diff and ciliated cell clusters were considered when identifying critical genes for the FOXN4+ trajectory.

Statistics. For comparisons between expression values, the Seurat function 'FindMarkers()' was used with the 'negbinom' method and days after onset of symptoms as the confounder variable. Cell type markers were obtained using the 'FindAllMarkers()' function with a negative binomial test and the case severity as the latent variable. Cell type numbers were compared by logistic regression followed by Tukey's test in multivariate cases and otherwise by Fisher's exact test. Weights corresponding to the cell count per sample were introduced into the logistic regression to account for differences in information content. Correlation was assessed using Spearman's P, and significance of correlation was calculated using the corr.test function in R. All tests were two sided. P values were corrected using the Benjamini-Hochberg (false discovery rate) method. Homoskedasticity was assessed using Bartlett's test. Cells were defined as positive for a gene if at least one UMI of that gene was found. Note that sample numbers in the figure legends refer to biological samples, and the n for statistical calculations is derived from the cell count in expression value comparisons.

**Identification of cell type and state.** Epithelial and immune cell types were primarily annotated based on their expression levels of their respective cell type markers. All previously described major epithelial cells of the conducting airways, including basal, secretory and ciliated cells, as well as the recently discovered FOXN4+ cells and ionocytes, were found. In addition, we identified a cell type from the most posterior region of the nasopharynx that we call 'squamous' cells that is characterized by a strong expression of SPRR genes essential to squamous cell cornification (Fig. 3b)^{30,31}. Basal, secretory and ciliated cells were present in different cell states from differentiating to terminally differentiated as shown by their intermediate expression levels of their respective cell type markers, inferred mitotic stages and pseudo-time analysis. In particular, secretory-diff and ciliated-diff cells represent the differentiating state, whereas secretory and ciliated cells are further differentiated. Notably, the FOXN4+ cells resemble the transient secretory cell type described as the most SARS-CoV- 2-vulnerable bronchial cells in non-infected individuals by virtue of their function as transitionary cell types differentiating from the secretory to ciliated lineage ⁵⁷. We also identified small clusters of cells that are predominantly derived from single patients that we call 'outliers epithelial', which have a strong expression of ciliated markers. Similarly, a cluster of cells called 'unknown epithelial' is also identified. These two cell types were excluded from any downstream analysis. Within the immune cell population, we identified 13 different cell types, including different types of macrophages, dendritic cells, NK cells, NKT cells, mast cells, neutrophils, B cells and T cells (Fig. 3b). In the monocyte-derived (MD) fraction, we distinguished resident macrophages (rMa), MD macrophages (moMas), MD dendritic cells (moDCs) and non-resident macrophages (nrMa)^{32,33}. In the T lymphocyte population, we identified CD8+ cytotoxic (CTL) CD4+ T cells together with regulatory T cells (CD4T/Treg) and NKT cells65. Within the immune cell population, we also found NK cells (NCAM1, FCGR3A and KLRD1), distinguishable from the NKT population as they were missing the canonical T cell markers. In addition, proliferating NKT as described previously65 and plasma cells (CD27, SDC1 and CD79A) were observed. The CTL and NKT cell populations were similar to each other and mainly showed a quantitative difference in the expression of genes rather than a strong difference in characteristic markers. By analyzing the epithelial compartment for trajectory inference, we were able to achieve a higher resolution of the epithelial cell types and identified a sub-cluster of basal cells differentiating to ciliated cells, called 'basal-diff'. This cell type was defined by the low but mixed marker expression of basal and ciliated cells and by pseudo-time information. Cell type identification was similarly done for samples from NSs, bronchial PSBs and BLs for two patients with critical COVID-19 (BIH-CoV-01 and BIH-CoV-04). We used the same markers as shown in Extended Data Fig. 2 to determine cell types and states. As additional cell types in these samples, we characterized secretory (Secretory LYPD2) and ciliated (Ciliated IFN) cell clusters with a heterogeneous expression of ISGs (IFI6, IFI16, IFI44, IFIT3 and IFITM3). For one particular patient (BIH-CoV-04), we identified a 'hybrid' cell type that expresses mixed markers for secretory and ciliated cells (SCGB1A1, MUC5AC, MSMB, TFF3, AGR2, CAPS and SNTN).

**Table 2: Clinical annotation of the COVID-19 patient cohort.**

| **Patient IDs** | **Age** | **Sex** | **Relative to symptoms start** | | **Ventilation type** | **SOFA Score sampling** | | **Highest SOFA** | **Multi-organ failure** | **Duration in hospital** | **COVID-19 severity** | **Sampling days post symptoms** | **Symptoms** | **Antiviral or immunomodulatory medication*** |
|---|---|---|---|---|---|---|---|---|---|---|---|---|---|---|
| | | | **Positive SARS-CoV-2** | **ICU duration** | | | | | | | | | | |
| **BIH-CoV-15** | 21 | m | 15 days | | | | | | no | 35 days | moderate | +25, +27, +30, +33 | dry cough, fever, cephalgia | none |
| **BIH-CoV-14** | 45 | m | 9 days | | | | | | | 14 days | | +11, +16 | cough, runny nose | |
| **BIH-CoV-16** | 50 | f | 7 days | | | | | | | 7 days | | +10 | fever, diarrhea, dry cough, sore throat, myalgia, malaise | |
| **BIH-CoV-13** | 52 | m | 5 days | | none | | | | | 7 days | | +10 | malaise, cephalgia, back pain; pneumonia during hospitalization | |
| **BIH-CoV-19** | 64 | m | 7 days | | | | | | | 8 days | | +11 | dyspnea | |
| **BIH-CoV-12** | 71 | m | 2 days | | | | | | | 8 days | | +16, +18, +21 | dry cough, fever | |
| **BIH-CoV-17** | 73 | m | 19 days | | | | | | | 13 days | | +30 | fever, cough, dyspnea | |
| **BIH-CoV-18** | 76 | m | 2 days | | | | | | | 8 days | | +3 | disoriented, dry cough | |
| **BIH-CoV-07** | 32 | m | 5 days | day 8 to day 9 | non-invasive | 3 | N/A | 3 | no | 15 days | | +8, +11 | dry cough, fever, myalgia, dyspnea | none |
| **BIH-CoV-11** | 61 | m | 10 days | day 9 to day 17 | non-invasive | 3 | | 3 | | 10 days | | +11 | dry cough, fever, common cold symptoms | |
| **BIH-CoV-06** | 63 | m | 1 day | day 3 to day 51 | invasive | 11 | 12 | 14 | yes | 65 days | | +4, +7 | myalgia, fever, malaise, dry cough dyspnea during hospitalization | |
| **BIH-CoV-05** | 75 | m | same day | day 6 to day 33 | invasive | 10 | | 13 | | 27 days | | +7 | fever, dispnea, malaise | prior to admission: Hydroxychloroquine and Lopinavir /Ritonavir |
| **BIH-CoV-09** | 53 | f | 5 days | day 8 to day 33 | invasive | 8 | | 11 | | 25 days | critical | +12 | sinusitis, fever, dry cough, dyspnea | none |
| **BIH-CoV-03** | 52 | m | 8 days | day 7 to day 24 | invasive | 6 | | 9 | | 18 days | | +10 | dry cough, fever, cephalgia, malaise | |
| **BIH-CoV-08** | 62 | f | 7 days | day 1 to day 56 + | invasive | 12 | | 13 | | 56 days ongoing | | +8 | dyspnea | Hydrocortisone |
| **BIH-CoV-04** | 73 | m | 4 days | day 7 to day 32 | invasive | N/A | | 12 | | 25 days | | +13 | N/A | |
| **BIH-CoV-10** | 68 | m | 7 days | day 7 to day 34 | invasive | 12 | 7 | 12 | | 37 days ongoing | | +20 | dry cough, malaise | none |
| **BIH-CoV-01** | 50 | f | 6 days | day 5 to day 25 | invasive | 15 | | 15 | | deceased 25 days post symptoms | | +17 | N/A | |
| **BIH-CoV-02** | 54 | m | unknown | day 4 to day 11 | invasive | N/A | | | | deceased 11 days post symptoms | | +7 | fever, dyspnea | Lopinavir /Ritonavir, Raltegravir, Fludrocortisone |

| | | | | | | | | | | | | | | |
|---|---|---|---|---|---|---|---|---|---|---|---|---|---|---|
| * other than NSAIDs | | | | | | | | | | | | | | |

### Example 2: Cellular and molecular characterization of COVID-19 severity.

Cellular and molecular characterization of COVID-19 severity. We performed 3'scRNA-seq on nasopharyngeal or pooled nasopharyngeal/ pharyngeal swabs (NSs), bronchial protected specimen brushes (PSBs) and bronchial lavages (BLs) (Fig. 2a). We analyzed a dual-center cohort from Charité - Universitätsmedizin Berlin and University Hospital Leipzig, comprising 19 patients with COVID-19 (15 males and 4 females; age range, 21-76 years; median age, 61 years; Fig. 2b and Table 2). Five SARS-CoV- 2-negative donors with no signs of disease were included as controls (two males and three females; age range, 24-41 years; median age, 34 years). On the basis of World Health Organization (WHO) guidelines10, we classified eight patients as moderate and 11 patients as critical, two of whom died several days later in the course of the disease (Fig. 2b and Table 2; patients BIH-CoV-01 and BIH-CoV-02). All patients and controls were tested by NS, which samples the primary infection site: the nasopharyngeal area. Five patients with COVID-19 were sampled longitudinally at 2-4 time points by NS to investigate cellular and transcriptional dynamics during the course of disease (patients BIH-CoV-06, BIH-CoV-07, BIH-CoV-12, BIH-CoV-14 and BIH-CoV-15). Fortwo patients, multiple samples from both the upper and lower airways were collected (NS, PSB and BL), allowing comparison between compartments of the respiratory tract (patients BIH-CoV-01 and BIH-CoV-04). We provide transcriptional profiles of 160,528 cells obtained from a total of 36 samples taken from 24 individuals (Fig. 3a). In NS samples, we identified 22 different cell types and states within the epithelial cell and immune cell populations (Fig. 3a). We detected all previously described major epithelial cell types of the conducting airways, including basal, secretory and ciliated cells, as well as the recently discovered FOXN4+ cells and ionocytes (Fig. 3a) ¹¹⁻¹³. Notably, we identified a subpopulation of epithelial cells differentiating from immature secretory cells to ciliated cells with a distinctively strong interferon gamma (IFNG) response signature (IFNG-responsive cells; (Fig. 3a), suggesting their stimulation by the host immune system^{14,15}. Within the immune cell population, we identified 13 different cell types and states, consisting of macrophages, dendritic cells, mast cells, neutrophils, B cells, T cells, natural killer (NK) cells and subsets thereof (Fig. 3a). Comparing the cellular landscapes, we observed that patients with critical COVID-19 showed a striking depletion of basal cells (P < 1.00 × 10-9) and a strong enrichment for neutrophils (P < 1.00 × 10-9) compared to both patients with moderate COVID-19 and controls (Fig. 3b). This finding should be interpreted with caution as cell populations change dramatically over the course of active viral infections¹⁶⁻¹⁸ and are affected by clinical and genetic differences, sampling dates and the number of days since the onset of symptoms.

### Example 3: Variable ACE2 expression in the upper airway epithelium

SARS-CoV-2 is thought to initially infect the mucosa of the upper respiratory tract facilitated by viral binding to the host receptor ACE2 and S protein priming by specific proteases, such as TMPRSS2 or FURIN⁴⁰⁻⁴⁴. Expression levels of ACE2 with either one or both S-priming proteases were increased by three-fold in secretory cells in patients with COVID-19 compared to non-infected controls. Although the number of ACE2+ ciliated cells was not increased after infection, this cell population had a high overall number of ACE2+ cells, suggesting a generally high susceptibility for infection. Indeed, this susceptibility was recently confirmed by preferential ACE2 protein localization on motile cilia and a strong infectivity of ciliated cells by SARS-CoV-2 in vitro^{45,46}. Most ciliated cells were ACE2+/TMPRSS2+, whereas secretory and FOXN4+ cells were predominantly ACE2+/TMPRSS2+/FURIN+. Secretory and ciliated cells also contained the highest fraction of SARS-CoV-2-infected cells. In agreement with recent findings⁴⁷, the number of detected viral transcripts as count per million (CPM) reads was rather low in all patients with COVID-19 and mostly detectable at considerable levels within the first 11 d after the onset of symptoms (Fig. 3b). ACE2+ cells were significantly overrepresented within the epithelial cells compared to the immune cells (58-98-fold enrichment, P < 0.001). Viral transcripts captured in a very low number of immune cells were likely due to viral association (for example, phagocytosis or surface binding of viral particles) rather than productive infection. This view is in line with a study reporting virtual absence of viral reads in peripheral blood mononuclear cell samples from patients with COVID-19⁴⁸.

### Example 4: Immune-mediated epithelial damage in severe COVID-19.

Infected epithelial cells secrete chemokines that recruit and activate different immune cell populations at the site of viral infection¹⁵. In patients with moderate COVID-19, secretory cells showed a significantly higher expression of the chemokine-ligand encoding genes CXCL1, CXCL3, CXCL6, CXCL16 and CXCL17 compared to the controls, likely promoting the recruitment of neutrophils, T cells (CTLs and CD4T/Treg) and mast cells, respectively (Fig. 7, middle panel). Ciliated cells strongly expressed CCL15, which might contribute to an inflow of monocytes/macrophages and neutrophils via CCR1. The chemokine and chemokine receptor expression of the different cell populations increased markedly in the critical compared to the moderate cases (Fig. 7, lower panel), suggesting an augmented recruitment of immune cells to the sites of inflammation. To map the complex interactions of epithelial and immune cells, we inferred all possible intercellular communications by the expression of ligand-receptor pairs in both cell populations with CellPhoneDB⁴⁹. In moderate cases, the strong interactions among ciliated cells, secretory cells and different immune cell types, including CTLs, CD4T/Tregs, B cells, non-resident macrophages (nrMa), monocyte-derived macrophages (moMa) and neutrophils, might reflect the necessary and well-balanced immune response required for the efficient elimination of virus-infected epithelial cells. Interactions between the different cell types were higher in patients with critical COVID-19 compared to patients with moderate COVID-19, in particular for CTL, nrMa and moMa. Higher numbers of epithelium-immune cell interactions in patients with critical COVID-19 are consistent with a higher activation status of nrMa, moMa and CTL (Fig. 4a). In particular, nrMa showed a highly inflammatory profile characterized by the expression of the chemokine encoding genes CCL2 (encoding MCP1), CCL3 (encoding MIP1a), CCL20, CXCL1 and CXCL3 and the pro-inflammatory cytokines IL1B, IL8, IL18 and TNF. Except for CXCL3 and IL1B, each of these inflammatory mediators was expressed at significantly higher levels in patients with critical COVID-19 (Fig. 4a). moMa were mainly characterized by CCL2, CCL3 and CXCL10 expression, with higher CCL3 expression levels in the patients with critical COVID-19 (Fig. 4a). Notably, increased CCL2 and CCL3 expression corresponded to a significant induction of CCR1—encoding the receptor binding to MIP1a and, with a lower affinity, to MCP1 as well⁵⁰—in neutrophils, CTLs and different macrophage populations (Fig. 4b and Fig. 7). In the patients with moderate COVID-19, CTL displayed the characteristic transcriptional profile of anti-viral CD8+ T lymphocytes with high expression of IFNG, TNF, CCL5 and PRF1 (encoding perforin) and GZMB and GZMA (encoding granzymes) together with genes encoding for cytotoxic receptors (KLRB1, KLRC1 and KLRD1) (Fig. 4a). In the patients with critical COVID-19, CTL expressed lower levels of CCL5, IFNG and TNF but strongly increased their cytotoxic potential (Fig. 4a). The potential resulting damage to epithelial cells is reflected by a statistically significantly increased death of ciliated and secretory cells in moderate and critical cases versus control cases. FOXN4+ cells, which were found only in patients with COVID- 19, displayed the highest levels of effector caspase 3 (CASP3). This increased level of cell death is reflected by the marked upregulation of genes encoding cell death receptors (FAS and TNFRS1A) and pro-apoptotic factors, including the initiator caspase 8 (CASP8), CASP3 and cytochrome c (CYCS), in ciliated, FOXN4+ and secretory cells. To assess whether the inflammatory macrophages and activated CTLs observed in the nasopharyngeal mucosa were also present in the lower airways, we collected multiple samples of two patients (BIH-CoV-01 and BIH-CoV-04) from the upper (NS) and lower (PSB and BL) airways. All epithelial and immune cell populations were present in the integrated scRNA-seq data set of all three sampled sites of the. Furthermore, distribution of ACE2+ cells was spread across all three sites, whereas cells containing viral reads primarily appeared in neutrophils in BL samples, potentially reflecting the phagocytosis of apoptotic virus-infected epithelial cells. This is in agreement with our observation that the viral load, as measured by total counts of viral reads and viral copy numbers, was higher in BL compared to the nasopharyngeal mucosa for both patients. Macrophages in the lower airways (PSB and BL) had a stronger inflammatory signature than those within the upper airways (NS) as both the expression level for genes encoding for inflammatory chemokines/cytokines and the number of positive macrophages were markedly elevated (Fig. 4c). The presence of highly inflammatory macrophages and CTLs (Fig. 4c) corresponded with an increased expression of effector CASP3 in the PSB as well as the BL samples. The potential for apoptosis in basal and ciliated cells was significantly higher in the lower (PSB or BL) compared to the upper (NS) airways, reflecting the presence of highly inflammatory macrophages and CTLs.

### Example 5: Patient-specific inflammatory profiles.

To systematically compare the pronounced differences in the cytokine/chemokine expression profiles between individual cases, we compared the expression patterns within nrMa in longitudinal samples of patients with different degrees of disease severity (Fig. 5). Patient BIH-CoV-12 (male, 71 years old) is a typical patient with a moderate course of COVID-19. Patient BIH-CoV-07, a middle-aged male without any risk factors, suffered from critical COVID-19 with transient need for high-flow nasal oxygen ventilation and intensive care monitoring. He was admitted to the intensive care unit (ICU) with an isolated respiratory failure and bipulmonary infiltrates. He was discharged from the ICU after 2 d and left the hospital in good health. Patient BIH-CoV-06 is a 63-year-old male, with obesity and a history of smoking, who presented with critical COVID-19. He required mechanical ventilation and developed severe acute respiratory distress syndrome (ARDS) and multi-organ failure. After 2 weeks in the ICU, his condition gradually improved, and he could be extubated. Patient BIH-CoV-02 had critical COVID- 19 with severe ARDS and died. The expression patterns of genes encoding for cytokines/chemokines in patients BIH-CoV-12 and BIH-CoV-07 were similar. Only a slight increase of IL8 expressing nrMa over time was observed for patient BIH-CoV-07 (Fig. 5). Both mechanically ventilated patients with critical COVID-19 (BIH-CoV-02 and BIH-CoV-06) showed increased expression of CCL20, CXCL5, CXCL3, CXCL1, CCL3 and CCL2, along with an enhanced expression of TNF and IL1B compared to patients BIH-CoV-12 and BIH-CoV-07 (Fig. 5). In patient BIH-CoV-06, these changes increased over time, converging on a similar pattern as observed for patient BIH-CoV-02, who died shortly after the depicted observation time point. This would be in line with the clinical course of patient BIH-CoV-06, as he remained ill in critical condition for a prolonged period of time. Extravasation of immune cells and the recruitment of blood monocytes to the infected or injured tissue and their migration across the endothelium are crucial events in early immune defense against viral infection, but they also promote capillary leakage and lung damage. A variety of molecules is involved in monocyte trafficking across the vessel wall (for example, integrins)⁵¹. Assessing the expression of markers along the extravasation cascade with respect to COVID-19 severity, we observed a strong increase of these genes in nrMa. ITGAL (encoding CD11a), ITGAM (encoding CD11b), ITGAX (encoding CD11c), ITGB1 (encoding CD29) and ITGB1 (encoding CD18) represent integrins forming the complexes LFA-1, Mac-1 and VLA-4, which are all described as essential for monocyte migration across the endothelium⁵². Patient BIH-CoV-06 showed a marked increase of cells expressing ITGAM, ITGAX, ITGB2 and ITGB1 at both time points investigated compared to patient BIH-CoV-12 with moderate COVID-19 (Fig. 5). This is in line with the increased proportion of CD44, a crucial mediator of host defense, expressing cells in this patient⁵³⁻⁵⁶. We found that ICAM1 and VEGFA—attractant factors commonly expressed by endothelial (and epithelial) cells to recruit leukocytes to sites of infection—also expressed by monocytes themselves, suggesting that activated monocytes induced their own recruitment. The increased expression of these extravasation markers indicates an enhanced anti-viral immune response, which might damage the epithelial and endothelial barrier alike. Patient BIH-CoV-02, with the most critical clinical course, showed a similar pattern of extravasation marker expression (Fig. 5). In general, a smaller proportion of nrMa expressed the extravasation markers in this patient.

### Discussion for Example 2-5

In this study, we provide the first comprehensive description of the transcriptional and cellular landscape of the upper and lower respiratory tract in patients with COVID-19. It has been repeatedly suggested that an excessive production of pro-inflammatory cytokines promotes the development of fatal pneumonia and acute lung injury in COVID-19^{4,9}. In agreement with recently published studies on bronchioalveolar lavage fluid cells of patients with mild and severe COVID-19^{5,6}, we show an inflammatory FCN1+ macrophage phenotype in patients with critical COVID-19, which shares similarities with our nrMa population. Expressing pro-inflammatory mediators such as CCL2, CCL3, CCL20, CXCL1, CXCL3, CXCL10, IL8, IL1B and TNF, this macrophage subpopulation might contribute to excessive inflammation by promoting monocyte recruitment and differentiation. Increased levels of those chemokines were found in plasma samples of patients with COVID-19 who died³. Although recent studies have investigated the immune cell response in patients with COVID-19 in the lower respiratory tract^{5,6}, our study adds fundamental understanding of the cellular response to SARS-CoV-2 infection in several aspects. First, we provide a cellular dissection of both the upper respiratory tract in the nasopharynx as the primary site of viral infection and the lower respiratory tract. The side-by-side comparison of the upper and lower respiratory tract in the same patients at the same time point showed that the earlier described hyperinflammatory phenotype^{5,6} is significantly enhanced in the bronchia compared to the nasopharynx. Second, we provide a detailed characterization of the interaction between immune cells and epithelial cells that is likely to contribute to the clinical observations of heightened inflammatory tissue damage, lung injury and respiratory failure^{4,9}. We demonstrated that infected epithelial cells upregulate the expression of the SARS-CoV-2 entry receptor ACE2, which correlates with interferon signals in immune cells. This upregulation of ACE2 as an ISG might counteract viral infection, which is in line with the anti-inflammatory and protective function of ACE2 in ARDS³⁵⁻³⁷. In patients with COVID-19, however, an increase in the number of ACE2+ cells potentially renders these patients even more vulnerable to SARS-CoV-2 infection. Together with the presently described IFNG-driven differentiation of immature secretory cells into the largely ACE2+ ciliated cells in patients with COVID-19, this host immune response pathway likely increases the number of ACE2+ cells and thus enhances the susceptibility of the airway epithelium to the SARS-CoV-2 infection. Third, longitudinal samples of patients with moderate and critical COVID-19 allowed us to correlate the clinical course of disease with changes in the cellular and transcriptional landscape, suggesting pharmacological interventions for patients with critical COVID-19. Development of COVID-19 therapies for immune hyperactivation is currently focused on inhibitors of key immunological players, such as IL-6, TNF and interferon³⁸ (NCT04359901, NCT04322773 and NCT04311697). Our data suggest the possibility of targeting chemokine receptors. We found a significant induction of CCL2 and CCL3 expression in macrophages together with an increased expression of CCR1, the receptor for both chemokines, in patients with critical COVID-19. Because binding of CCL2 or CCL3 to CCR1, CCR2 or CCR5 can induce monocyte recruitment into the lung parenchyma with subsequent differentiation into inflammatory macrophages and consecutive recruitment and activation of additional immune cells and epithelial damage, CCR1, CCR2 and CCR5 might represent promising anti-inflammatory targets in COVID-19. Targeting the CCR2/CCL2 axis has been introduced in HIV and other viral infections³⁹. However, we did not observe CCR2 expression in the respiratory tract of patients with COVID-19 (presumably because of its rapid downregulation in monocytes as they exit the bloodstream and enter tissues; leaving CCR1 and/or CCR5 as potential therapeutic targets. A shortcoming of our study is that we were not able to stratify the patients included in our study, owing to a limited number of hospitalized patients during the COVID-19 pandemic. This impeded a more detailed analysis of potential contributing factors such as age, gender and further comorbidities. In addition, as we were not able to include patients with COVID-19 who showed mild symptoms and as cases without the need for hospitalization were lacking, future studies will have to assess the single-cell landscape of such patients, which, in comparison to our data, might lead to a better understanding of differences in the vulnerability to SARS-CoV-2 infection.

### Example 6: Exacerbated expression of CCL3 and CCL4 observed in ARB-treated hypertensive patients correlates with disease severity.

We evaluated whether the observed hypertension-related inflammatory predisposition of immune cells might contribute to an increased risk for critical COVID-19. All genes showing a hypertension-related inflammatory priming were overlapped with the genes with a significantly increased expression in critical compared to non-critical COVID- 19 (Fig. 8a). The resulting intersection included three genes, namely CCL3, CCL4 and CXCR4 (Fig. 8a). Using a logistic regression model considering age, gender, days after onset of symptoms and study center as potential confounding factors, we confirmed a significant positive relationship between expression of CCL4 derived from nrMa (adjOR/95% CI = 1.04/1.00-1.07, P = 0.027) and CCL3 expressed by Neu (adjOR/95% CI = 1.13/1.01-1.27, P = 0.02) with an increased risk for critical COVID-19 (Fig. 8b). Notably, expression of CCR1, the receptor bound by CCL3 and CCL4, increased in nrMa and Neu concomitantly with severity of COVID-19, supporting the potential of CCR1 as a therapeutic target20 (Fig. 8c). In summary, we conclude that, in contrast to ACEI treatment, ARB therapy was not as efficient in alleviating hypertension-related hyperinflammation, especially in nrMa and Neu, possibly contributing to critical COVID-19 course (Fig. 8d).

### References:

1. Wu, Z. & McGoogan, J. M. Characteristics of and important lessons from the coronavirus disease 2019 (COVID-19) outbreak in China: summary of a report of 72314 cases from the Chinese Center for Disease Control and Prevention. JAMA 323, 1239-1242 (2020).
2. Guan, W. J. et al. Clinical characteristics of coronavirus disease 2019 in China. N. Engl. J. Med. 382, 1708-1720 (2020).
3. Huang, C. et al. Clinical features of patients infected with 2019 novel coronavirus in Wuhan, China. Lancet 395, 497-506 (2020).
4. Mehta, P. et al. COVID-19: consider cytokine storm syndromes and immunosuppression. Lancet 395, 1033-1034 (2020).
5. Bost, P. et al. Host-viral infection maps reveal signatures of severe COVID-19 patients. Cell https://doi.org/10.1016/j.cell.2020.05.006 (2020).
6. Liao, M. et al. Single-cell landscape of bronchoalveolar immune cells in patients with COVID-19. Nat. Med. 26, 842-844 (2020).
7. Branchett, W. J. & Lloyd, C. M. Regulatory cytokine function in the respiratory tract. Mucosal Immunol. 12, 589-600 (2019).
8. Tisoncik, J. R. et al. Into the eye of the cytokine storm. Microbiol. Mol. Biol. Rev. 76, 16-32 (2012).
9. Wen, W. et al. Immune cell profiling of COVID-19 patients in the recovery stage by single-cell sequencing. Cell Discov. 6, 31 (2020).
10. Report of the WHO-China Joint Mission on Coronavirus Disease 2019 (COVID-19) https://www.who.int/docs/default-source/coronaviruse/who-china-joint-mission-on-covid-19-final-report.pdf (2020).
11. Montoro, D. T. et al. A revised airway epithelial hierarchy includes CFTR-expressing ionocytes. Nature 560, 319-324 (2018).
12. Plasschaert, L. W. et al. A single-cell atlas of the airway epithelium reveals the CFTR-rich pulmonary ionocyte. Nature 560, 377-381 (2018).
13. Vieira Braga, F. A. et al. A cellular census of human lungs identifies novel cell states in health and in asthma. Nat. Med. 25, 1153-1163 (2019).
14. Schneider, W. M., Chevillotte, M. D. & Rice, C. M. Interferon-stimulated genes: a complex web of host defenses. Annu. Rev. Immunol. 32, 513-545 (2014).
15. Newton, A. H., Cardani, A. & Braciale, T. J. The host immune response in respiratory virus infection: balancing virus clearance and immunopathology. Semin. Immunopathol. 38, 471-482 (2016).
16. Ibricevic, A. et al. Influenza virus receptor specificity and cell tropism in mouse and human airway epithelial cells. J. Virol. 80, 7469-7480 (2006).
17. Lam, W. Y. et al. Avian influenza virus A/HK/483/97(H5N1) NS1 protein induces apoptosis in human airway epithelial cells. J. Virol. 82, 2741-2751 (2008).
18. Steuerman, Y. et al. Dissection of influenza infection in vivo by single-cell RNA sequencing. Cell Syst. 6, 679-691 (2018).
19. Pardo-Saganta, A. et al. Injury induces direct lineage segregation of functionally distinct airway basal stem/progenitor cell subpopulations. Cell Stem Cell 16, 184-197 (2015).
20. Vaughan, A. E. et al. Lineage-negative progenitors mobilize to regenerate lung epithelium after major injury. Nature 517, 621-625 (2015).
21. Zuo, W. et al. p63(+)Krt5(+) distal airway stem cells are essential for lung regeneration. Nature 517, 616-620 (2015).
22. Qiu, X. et al. Reversed graph embedding resolves complex single-cell trajectories. Nat. Methods 14, 979-982 (2017).
23. Kurth, F. et al. Studying the pathophysiology of coronavirus disease 2019 - a protocol for the Berlin prospective COVID-19 patient cohort (Pa-COVID-19). Preprint at https://www.medrxiv.org/content/10.1101/2020.05.06. 20092833v1 (2020).
24. Corman, V. M. et al. Detection of 2019 novel coronavirus (2019-nCoV) by real-time RT-PCR. Euro. Surveill. 25, 2000045 (2020).
25. Young, M. & Behjati, S. SoupX removes ambient RNA contamination from droplet based single-cell RNA sequencing data. Preprint at https://www.biorxiv.org/content/10.1101/303727v1 (2020).
26. Stuart, T. et al. Comprehensive integration of single-cell data. Cell 177,1888-1902 (2019).
27. Moerman, T. et al. GRNBoost2 and Arboreto: efficient and scalable inference of gene regulatory networks. Bioinformatics 35, 2159-2161 (2019).
28. Aibar, S. et al. SCENIC: single-cell regulatory network inference and clustering. Nat. Methods 14, 1083-1086 (2017).
29. Cao, J. et al. The single-cell transcriptional landscape of mammalian organogenesis. Nature 566, 496-502 (2019).
30. Steinert, P. M., Candi, E., Kartasova, T. & Marekov, L. Small proline-rich proteins are cross-bridging proteins in the cornified cell envelopes of stratified squamous epithelia. J. Struct. Biol. 122, 76-85 (1998).
31. Kalinin, A. E., Kajava, A. V. & Steinert, P. M. Epithelial barrier function: assembly and structural features of the cornified cell envelope. Bioessays 24, 789-800 (2002).
32. Martinez, F. O., Gordon, S., Locati, M. & Mantovani, A. Transcriptional profiling of the human monocyte-to-macrophage differentiation and polarization: new molecules and patterns of gene expression. J. Immunol. 177, 7303-7311 (2006).
33. Gren, S. T. et al. A single-cell gene-expression profile reveals intercellular heterogeneity within human monocyte subsets. PLoS ONE 10, e0144351 (2015).
34. Travaglini, K. J. et al. A molecular cell atlas of the human lung from single cell RNA sequencing. Preprint at https://www.biorxiv.org/content/10.1101/742320v1 (2019).
35. Rodrigues Prestes, T. R., Rocha, N. P., Miranda, A. S., Teixeira, A. L. & Simoes, E. S. A. C. The anti-inflammatory potential of ACE2/angiotensin-(1-7)/Mas receptor axis: evidence from basic and clinical research. Curr. Drug Targets 18, 1301-1313 (2017).
36. Verdecchia, P., Cavallini, C., Spanevello, A. & Angeli, F. The pivotal link between ACE2 deficiency and SARS-CoV-2 infection. Eur. J. Intern. Med. 76, 14-20 (2020).
37. Imai, Y. et al. Angiotensin-converting enzyme 2 protects from severe acute lung failure. Nature 436, 112-116 (2005).
38. Coomes, E. A., & Haghbayan, H. Interleukin-6 in COVID-19: a systematic review and meta-analysis. Preprint at https://www.medrxiv.org/content/10.1101/2020.03.30.20048058v1 (2020).
39. Covino, D. A., Sabbatucci, M. & Fantuzzi, L. The CCL2/CCR2 axis in the pathogenesis of HIV-1 infection: a new cellular target for therapy? Curr. Drug Targets 17, 76-110 (2016).
40. Hoffmann, M. et al. SARS-CoV-2 cell entry depends on ACE2 and TMPRSS2 and is blocked by a clinically proven protease inhibitor. Cell 181, 271-280 (2020).
41. Walls, A. C. et al. Structure, function and antigenicity of the SARS-CoV-2 spike glycoprotein. Cell 181, 281-292 (2020).
42. Yan, R. et al. Structural basis for the recognition of the SARS-CoV-2 by full-length human ACE2. Science 367, 1444-1448 (2020).
43. Zhou, P. et al. A pneumonia outbreak associated with a new coronavirus of probable bat origin. Nature 579, 270-273 (2020).
44. Coutard, B. et al. The spike glycoprotein of the new coronavirus 2019-nCoV contains a furin-like cleavage site absent in CoV of the same clade. Antiviral Res. 176, 104742 (2020).
45. Ravindra, N. G. et al. Single-cell longitudinal analysis of SARS-CoV-2 infection in human bronchial epithelial cells. Preprint at https://www.biorxiv.org/content/10.1101/2020.05.06.081695v1 (2020).
46. Lee, I. T. et al. Robust ACE2 protein expression localizes to the motile cilia of the respiratory tract epithelia and is not increased by ACE inhibitors or angiotensin receptor blockers. Preprint at https://www.medrxiv.org/content/10.1101/2020.05.08.20092866v1 (2020).
47. Wolfel, R. et al. Virological assessment of hospitalized patients with COVID-2019. Nature 581, 465-469 (2020).
48. Xiong, Y. et al. Transcriptomic characteristics of bronchoalveolar lavage fluid and peripheral blood mononuclear cells in COVID-19 patients. Emerg. Microbes Infect. 9, 761-770 (2020).
49. Efremova, M., Vento-Tormo, M., Teichmann, S. A. & Vento-Tormo, R. CellPhoneDB: inferring cell-cell communication from combined expression of multi-subunit ligand-receptor complexes. Nat. Protoc. 15, 1484-1506 (2020).
50. Neote, K., DiGregorio, D., Mak, J. Y., Horuk, R. & Schall, T. J. Molecular cloning, functional expression, and signaling characteristics of a C-C chemokine receptor. Cell 72, 415-425 (1993).
51. Gerhardt, T. & Ley, K. Monocyte trafficking across the vessel wall. Cardiovasc. Res. 107, 321-330 (2015).
52. Meerschaert, J. & Furie, M. B. The adhesion molecules used by monocytes for migration across endothelium include CD11a/CD18, CD11b/CD18, and VLA-4 on monocytes and ICAM-1, VCAM-1, and other ligands on endothelium. J. Immunol. 154, 4099-4112 (1995).
53. Rivadeneira, E. D., Sauls, D. L., Yu, Y., Haynes, B. F. & Weinberg, J. B. Inhibition of HIV type 1 infection of mononuclear phagocytes by anti-CD44 antibodies. AIDS Res. Hum. Retroviruses 11, 541-546 (1995).
54. Vachon, E. et al. CD44-mediated phagocytosis induces inside-out activation of complement receptor-3 in murine macrophages. Blood 110, 4492-4502 (2007).
55. Xu, H., Manivannan, A., Crane, I., Dawson, R. & Liversidge, J. Critical but divergent roles for CD62L and CD44 in directing blood monocyte trafficking in vivo during inflammation. Blood 112, 1166-1174 (2008).
56. He, X. et al. CD44-mediated monocyte transmigration across Cryptococcus neoformans-infected brain microvascular endothelial cells is enhanced by HIV-1 gp41-I90 ectodomain. J. Biomed. Sci. 23, 28 (2016).
57. Lukassen, S. et al. SARS-CoV-2 receptor ACE2 and TMPRSS2 are primarily expressed in bronchial transient secretory cells. EMBO J. 39, e105114 (2020).
58. Vandemeulebroecke, M. et al. Assessment of QT(c)-prolonging potential of BX471 in healthy volunteers. A 'thorough QT study' following ICH E14 using various QT(c) correction methods. British journal of clinical pharmacology vol. 68,3 (2009): 435-46.
59. Zipp F. et al. CCR1 Antagonist Study Group. Blockade of chemokine signaling in patients with multiple sclerosis. Neurology. 2006 Nov 28;67(10):1880-3.

## Claims

1. A pharmaceutical composition comprising BAY 86-5277 or salt thereof for use in the treatment and/or prevention of a medical disorder associated with a viral infection and comprising hyperinflammation in a human subject.

2. The pharmaceutical composition for use according to the preceding claim, wherein the subject is hospitalized and receiving treatment comprising respiratory support.

3. The pharmaceutical composition for use according to the preceding claim, wherein the subject receives non-invasive respiratory support comprising supplementary oxygen and/or invasive mechanical ventilation.

4. The pharmaceutical composition for use according to any one of the preceding claims, wherein the medical disorder comprises hyperinflammation in the respiratory tract, acute respiratory failure, pneumonia, acute respiratory distress syndrome and/or multiorgan failure.

5. The pharmaceutical composition for use according to any one of the preceding claims, wherein the medical disorder is associated with a respiratory viral infection.

6. The pharmaceutical composition for use according to any one of the preceding claims, wherein the medical disorder is associated with a SARS coronavirus infection.

7. The pharmaceutical composition for use according to the preceding claim, wherein the medical disorder is associated with a SARS-CoV-2 infection.

8. The pharmaceutical composition for use according to any one of the preceding claims, wherein the subject has an elevated level of one or more cytokines and/or chemokines selected from CCL2, CCL3, CCL4 and/or chemokine receptor CCR1, wherein said level is elevated in comparison to a level in one or more healthy control subjects and is determined in a sample comprising and/or derived from a bodily fluid or cells from the subject.

9. The pharmaceutical composition for use according to any one of the preceding claims, wherein the subject is or has been treated additionally with a guideline treatment for severe respiratory disease, wherein the guideline treatment is not a CCR1 antagonist.

10. The pharmaceutical composition for use according to the preceding claim, wherein the guideline treatment for severe respiratory disease is glucocorticoid treatment.

11. The pharmaceutical composition for use according to the preceding claim, wherein the glucocorticoid is dexamethasone or salt thereof.

12. The pharmaceutical composition for use according to any one of the preceding claims, wherein BAY 86-5277 or salt thereof and dexamethasone or salt thereof, are administered at a dosage and frequency configured to act synergistically to reduce inflammation, inhibit infection of a target cell and/or inhibit replication of a SARS coronavirus, preferably SARS-CoV-2.

13. The pharmaceutical composition for use according to any one of the preceding claims, wherein said treatment is administered at a dosage and frequency configured to prevent and/or to reduce a level of hyperinflammation and/or to reduce a level of one or more anti-inflammatory cytokines and/or chemokines and/or functional chemokine receptor to a level of at least 20% below a level at treatment initiation.

14. The pharmaceutical composition for use according to any one of the preceding claims, wherein said treatment comprises administering BAY 86-5277 or salt thereof at 500-5000 mg/day to a human subject, preferably at 1000-3000 mg/day.

15. The pharmaceutical composition for use according to the preceding claim, wherein the treatment comprises administration of BAY 86-5277 or a salt thereof 2 to 4 times daily for 5 to 15 days, preferably 4 times daily for 10 days, wherein the route of administration is preferably via a drinking solution of 1 to 100 ml, preferably 5 to 15 ml per dose, via oral delivery or via a gastric tube.

16. The pharmaceutical composition for use according to any one of the preceding claims, wherein said treatment comprises administering a glucocorticoid, preferably dexamethasone or salt thereof at 1 to 20 mg/day, preferably 3 to 10 mg/day or it's corresponding glucocorticoid equivalent dose, respectively.

17. A pharmaceutical combination for use according to any one of the preceding claims, comprising
- (a.) BAY 86-5277 or salt thereof in a pharmaceutical composition in admixture with a pharmaceutically acceptable carrier, and (b.) a glucocorticoid, preferably dexamethasone or salt thereof, in a separate pharmaceutical composition in admixture with a pharmaceutically acceptable carrier,
- (a.) BAY 86-5277 or salt thereof and (b.) a glucocorticoid, preferably dexamethasone or salt thereof, in a kit, in spatial proximity but in separate containers and/or compositions, or
- (a.) BAY 86-5277 or salt thereof and (b.) a glucocorticoid, preferably dexamethasone or salt thereof, combined in a single pharmaceutical composition in admixture with a pharmaceutically acceptable carrier.
- preferably wherein (a.) BAY 86-5277 or salt thereof and (b.) dexamethasone or salt thereof have relative amounts of 10000:1 to 1:1000 by weight, preferably 1000:1 to 1:1 by weight, more preferably about 300:1 by weight.
